# EUROPEAN PATENT APPLICATION

(11) **EP 4 223 876 A1**
(43) Date of publication of application: **09.08.2023**
(21) Application number: 21875667.4
(22) Date of filing: 29.09.2021
(51) Int. Cl.: C12N 15/113, A61K 31/713, A61K 48/00, A61P 31/20, C12N 15/63

(54) **DOUBLE-STRANDED RNA THAT INHIBITS PRODUCTION OF HEPATITIS B VIRUS PROTEIN, AND PHARMACEUTICAL COMPOSITION**

(30) Priority: 30.09.2020 JP 2020164561; 27.04.2021 JP 2021074682
(71) Applicant: FUJIFILM Corporation, Tokyo 106-8620 (JP)
(72) Inventor: HABA Ryota, Ashigarakami-gun, Kanagawa 258-8577 (JP); UEDA Mayumi, Ashigarakami-gun, Kanagawa 258-8577 (JP)
(74) Representative: Hoffmann Eitle
(86) International application number: PCT/JP2021/035781
(87) International publication number: WO 2022/071367

(57) **Abstract**

An object of the present invention is to provide a double-stranded RNA that is useful as a novel anti-hepatitis B virus agent, and a pharmaceutical composition containing the above-described double-stranded RNA. According to the present invention, there is provided a double-stranded RNA that inhibits the production of a hepatitis B virus protein and that inhibits the expression of YTHDC1.

## Description

### BACKGROUND OF THE INVENTION

### 1. Field of the Invention

The present invention relates to a double-stranded RNA having an anti-hepatitis B virus activity.

### 2. Description of the Related Art

Hepatitis B virus (HBV) is a virus belonging to the genus Hepadnavirus and is a spherical DNA virus having a diameter of about 42 nm. The virus itself is a DNA virus consisting of a core having a diameter of 27 nm, which contains a coat (an envelope), an incomplete double-stranded DNA, a DNA polymerase, a reverse transcriptase, and the like, and is called a Dane particle. The Hepatitis B virus is formed in a double structure in which the outside consists of a hepatitis B surface antigen (HBs antigen, HBsAg) and the inside consists of a hepatitis B core antigen (HBc antigen, HBcAg) and a genetic DNA (Non-Patent Document 1).

In addition to the Dane particle, the HBs antigen is present as a hollow particle, a small spherical particle, or a rod-shaped particle. The HBV genome DNA includes four kinds of open reading frames (ORFs), a pre-S/S gene region, a P gene region, an X gene region, and a pre-C/C gene region. The core promoter of the X gene region and the precore region of the C gene region are involved in the production of a hepatitis B envelope antigen (HBe antigen, HBeAg) constituent protein, and the point mutation of this region makes the production of the HBe antigen constituent protein impossible, which results in the HBe antigen negativity.

In a case where HBV invades hepatocytes, the incomplete circular double-stranded DNA is incorporated into the nucleus of the hepatocytes and converted to a covalently closed circular DNA (cccDNA). In the state of chronically being infected by HBV, the cccDNA is present at a quantity of 5 to 50 per hepatocyte, as a mini-chromosome. Four kinds of mRNAs are transcribed from the cccDNA in the hepatocyte nucleus, from which the HBs antigen, the HBc antigen, and the HBe antigen as structural proteins, the X protein, and polymerases including a reverse transcriptase are translated. One of the mRNAs is incorporated into a core particle as the pregenomic RNA, a minus-strand DNA is synthesized by the action of the reverse transcriptase, and then a plus-strand DNA is synthesized to become an incomplete circular double-stranded DNA. Furthermore, the relaxed circular double-stranded DNA is enveloped in an envelope formed of the HBs antigen to become a virus particle (Dane particle), which is released into the blood. The hollow particle (the particle not having a nucleus with DNA) containing the HBs antigen, the HBc antigen, and the p22cr antigen, which are translated from the mRNAs, the HBe antigen that passes through the hepatocyte membrane are released and secreted in a large amount into the blood separately from the Dane particle blood release route. This action is conceived to be an action of escaping the attack of the host immune system against the HBV infection. The HBe antigen, the HBc antigen, and the p22cr antigen can be measured as a hepatitis B core-related antigen (HBcr antigen, HBcrAg) and are used as a marker of virus replication.

Hepatitis B is a kind of viral hepatitis caused by infection with HBV, and the number of infected people is said to be about 240 million worldwide. As described above, in a case of being infected with HBV, Dane particles are released into the blood, and a large amount of the viral protein is secreted into the blood separately from the release route of the Dane particles. It has been pointed out that among viral proteins, the HBs antigen, in particular, may interfere with the elimination of infected cells by the host immune system (Non-Patent Documents 2 and 3). Accordingly, in the medical treatment of hepatitis B, it is conceived important to reduce Dane particles, that is, to suppress the proliferation of the virus and to reduce viral proteins such as the HBs antigen.

Nucleic acid analogs such as entecavir and tenofovir are used as the first-choice drug for hepatitis B. A nucleic acid analog preparation can reduce the number of Dane particles in the blood, that is, suppress the proliferation of the virus, by inhibiting the activity of the HBV polymerase protein and inhibiting the generation of the incomplete circular double-stranded DNA. However, since the nucleic acid analog preparation cannot reduce the HBs antigen, it is difficult to achieve the elimination of infected cells by the host immune system. For this reason, it has been desired to develop a pharmaceutical drug capable of achieving the reduction of the HBs antigen.

The YT-521B homology (YTH) domain-containing protein family is a protein group composed of YTHDC1, YTHDC2, YTHDF1, YTHDF2, and YTHDF3. It has been revealed that all of the above proteins bind to RNA through a recognition sequence containing a methylated adenosine (m6A) on the RNA and regulate biological phenomena such as RNA splicing and protein translation (Non-Patent Document 4). Focusing on the functions of YTH family molecules, it has been disclosed that compounds that inhibit the functions of YTH family molecules are useful for the medical treatment of infectious diseases and cancers (Patent Documents 1 and 2). However, Patent Documents 1 and 2 do not describe the relationship between the inhibition of YTHDC1 and the anti-HBV activity.

### Prior Art Documents

### Patent Documents

Patent Document 1: JP2018-503663A
Patent Document 2: WO2010/134939A

### Non-Patent Documents

Non-Patent Document 1: Tanaka et al., Modem Media, Vol. 54, No. 12, pp. 347 to 352, 2008
Non-Patent Document 2: Brouw et al., Immunology, Vol. 126, pp. 280 to 289, 2008
Non-Patent Document 3: Vanlandschoot et al., Journal of General Virology, Vol. 83, pp. 1281 to 1289, 2002
Non-Patent Document 4: Liao et al., (2018) Genomics Proteomics Bioinformatics, Vol. 16, pp. 99 to 107

### SUMMARY OF THE INVENTION

An object of the present invention is to provide a double-stranded RNA having anti-hepatitis B virus (HBV) activity, and a pharmaceutical composition containing the above-described double-stranded RNA. In addition, another object of the present invention is to provide a double-stranded RNA that inhibits the production of a hepatitis B virus protein, and a pharmaceutical composition containing the above-described double-stranded RNA.

As a result of diligent research on the above-described objects, the inventors of the present invention have found that a specific double-stranded RNA that inhibits the expression of YTHDC1 has an ability to inhibit the production of an HBV protein and have completed the present invention.

That is, the present invention provides the followings.
<1> A double-stranded RNA that inhibits production of a hepatitis B virus protein (HBV protein), in which the double-stranded RNA is formed from a sense strand and an antisense strand, and the sense strand includes a nucleobase sequence of nucleotide, which is represented by any one of SEQ ID NOs: 1, 7, 11, 13, 17, 19, 25, 27, 29, 31, 33, 35, 37, 76, 80, 84, 86, 88, 92, 96, 102, 104, 110, 112, 114, 118, 128, 132, 136, 138, 140, 142, 144, 150, 152, 158, 160, 164, 168, 174, 176, 178, 182, 184, 186, 188, 194, 196, 198, 200, 202, 204, 206, 210, 212, 214, 220, 222, 226, 234, 236, 238, 242, 244, 246, 248, 250, 252, 254, 256, 258, 260, 262, 264, 266, 268, 270, 280, 282, 284, 286, 288, 290, 292, 294, 296, 302, 308, 312, 316, 322, 330, 332, 336, 344, 346, 348, 350, 352, 356, 358, 360, 364, 368, 372, 378, 380, 386, 390, 392, 394, 396, 398, 400, 402, 404, 406, 410, 412, 414, 416, 418, 422, 424, 426, 428, 430, 432, 434, 436, 438, 440, 442, 444, or 446, or includes a nucleobase sequence having a sequence identity of 90% or more with the nucleobase sequence.
<2> The double-stranded RNA according to <1>, in which the antisense strand includes a nucleobase sequence of nucleotide, which is represented by any one of SEQ ID NOs: 2, 8, 12, 14, 18, 20, 26, 28, 30, 32, 34, 36, 38, 77, 81, 85, 87, 89, 93, 97, 103, 105, 111, 113, 115, 119, 129, 133, 137, 139, 141, 143, 145, 151, 153, 159, 161, 165, 169, 175, 177, 179, 183, 185, 187, 189, 195, 197, 199, 201, 203, 205, 207, 211, 213, 215, 221, 223, 227, 235, 237, 239, 243, 245, 247, 249, 251, 253, 255, 257, 259, 261, 263, 265, 267, 269, 271, 281, 283, 285, 287, 289, 291, 293, 295, 297, 303, 309, 313, 317, 323, 331, 333, 337, 345, 347, 349, 351, 353, 357, 359, 361, 365, 369, 373, 379, 381, 387, 391, 393, 395, 397, 399, 401, 403, 405, 407, 411, 413, 415, 417, 419, 423, 425, 427, 429, 431, 433, 435, 437, 439, 441, 443, 445, or 447, or includes a nucleobase sequence having a sequence identity of 90% or more with the nucleobase sequence.
<3> The double-stranded RNA according to <1> or <2>, in which a combination of the sense strand and the antisense strand is selected from the following dsRNAs,
   (a) a dsRNA formed from a sense strand including a nucleobase sequence of a nucleotide represented by SEQ ID NO: 1 and an antisense strand including a nucleobase sequence of a nucleotide represented by SEQ ID NO: 2,
   (b) a dsRNA formed from a sense strand including a nucleobase sequence of a nucleotide represented by SEQ ID NO: 7 and an antisense strand including a nucleobase sequence of a nucleotide represented by SEQ ID NO: 8,
   (c) a dsRNA formed from a sense strand including a nucleobase sequence of a nucleotide represented by SEQ ID NO: 11 and an antisense strand including a nucleobase sequence of a nucleotide represented by SEQ ID NO: 12,
   (d) a dsRNA formed from a sense strand including a nucleobase sequence of a nucleotide represented by SEQ ID NO: 13 and an antisense strand including a nucleobase sequence of a nucleotide represented by SEQ ID NO: 14,
   (e) a dsRNA formed from a sense strand including a nucleobase sequence of a nucleotide represented by SEQ ID NO: 17 and an antisense strand including a nucleobase sequence of a nucleotide represented by SEQ ID NO: 18,
   (f) a dsRNA formed from a sense strand including a nucleobase sequence of a nucleotide represented by SEQ ID NO: 19 and an antisense strand including a nucleobase sequence of a nucleotide represented by SEQ ID NO: 20,
   (g) a dsRNA formed from a sense strand including a nucleobase sequence of a nucleotide represented by SEQ ID NO: 25 and an antisense strand including a nucleobase sequence of a nucleotide represented by SEQ ID NO: 26,
   (h) a dsRNA formed from a sense strand including a nucleobase sequence of a nucleotide represented by SEQ ID NO: 27 and an antisense strand including a nucleobase sequence of a nucleotide represented by SEQ ID NO: 28,
   (i) a dsRNA formed from a sense strand including a nucleobase sequence of a nucleotide represented by SEQ ID NO: 29 and an antisense strand including a nucleobase sequence of a nucleotide represented by SEQ ID NO: 30,
   (j) a dsRNA formed from a sense strand including a nucleobase sequence of a nucleotide represented by SEQ ID NO: 31 and an antisense strand including a nucleobase sequence of a nucleotide represented by SEQ ID NO: 32,
   (k) a dsRNA formed from a sense strand including a nucleobase sequence of a nucleotide represented by SEQ ID NO: 33 and an antisense strand including a nucleobase sequence of a nucleotide represented by SEQ ID NO: 34,
   (1) a dsRNA formed from a sense strand including a nucleobase sequence of a nucleotide represented by SEQ ID NO: 35 and an antisense strand including a nucleobase sequence of a nucleotide represented by SEQ ID NO: 36,
   (m) a dsRNA formed from a sense strand including a nucleobase sequence of a nucleotide represented by SEQ ID NO: 37 and an antisense strand including a nucleobase sequence of a nucleotide represented by SEQ ID NO: 38,
   (n) a dsRNA formed from a sense strand including a nucleobase sequence of a nucleotide represented by SEQ ID NO: 76 and an antisense strand including a nucleobase sequence of a nucleotide represented by SEQ ID NO: 77,
   (o) a dsRNA formed from a sense strand including a nucleobase sequence of a nucleotide represented by SEQ ID NO: 80 and an antisense strand including a nucleobase sequence of a nucleotide represented by SEQ ID NO: 81,
   (p) a dsRNA formed from a sense strand including a nucleobase sequence of a nucleotide represented by SEQ ID NO: 84 and an antisense strand including a nucleobase sequence of a nucleotide represented by SEQ ID NO: 85,
   (q) a dsRNA formed from a sense strand including a nucleobase sequence of a nucleotide represented by SEQ ID NO: 86 and an antisense strand including a nucleobase sequence of a nucleotide represented by SEQ ID NO: 87,
   (r) a dsRNA formed from a sense strand including a nucleobase sequence of a nucleotide represented by SEQ ID NO: 88 and an antisense strand including a nucleobase sequence of a nucleotide represented by SEQ ID NO: 89,
   (s) a dsRNA formed from a sense strand including a nucleobase sequence of a nucleotide represented by SEQ ID NO: 92 and an antisense strand including a nucleobase sequence of a nucleotide represented by SEQ ID NO: 93,
   (t) a dsRNA formed from a sense strand including a nucleobase sequence of a nucleotide represented by SEQ ID NO: 96 and an antisense strand including a nucleobase sequence of a nucleotide represented by SEQ ID NO: 97,
   (u) a dsRNA formed from a sense strand including a nucleobase sequence of a nucleotide represented by SEQ ID NO: 102 and an antisense strand including a nucleobase sequence of a nucleotide represented by SEQ ID NO: 103,
   (v) a dsRNA formed from a sense strand including a nucleobase sequence of a nucleotide represented by SEQ ID NO: 104 and an antisense strand including a nucleobase sequence of a nucleotide represented by SEQ ID NO: 105,
   (w) a dsRNA formed from a sense strand including a nucleobase sequence of a nucleotide represented by SEQ ID NO: 110 and an antisense strand including a nucleobase sequence of a nucleotide represented by SEQ ID NO: 111,
   (x) a dsRNA formed from a sense strand including a nucleobase sequence of a nucleotide represented by SEQ ID NO: 112 and an antisense strand including a nucleobase sequence of a nucleotide represented by SEQ ID NO: 113,
   (y) a dsRNA formed from a sense strand including a nucleobase sequence of a nucleotide represented by SEQ ID NO: 114 and an antisense strand including a nucleobase sequence of a nucleotide represented by SEQ ID NO: 115,
   (z) a dsRNA formed from a sense strand including a nucleobase sequence of a nucleotide represented by SEQ ID NO: 118 and an antisense strand including a nucleobase sequence of a nucleotide represented by SEQ ID NO: 119,
   (aa) a dsRNA formed from a sense strand including a nucleobase sequence of a nucleotide represented by SEQ ID NO: 128 and an antisense strand including a nucleobase sequence of a nucleotide represented by SEQ ID NO: 129,
   (ab) a dsRNA formed from a sense strand including a nucleobase sequence of a nucleotide represented by SEQ ID NO: 132 and an antisense strand including a nucleobase sequence of a nucleotide represented by SEQ ID NO: 133,
   (ac) a dsRNA formed from a sense strand including a nucleobase sequence of a nucleotide represented by SEQ ID NO: 136 and an antisense strand including a nucleobase sequence of a nucleotide represented by SEQ ID NO: 137,
   (ad) a dsRNA formed from a sense strand including a nucleobase sequence of a nucleotide represented by SEQ ID NO: 138 and an antisense strand including a nucleobase sequence of a nucleotide represented by SEQ ID NO: 139,
   (ae) a dsRNA formed from a sense strand including a nucleobase sequence of a nucleotide represented by SEQ ID NO: 140 and an antisense strand including a nucleobase sequence of a nucleotide represented by SEQ ID NO: 141,
   (af) a dsRNA formed from a sense strand including a nucleobase sequence of a nucleotide represented by SEQ ID NO: 142 and an antisense strand including a nucleobase sequence of a nucleotide represented by SEQ ID NO: 143,
   (ag) a dsRNA formed from a sense strand including a nucleobase sequence of a nucleotide represented by SEQ ID NO: 144 and an antisense strand including a nucleobase sequence of a nucleotide represented by SEQ ID NO: 145,
   (ah) a dsRNA formed from a sense strand including a nucleobase sequence of a nucleotide represented by SEQ ID NO: 150 and an antisense strand including a nucleobase sequence of a nucleotide represented by SEQ ID NO: 151,
   (ai) a dsRNA formed from a sense strand including a nucleobase sequence of a nucleotide represented by SEQ ID NO: 152 and an antisense strand including a nucleobase sequence of a nucleotide represented by SEQ ID NO: 153,
   (aj) a dsRNA formed from a sense strand including a nucleobase sequence of a nucleotide represented by SEQ ID NO: 158 and an antisense strand including a nucleobase sequence of a nucleotide represented by SEQ ID NO: 159,
   (ak) a dsRNA formed from a sense strand including a nucleobase sequence of a nucleotide represented by SEQ ID NO: 160 and an antisense strand including a nucleobase sequence of a nucleotide represented by SEQ ID NO: 161,
   (al) a dsRNA formed from a sense strand including a nucleobase sequence of a nucleotide represented by SEQ ID NO: 164 and an antisense strand including a nucleobase sequence of a nucleotide represented by SEQ ID NO: 165,
   (am) a dsRNA formed from a sense strand including a nucleobase sequence of a nucleotide represented by SEQ ID NO: 168 and an antisense strand including a nucleobase sequence of a nucleotide represented by SEQ ID NO: 169,
   (an) a dsRNA formed from a sense strand including a nucleobase sequence of a nucleotide represented by SEQ ID NO: 174 and an antisense strand including a nucleobase sequence of a nucleotide represented by SEQ ID NO: 175,
   (ao) a dsRNA formed from a sense strand including a nucleobase sequence of a nucleotide represented by SEQ ID NO: 176 and an antisense strand including a nucleobase sequence of a nucleotide represented by SEQ ID NO: 177,
   (ap) a dsRNA formed from a sense strand including a nucleobase sequence of a nucleotide represented by SEQ ID NO: 178 and an antisense strand including a nucleobase sequence of a nucleotide represented by SEQ ID NO: 179,
   (aq) a dsRNA formed from a sense strand including a nucleobase sequence of a nucleotide represented by SEQ ID NO: 182 and an antisense strand including a nucleobase sequence of a nucleotide represented by SEQ ID NO: 183,
   (ar) a dsRNA formed from a sense strand including a nucleobase sequence of a nucleotide represented by SEQ ID NO: 184 and an antisense strand including a nucleobase sequence of a nucleotide represented by SEQ ID NO: 185,
   (as) a dsRNA formed from a sense strand including a nucleobase sequence of a nucleotide represented by SEQ ID NO: 186 and an antisense strand including a nucleobase sequence of a nucleotide represented by SEQ ID NO: 187,
   (at) a dsRNA formed from a sense strand including a nucleobase sequence of a nucleotide represented by SEQ ID NO: 188 and an antisense strand including a nucleobase sequence of a nucleotide represented by SEQ ID NO: 189,
   (au) a dsRNA formed from a sense strand including a nucleobase sequence of a nucleotide represented by SEQ ID NO: 194 and an antisense strand including a nucleobase sequence of a nucleotide represented by SEQ ID NO: 195,
   (av) a dsRNA formed from a sense strand including a nucleobase sequence of a nucleotide represented by SEQ ID NO: 196 and an antisense strand including a nucleobase sequence of a nucleotide represented by SEQ ID NO: 197,
   (aw) a dsRNA formed from a sense strand including a nucleobase sequence of a nucleotide represented by SEQ ID NO: 198 and an antisense strand including a nucleobase sequence of a nucleotide represented by SEQ ID NO: 199,
   (ax) a dsRNA formed from a sense strand including a nucleobase sequence of a nucleotide represented by SEQ ID NO: 200 and an antisense strand including a nucleobase sequence of a nucleotide represented by SEQ ID NO: 201,
   (ay) a dsRNA formed from a sense strand including a nucleobase sequence of a nucleotide represented by SEQ ID NO: 202 and an antisense strand including a nucleobase sequence of a nucleotide represented by SEQ ID NO: 203,
   (az) a dsRNA formed from a sense strand including a nucleobase sequence of a nucleotide represented by SEQ ID NO: 204 and an antisense strand including a nucleobase sequence of a nucleotide represented by SEQ ID NO: 205,
   (ba) a dsRNA formed from a sense strand including a nucleobase sequence of a nucleotide represented by SEQ ID NO: 206 and an antisense strand including a nucleobase sequence of a nucleotide represented by SEQ ID NO: 207,
   (bb) a dsRNA formed from a sense strand including a nucleobase sequence of a nucleotide represented by SEQ ID NO: 210 and an antisense strand including a nucleobase sequence of a nucleotide represented by SEQ ID NO: 211,
   (bc) a dsRNA formed from a sense strand including a nucleobase sequence of a nucleotide represented by SEQ ID NO: 212 and an antisense strand including a nucleobase sequence of a nucleotide represented by SEQ ID NO: 213,
   (bd) a dsRNA formed from a sense strand including a nucleobase sequence of a nucleotide represented by SEQ ID NO: 214 and an antisense strand including a nucleobase sequence of a nucleotide represented by SEQ ID NO: 215,
   (be) a dsRNA formed from a sense strand including a nucleobase sequence of a nucleotide represented by SEQ ID NO: 220 and an antisense strand including a nucleobase sequence of a nucleotide represented by SEQ ID NO: 221,
   (bf) a dsRNA formed from a sense strand including a nucleobase sequence of a nucleotide represented by SEQ ID NO: 222 and an antisense strand including a nucleobase sequence of a nucleotide represented by SEQ ID NO: 223,
   (bg) a dsRNA formed from a sense strand including a nucleobase sequence of a nucleotide represented by SEQ ID NO: 226 and an antisense strand including a nucleobase sequence of a nucleotide represented by SEQ ID NO: 227,
   (bh) a dsRNA formed from a sense strand including a nucleobase sequence of a nucleotide represented by SEQ ID NO: 234 and an antisense strand including a nucleobase sequence of a nucleotide represented by SEQ ID NO: 235,
   (bi) a dsRNA formed from a sense strand including a nucleobase sequence of a nucleotide represented by SEQ ID NO: 236 and an antisense strand including a nucleobase sequence of a nucleotide represented by SEQ ID NO: 237,
   (bj) a dsRNA formed from a sense strand including a nucleobase sequence of a nucleotide represented by SEQ ID NO: 238 and an antisense strand including a nucleobase sequence of a nucleotide represented by SEQ ID NO: 239,
   (bk) a dsRNA formed from a sense strand including a nucleobase sequence of a nucleotide represented by SEQ ID NO: 242 and an antisense strand including a nucleobase sequence of a nucleotide represented by SEQ ID NO: 243,
   (bl) a dsRNA formed from a sense strand including a nucleobase sequence of a nucleotide represented by SEQ ID NO: 244 and an antisense strand including a nucleobase sequence of a nucleotide represented by SEQ ID NO: 245,
   (bm) a dsRNA formed from a sense strand including a nucleobase sequence of a nucleotide represented by SEQ ID NO: 246 and an antisense strand including a nucleobase sequence of a nucleotide represented by SEQ ID NO: 247,
   (bn) a dsRNA formed from a sense strand including a nucleobase sequence of a nucleotide represented by SEQ ID NO: 248 and an antisense strand including a nucleobase sequence of a nucleotide represented by SEQ ID NO: 249,
   (bo) a dsRNA formed from a sense strand including a nucleobase sequence of a nucleotide represented by SEQ ID NO: 250 and an antisense strand including a nucleobase sequence of a nucleotide represented by SEQ ID NO: 251,
   (bp) a dsRNA formed from a sense strand including a nucleobase sequence of a nucleotide represented by SEQ ID NO: 252 and an antisense strand including a nucleobase sequence of a nucleotide represented by SEQ ID NO: 253,
   (bq) a dsRNA formed from a sense strand including a nucleobase sequence of a nucleotide represented by SEQ ID NO: 254 and an antisense strand including a nucleobase sequence of a nucleotide represented by SEQ ID NO: 255,
   (br) a dsRNA formed from a sense strand including a nucleobase sequence of a nucleotide represented by SEQ ID NO: 256 and an antisense strand including a nucleobase sequence of a nucleotide represented by SEQ ID NO: 257,
   (bs) a dsRNA formed from a sense strand including a nucleobase sequence of a nucleotide represented by SEQ ID NO: 258 and an antisense strand including a nucleobase sequence of a nucleotide represented by SEQ ID NO: 259,
   (bt) a dsRNA formed from a sense strand including a nucleobase sequence of a nucleotide represented by SEQ ID NO: 260 and an antisense strand including a nucleobase sequence of a nucleotide represented by SEQ ID NO: 261,
   (bu) a dsRNA formed from a sense strand including a nucleobase sequence of a nucleotide represented by SEQ ID NO: 262 and an antisense strand including a nucleobase sequence of a nucleotide represented by SEQ ID NO: 263,
   (bv) a dsRNA formed from a sense strand including a nucleobase sequence of a nucleotide represented by SEQ ID NO: 264 and an antisense strand including a nucleobase sequence of a nucleotide represented by SEQ ID NO: 265,
   (bw) a dsRNA formed from a sense strand including a nucleobase sequence of a nucleotide represented by SEQ ID NO: 266 and an antisense strand including a nucleobase sequence of a nucleotide represented by SEQ ID NO: 267,
   (bx) a dsRNA formed from a sense strand including a nucleobase sequence of a nucleotide represented by SEQ ID NO: 268 and an antisense strand including a nucleobase sequence of a nucleotide represented by SEQ ID NO: 269,
   (by) a dsRNA formed from a sense strand including a nucleobase sequence of a nucleotide represented by SEQ ID NO: 270 and an antisense strand including a nucleobase sequence of a nucleotide represented by SEQ ID NO: 271,
   (bz) a dsRNA formed from a sense strand including a nucleobase sequence of a nucleotide represented by SEQ ID NO: 280 and an antisense strand including a nucleobase sequence of a nucleotide represented by SEQ ID NO: 281,
   (ca) a dsRNA formed from a sense strand including a nucleobase sequence of a nucleotide represented by SEQ ID NO: 282 and an antisense strand including a nucleobase sequence of a nucleotide represented by SEQ ID NO: 283,
   (cb) a dsRNA formed from a sense strand including a nucleobase sequence of a nucleotide represented by SEQ ID NO: 284 and an antisense strand including a nucleobase sequence of a nucleotide represented by SEQ ID NO: 285,
   (cc) a dsRNA formed from a sense strand including a nucleobase sequence of a nucleotide represented by SEQ ID NO: 286 and an antisense strand including a nucleobase sequence of a nucleotide represented by SEQ ID NO: 287,
   (cd) a dsRNA formed from a sense strand including a nucleobase sequence of a nucleotide represented by SEQ ID NO: 288 and an antisense strand including a nucleobase sequence of a nucleotide represented by SEQ ID NO: 289,
   (ce) a dsRNA formed from a sense strand including a nucleobase sequence of a nucleotide represented by SEQ ID NO: 290 and an antisense strand including a nucleobase sequence of a nucleotide represented by SEQ ID NO: 291,
   (cf) a dsRNA formed from a sense strand including a nucleobase sequence of a nucleotide represented by SEQ ID NO: 292 and an antisense strand including a nucleobase sequence of a nucleotide represented by SEQ ID NO: 293,
   (cg) a dsRNA formed from a sense strand including a nucleobase sequence of a nucleotide represented by SEQ ID NO: 294 and an antisense strand including a nucleobase sequence of a nucleotide represented by SEQ ID NO: 295,
   (ch) a dsRNA formed from a sense strand including a nucleobase sequence of a nucleotide represented by SEQ ID NO: 296 and an antisense strand including a nucleobase sequence of a nucleotide represented by SEQ ID NO: 297,
   (ci) a dsRNA formed from a sense strand including a nucleobase sequence of a nucleotide represented by SEQ ID NO: 302 and an antisense strand including a nucleobase sequence of a nucleotide represented by SEQ ID NO: 303,
   (cj) a dsRNA formed from a sense strand including a nucleobase sequence of a nucleotide represented by SEQ ID NO: 308 and an antisense strand including a nucleobase sequence of a nucleotide represented by SEQ ID NO: 309,
   (ck) a dsRNA formed from a sense strand including a nucleobase sequence of a nucleotide represented by SEQ ID NO: 312 and an antisense strand including a nucleobase sequence of a nucleotide represented by SEQ ID NO: 313,
   (cl) a dsRNA formed from a sense strand including a nucleobase sequence of a nucleotide represented by SEQ ID NO: 316 and an antisense strand including a nucleobase sequence of a nucleotide represented by SEQ ID NO: 317,
   (cm) a dsRNA formed from a sense strand including a nucleobase sequence of a nucleotide represented by SEQ ID NO: 322 and an antisense strand including a nucleobase sequence of a nucleotide represented by SEQ ID NO: 323,
   (cn) a dsRNA formed from a sense strand including a nucleobase sequence of a nucleotide represented by SEQ ID NO: 330 and an antisense strand including a nucleobase sequence of a nucleotide represented by SEQ ID NO: 331,
   (co) a dsRNA formed from a sense strand including a nucleobase sequence of a nucleotide represented by SEQ ID NO: 332 and an antisense strand including a nucleobase sequence of a nucleotide represented by SEQ ID NO: 333,
   (cp) a dsRNA formed from a sense strand including a nucleobase sequence of a nucleotide represented by SEQ ID NO: 336 and an antisense strand including a nucleobase sequence of a nucleotide represented by SEQ ID NO: 337,
   (cq) a dsRNA formed from a sense strand including a nucleobase sequence of a nucleotide represented by SEQ ID NO: 344 and an antisense strand including a nucleobase sequence of a nucleotide represented by SEQ ID NO: 345,
   (cr) a dsRNA formed from a sense strand including a nucleobase sequence of a nucleotide represented by SEQ ID NO: 346 and an antisense strand including a nucleobase sequence of a nucleotide represented by SEQ ID NO: 347,
   (cs) a dsRNA formed from a sense strand including a nucleobase sequence of a nucleotide represented by SEQ ID NO: 348 and an antisense strand including a nucleobase sequence of a nucleotide represented by SEQ ID NO: 349,
   (ct) a dsRNA formed from a sense strand including a nucleobase sequence of a nucleotide represented by SEQ ID NO: 350 and an antisense strand including a nucleobase sequence of a nucleotide represented by SEQ ID NO: 351,
   (cu) a dsRNA formed from a sense strand including a nucleobase sequence of a nucleotide represented by SEQ ID NO: 352 and an antisense strand including a nucleobase sequence of a nucleotide represented by SEQ ID NO: 353,
   (cv) a dsRNA formed from a sense strand including a nucleobase sequence of a nucleotide represented by SEQ ID NO: 356 and an antisense strand including a nucleobase sequence of a nucleotide represented by SEQ ID NO: 357,
   (cw) a dsRNA formed from a sense strand including a nucleobase sequence of a nucleotide represented by SEQ ID NO: 358 and an antisense strand including a nucleobase sequence of a nucleotide represented by SEQ ID NO: 359,
   (cx) a dsRNA formed from a sense strand including a nucleobase sequence of a nucleotide represented by SEQ ID NO: 360 and an antisense strand including a nucleobase sequence of a nucleotide represented by SEQ ID NO: 361,
   (cy) a dsRNA formed from a sense strand including a nucleobase sequence of a nucleotide represented by SEQ ID NO: 364 and an antisense strand including a nucleobase sequence of a nucleotide represented by SEQ ID NO: 365,
   (cz) a dsRNA formed from a sense strand including a nucleobase sequence of a nucleotide represented by SEQ ID NO: 368 and an antisense strand including a nucleobase sequence of a nucleotide represented by SEQ ID NO: 369,
   (da) a dsRNA formed from a sense strand including a nucleobase sequence of a nucleotide represented by SEQ ID NO: 372 and an antisense strand including a nucleobase sequence of a nucleotide represented by SEQ ID NO: 373,
   (db) a dsRNA formed from a sense strand including a nucleobase sequence of a nucleotide represented by SEQ ID NO: 378 and an antisense strand including a nucleobase sequence of a nucleotide represented by SEQ ID NO: 379,
   (dc) a dsRNA formed from a sense strand including a nucleobase sequence of a nucleotide represented by SEQ ID NO: 380 and an antisense strand including a nucleobase sequence of a nucleotide represented by SEQ ID NO: 381,
   (dd) a dsRNA formed from a sense strand including a nucleobase sequence of a nucleotide represented by SEQ ID NO: 386 and an antisense strand including a nucleobase sequence of a nucleotide represented by SEQ ID NO: 387,
   (de) a dsRNA formed from a sense strand including a nucleobase sequence of a nucleotide represented by SEQ ID NO: 390 and an antisense strand including a nucleobase sequence of a nucleotide represented by SEQ ID NO: 391,
   (df) a dsRNA formed from a sense strand including a nucleobase sequence of a nucleotide represented by SEQ ID NO: 392 and an antisense strand including a nucleobase sequence of a nucleotide represented by SEQ ID NO: 393,
   (dg) a dsRNA formed from a sense strand including a nucleobase sequence of a nucleotide represented by SEQ ID NO: 394 and an antisense strand including a nucleobase sequence of a nucleotide represented by SEQ ID NO: 395,
   (dh) a dsRNA formed from a sense strand including a nucleobase sequence of a nucleotide represented by SEQ ID NO: 396 and an antisense strand including a nucleobase sequence of a nucleotide represented by SEQ ID NO: 397,
   (di) a dsRNA formed from a sense strand including a nucleobase sequence of a nucleotide represented by SEQ ID NO: 398 and an antisense strand including a nucleobase sequence of a nucleotide represented by SEQ ID NO: 399,
   (dj) a dsRNA formed from a sense strand including a nucleobase sequence of a nucleotide represented by SEQ ID NO: 400 and an antisense strand including a nucleobase sequence of a nucleotide represented by SEQ ID NO: 401,
   (dk) a dsRNA formed from a sense strand including a nucleobase sequence of a nucleotide represented by SEQ ID NO: 402 and an antisense strand including a nucleobase sequence of a nucleotide represented by SEQ ID NO: 403,
   (dl) a dsRNA formed from a sense strand including a nucleobase sequence of a nucleotide represented by SEQ ID NO: 404 and an antisense strand including a nucleobase sequence of a nucleotide represented by SEQ ID NO: 405,
   (dm) a dsRNA formed from a sense strand including a nucleobase sequence of a nucleotide represented by SEQ ID NO: 406 and an antisense strand including a nucleobase sequence of a nucleotide represented by SEQ ID NO: 407,
   (dn) a dsRNA formed from a sense strand including a nucleobase sequence of a nucleotide represented by SEQ ID NO: 410 and an antisense strand including a nucleobase sequence of a nucleotide represented by SEQ ID NO: 411,
   (do) a dsRNA formed from a sense strand including a nucleobase sequence of a nucleotide represented by SEQ ID NO: 412 and an antisense strand including a nucleobase sequence of a nucleotide represented by SEQ ID NO: 413,
   (dp) a dsRNA formed from a sense strand including a nucleobase sequence of a nucleotide represented by SEQ ID NO: 414 and an antisense strand including a nucleobase sequence of a nucleotide represented by SEQ ID NO: 415,
   (dq) a dsRNA formed from a sense strand including a nucleobase sequence of a nucleotide represented by SEQ ID NO: 416 and an antisense strand including a nucleobase sequence of a nucleotide represented by SEQ ID NO: 417,
   (dr) a dsRNA formed from a sense strand including a nucleobase sequence of a nucleotide represented by SEQ ID NO: 418 and an antisense strand including a nucleobase sequence of a nucleotide represented by SEQ ID NO: 419,
   (ds) a dsRNA formed from a sense strand including a nucleobase sequence of a nucleotide represented by SEQ ID NO: 422 and an antisense strand including a nucleobase sequence of a nucleotide represented by SEQ ID NO: 423,
   (dt) a dsRNA formed from a sense strand including a nucleobase sequence of a nucleotide represented by SEQ ID NO: 424 and an antisense strand including a nucleobase sequence of a nucleotide represented by SEQ ID NO: 425,
   (du) a dsRNA formed from a sense strand including a nucleobase sequence of a nucleotide represented by SEQ ID NO: 426 and an antisense strand including a nucleobase sequence of a nucleotide represented by SEQ ID NO: 427,
   (dv) a dsRNA formed from a sense strand including a nucleobase sequence of a nucleotide represented by SEQ ID NO: 428 and an antisense strand including a nucleobase sequence of a nucleotide represented by SEQ ID NO: 429,
   (dw) a dsRNA formed from a sense strand including a nucleobase sequence of a nucleotide represented by SEQ ID NO: 430 and an antisense strand including a nucleobase sequence of a nucleotide represented by SEQ ID NO: 431,
   (dx) a dsRNA formed from a sense strand including a nucleobase sequence of a nucleotide represented by SEQ ID NO: 432 and an antisense strand including a nucleobase sequence of a nucleotide represented by SEQ ID NO: 433,
   (dy) a dsRNA formed from a sense strand including a nucleobase sequence of a nucleotide represented by SEQ ID NO: 434 and an antisense strand including a nucleobase sequence of a nucleotide represented by SEQ ID NO: 435,
   (dz) a dsRNA formed from a sense strand including a nucleobase sequence of a nucleotide represented by SEQ ID NO: 436 and an antisense strand including a nucleobase sequence of a nucleotide represented by SEQ ID NO: 437,
   (ea) a dsRNA formed from a sense strand including a nucleobase sequence of a nucleotide represented by SEQ ID NO: 438 and an antisense strand including a nucleobase sequence of a nucleotide represented by SEQ ID NO: 439,
   (eb) a dsRNA formed from a sense strand including a nucleobase sequence of a nucleotide represented by SEQ ID NO: 440 and an antisense strand including a nucleobase sequence of a nucleotide represented by SEQ ID NO: 441,
   (ec) a dsRNA formed from a sense strand including a nucleobase sequence of a nucleotide represented by SEQ ID NO: 442 and an antisense strand including a nucleobase sequence of a nucleotide represented by SEQ ID NO: 443,
   (ed) a dsRNA formed from a sense strand including a nucleobase sequence of a nucleotide represented by SEQ ID NO: 444 and an antisense strand including a nucleobase sequence of a nucleotide represented by SEQ ID NO: 445, and
   (ee) a dsRNA formed from a sense strand including a nucleobase sequence of a nucleotide represented by SEQ ID NO: 446 and an antisense strand including a nucleobase sequence of a nucleotide represented by SEQ ID NO: 447.
<4> The double-stranded RNA according to any one of <1> to <3>, further including a modified nucleotide.
<5> The double-stranded RNA according to any one of <1> to <4>, in which the HBV protein is an HBs antigen (HBsAg) protein.
<6> The double-stranded RNA according to any one of <1> to <5>, in which the double-stranded RNA reduces an expression level of the HBV DNA.
<7> The double-stranded RNA according to any one of <1> to <6>, in which the double-stranded RNA reduces an expression level of a covalently closed circular DNA (cccDNA).
<8> The double-stranded RNA according to any one of <1> to <7>, in which the double-stranded RNA is an RNA interfering agent.
<9> A pharmaceutical composition comprising the double-stranded RNA according to any one of <1> to <8>.
<10> The pharmaceutical composition according to <9>, in which the double-stranded RNA is encoded in a recombinant expression vector.
<11> The pharmaceutical composition according to < 10>, in which the recombinant expression vector is an adeno-associated virus vector, a retrovirus vector, an adenovirus vector, or an alphavirus vector.
<12> The pharmaceutical composition according to <9>, further including a lipid nanoparticles (LNP) or N-acetylgalactosamine (GalNAc).
<13> A vector including the double-stranded RNA according to any one of <1> to <8>.
<14> The vector according to <13>, in which the vector is an expression vector, and the expression vector is an adeno-associated virus vector, a retrovirus vector, an adenovirus vector, or an alphavirus vector.

In addition, the present invention provides the followings.
<a> A treatment method including administering the double-stranded RNA according to any one of <1> to <8> to a subject who is infected with HBV or suffering a disease associated with HBV infection.
<b> The treatment method according to <a>, in which the disease associated with HBV infection is hepatitis B, liver cirrhosis caused by hepatitis B, or liver cancer caused by hepatitis B or liver cirrhosis.
<c> A kit for treating a disease associated with HBV infection, the kit including:
   the double-stranded RNA according to any one of <1> to <8>; and
   an instruction in which a treatment method for a disease associated with HBV infection is described.
<d> The kit for treating a disease associated with HBV infection according to <c>, in which the treatment method for a disease associated with HBV infection includes administering the double-stranded RNA to a subject who is infected with HBV or suffering a disease associated with HBV infection.
<e> A double-stranded RNA that inhibits the expression of YTHDC1, which is for use in a treatment that inhibits the production of a hepatitis B virus protein (HBV protein).
<f> A YTHDC1 inhibitor for use in the treatment of hepatitis B.
<g> Use of a double-stranded RNA that inhibits the expression of YTHDC1, which is for the manufacture of a double-stranded RNA that inhibits the production of a hepatitis B virus protein (HBV protein).
<h> Use of a YTHDC1 inhibitor for the manufacture of a double-stranded RNA for treating hepatitis B in a subject.

The double-stranded RNA according to the aspect of the present invention has an excellent anti-HBV activity and is useful as an anti-HBV agent.

### BRIEF DESCRIPTION OF THE DRAWINGS

Fig. 1 shows the relative value (%) of cell viability in each of the dsRNA-added wells in each concentration to the negative control dsRNA-added well.
Fig. 2 shows the relative value (%) of the HBs antigen (HBsAg) in each of the dsRNA-added wells in each concentration to the negative control dsRNA-added well.
Fig. 3 shows the relative value (%) of the DNA of HBV (HBV DNA) in each of the dsRNA-added wells in each concentration to the negative control dsRNA-added well.
Fig. 4 shows the relative value (%) of the YTHDC1 expression level in each of the dsRNA-added wells to the YTHDC1 expression level in the negative control dsRNA-added well.
Fig. 5 shows the relative value (%) of cell viability in each of the dsRNA-added wells in each concentration to the negative control dsRNA-added well.
Fig. 6 shows the relative value (%) of the HBs antigen in each of the dsRNA-added wells in each concentration to the negative control dsRNA-added well.
Fig. 7 shows the relative value (%) of the HBV DNA in each of the dsRNA-added wells in each concentration to the negative control dsRNA-added well.
Fig. 8 shows the relative value (%) of the YTHDC1 expression level in each of the dsRNA-added wells to the YTHDC1 expression level in the negative control dsRNA-added well.

### DESCRIPTION OF THE PREFERRED EMBODIMENTS

Hereinafter, the present invention will be described in detail.

In the present specification, unless otherwise specified, each term has the following meaning.

The prevention means the inhibition of the onset of a disease, the reduction of the risk of the onset of a disease, the delay of the onset of a disease, or the like.

The medical treatment means the amelioration, suppression of progression, or the like of a disease or a condition.

The treatment means the prevention, medical treatment, or the like of various diseases.

The effective amount means a therapeutically effective amount, a preventive effective amount, or the like.

The therapeutically effective amount is an amount sufficient to stabilize HBV infection, reduce HBV infection, or eradicate HBV infection in an infected subject. In addition, the preventive effective amount is an amount sufficient for preventing HBV infection in a subject under the risk of infection.

The subject means a mammal including a human.

### <Hepatitis B virus (HBV)>

Hepatitis B virus (HBV) means a virus that has the ability to develop hepatitis B. HBV is currently classified into eight genotypes, from A type to H type, based on the difference in the base sequence, which is derived from the gene mutation. The HBV to be prevented or medically treated with the double-stranded RNA according to the embodiment of the present invention includes all genotypes thereof.

In the present invention, as a disease associated with HBV infection, hepatitis B is mentioned, and examples thereof include acute hepatitis, chronic hepatitis, and fulminant hepatitis. In addition, liver cirrhosis, liver fibrosis, and liver cancer such as hepatocellular carcinoma are also included in a case where the disease is caused by HBV infection to a living body including a human.

### <Hepatitis B virus protein (HBV protein)>

The hepatitis B virus protein (HBV protein) is a protein constituting HBV, and examples thereof include an HBs antigen, an HBc antigen, and an HBe antigen.

In the present invention, the HBV protein is not particularly limited; however, it is preferably an HBs antigen.

A "double-stranded RNA" includes, but is not limited to, a small interfering RNA (siRNA), a short hairpin RNA (an shRNA), or a nucleic acid that interferes with or inhibits the expression of a target gene by RNA interference (RNAi).

An "RNA interfering agent" is a nucleic acid that interferes with or inhibits the expression of a target gene via RNA interference.

The RNA interference is a post-transcriptional target gene silencing technology that uses a double-stranded RNA (a dsRNA) to degrade an mRNA including the same sequence as the dsRNA (Sharp et al., Science, 287, 2431 to 2432, 2000; Zamore et al., Cell, Vol. 101, pp. 25 to 33, 2000; and Tuschl et al., Genes & Development, Vol. 13, 3191 to 3197, 1999). The RNA interference occurs in a case where an endogenous ribonuclease cleaves a long dsRNA to generate a shorter RNA having a length of 21 or 22 nucleotides, called as a small interfering RNA (siRNA). This siRNA mediates the degradation of a target mRNA. Synthesis kits for RNAi are commercially available, for example, from New England Biolabs and Ambion. In an aspect, one or more of the above-described kits for use in antisense RNA can be used.

The double-stranded RNA according to the embodiment of present invention is preferably an RNA interfering agent.

The sense strand in the present invention includes a nucleobase sequence of nucleotide, which is represented by any one of SEQ ID NOs: 1, 7, 11, 13, 17, 19, 25, 27, 29, 31, 33, 35, 37, 76, 80, 84, 86, 88, 92, 96, 102, 104, 110, 112, 114, 118, 128, 132, 136, 138, 140, 142, 144, 150, 152, 158, 160, 164, 168, 174, 176, 178, 182, 184, 186, 188, 194, 196, 198, 200, 202, 204, 206, 210, 212, 214, 220, 222, 226, 234, 236, 238, 242, 244, 246, 248, 250, 252, 254, 256, 258, 260, 262, 264, 266, 268, 270, 280, 282, 284, 286, 288, 290, 292, 294, 296, 302, 308, 312, 316, 322, 330, 332, 336, 344, 346, 348, 350, 352, 356, 358, 360, 364, 368, 372, 378, 380, 386, 390, 392, 394, 396, 398, 400, 402, 404, 406, 410, 412, 414, 416, 418, 422, 424, 426, 428, 430, 432, 434, 436, 438, 440, 442, 444, or 446, or includes a nucleobase sequence having a sequence identity of 90% or more with the nucleobase sequence.

Preferably, the sense strand in the present invention includes a nucleobase sequence of nucleotide, which is represented by any one of SEQ ID NOs: 1, 7, 11, 13, 17, 19, 25, 27, 29, 31, 33, 35, 37, 76, 80, 84, 86, 88, 92, 96, 102, 104, 110, 112, 114, 118, 128, 132, 136, 138, 140, 142, 144, 150, 152, 158, 160, 164, 168, 174, 176, 178, 182, 184, 186, 188, 194, 196, 198, 200, 202, 204, 206, 210, 212, 214, 220, 222, 226, 234, 236, 238, 242, 244, 246, 248, 250, 252, 254, 256, 258, 260, 262, 264, 266, 268, 270, 280, 282, 284, 286, 288, 290, 292, 294, 296, 302, 308, 312, 316, 322, 330, 332, 336, 344, 346, 348, 350, 352, 356, 358, 360, 364, 368, 372, 378, 380, 386, 390, 392, 394, 396, 398, 400, 402, 404, 406, 410, 412, 414, 416, 418, 422, 424, 426, 428, 430, 432, 434, 436, 438, 440, 442, 444, or 446, or includes a nucleobase sequence having a sequence identity of 92% or more with the nucleobase sequence.

More preferably, the sense strand in the present invention includes a nucleobase sequence of nucleotide, which is represented by any one of SEQ ID NOs: 1, 7, 11, 13, 17, 19, 25, 27, 29, 31, 33, 35, 37, 76, 80, 84, 86, 88, 92, 96, 102, 104, 110, 112, 114, 118, 128, 132, 136, 138, 140, 142, 144, 150, 152, 158, 160, 164, 168, 174, 176, 178, 182, 184, 186, 188, 194, 196, 198, 200, 202, 204, 206, 210, 212, 214, 220, 222, 226, 234, 236, 238, 242, 244, 246, 248, 250, 252, 254, 256, 258, 260, 262, 264, 266, 268, 270, 280, 282, 284, 286, 288, 290, 292, 294, 296, 302, 308, 312, 316, 322, 330, 332, 336, 344, 346, 348, 350, 352, 356, 358, 360, 364, 368, 372, 378, 380, 386, 390, 392, 394, 396, 398, 400, 402, 404, 406, 410, 412, 414, 416, 418, 422, 424, 426, 428, 430, 432, 434, 436, 438, 440, 442, 444, or 446, or includes a nucleobase sequence having a sequence identity of 95% or more with the nucleobase sequence.

Still more preferably, the sense strand in the present invention includes a nucleobase sequence of nucleotide, which is represented by any one of SEQ ID NOs: 1, 7, 11, 13, 17, 19, 25, 27, 29, 31, 33, 35, 37, 76, 80, 84, 86, 88, 92, 96, 102, 104, 110, 112, 114, 118, 128, 132, 136, 138, 140, 142, 144, 150, 152, 158, 160, 164, 168, 174, 176, 178, 182, 184, 186, 188, 194, 196, 198, 200, 202, 204, 206, 210, 212, 214, 220, 222, 226, 234, 236, 238, 242, 244, 246, 248, 250, 252, 254, 256, 258, 260, 262, 264, 266, 268, 270, 280, 282, 284, 286, 288, 290, 292, 294, 296, 302, 308, 312, 316, 322, 330, 332, 336, 344, 346, 348, 350, 352, 356, 358, 360, 364, 368, 372, 378, 380, 386, 390, 392, 394, 396, 398, 400, 402, 404, 406, 410, 412, 414, 416, 418, 422, 424, 426, 428, 430, 432, 434, 436, 438, 440, 442, 444, or 446.

The antisense strand in the present invention includes a nucleobase sequence of nucleotide, which is represented by any one of SEQ ID NOs: 2, 8, 12, 14, 18, 20, 26, 28, 30, 32, 34, 36, 38, 77, 81, 85, 87, 89, 93, 97, 103, 105, 111, 113, 115, 119, 129, 133, 137, 139, 141, 143, 145, 151, 153, 159, 161, 165, 169, 175, 177, 179, 183, 185, 187, 189, 195, 197, 199, 201, 203, 205, 207, 211, 213, 215, 221, 223, 227, 235, 237, 239, 243, 245, 247, 249, 251, 253, 255, 257, 259, 261, 263, 265, 267, 269, 271, 281, 283, 285, 287, 289, 291, 293, 295, 297, 303, 309, 313, 317, 323, 331, 333, 337, 345, 347, 349, 351, 353, 357, 359, 361, 365, 369, 373, 379, 381, 387, 391, 393, 395, 397, 399, 401, 403, 405, 407, 411, 413, 415, 417, 419, 423, 425, 427, 429, 431, 433, 435, 437, 439, 441, 443, 445, or 447, or includes a nucleobase sequence having a sequence identity of 90% or more with the nucleobase sequence.

Preferably, the antisense strand in the present invention includes a nucleobase sequence of nucleotide, which is represented by any one of SEQ ID NOs: 2, 8, 12, 14, 18, 20, 26, 28, 30, 32, 34, 36, 38, 77, 81, 85, 87, 89, 93, 97, 103, 105, 111, 113, 115, 119, 129, 133, 137, 139, 141, 143, 145, 151, 153, 159, 161, 165, 169, 175, 177, 179, 183, 185, 187, 189, 195, 197, 199, 201, 203, 205, 207, 211, 213, 215, 221, 223, 227, 235, 237, 239, 243, 245, 247, 249, 251, 253, 255, 257, 259, 261, 263, 265, 267, 269, 271, 281, 283, 285, 287, 289, 291, 293, 295, 297, 303, 309, 313, 317, 323, 331, 333, 337, 345, 347, 349, 351, 353, 357, 359, 361, 365, 369, 373, 379, 381, 387, 391, 393, 395, 397, 399, 401, 403, 405, 407, 411, 413, 415, 417, 419, 423, 425, 427, 429, 431, 433, 435, 437, 439, 441, 443, 445, or 447, or includes a nucleobase sequence having a sequence identity of 92% or more with the nucleobase sequence.

More preferably, the antisense strand in the present invention includes a nucleobase sequence of nucleotide, which is represented by any one of SEQ ID NOs: 2, 8, 12, 14, 18, 20, 26, 28, 30, 32, 34, 36, 38, 77, 81, 85, 87, 89, 93, 97, 103, 105, 111, 113, 115, 119, 129, 133, 137, 139, 141, 143, 145, 151, 153, 159, 161, 165, 169, 175, 177, 179, 183, 185, 187, 189, 195, 197, 199, 201, 203, 205, 207, 211, 213, 215, 221, 223, 227, 235, 237, 239, 243, 245, 247, 249, 251, 253, 255, 257, 259, 261, 263, 265, 267, 269, 271, 281, 283, 285, 287, 289, 291, 293, 295, 297, 303, 309, 313, 317, 323, 331, 333, 337, 345, 347, 349, 351, 353, 357, 359, 361, 365, 369, 373, 379, 381, 387, 391, 393, 395, 397, 399, 401, 403, 405, 407, 411, 413, 415, 417, 419, 423, 425, 427, 429, 431, 433, 435, 437, 439, 441, 443, 445, or 447, or includes a nucleobase sequence having a sequence identity of 95% or more with the nucleobase sequence.

Still more preferably, the antisense strand in the present invention includes a nucleobase sequence of nucleotide, which is represented by any one of SEQ ID NOs: 2, 8, 12, 14, 18, 20, 26, 28, 30, 32, 34, 36, 38, 77, 81, 85, 87, 89, 93, 97, 103, 105, 111, 113, 115, 119, 129, 133, 137, 139, 141, 143, 145, 151, 153, 159, 161, 165, 169, 175, 177, 179, 183, 185, 187, 189, 195, 197, 199, 201, 203, 205, 207, 211, 213, 215, 221, 223, 227, 235, 237, 239, 243, 245, 247, 249, 251, 253, 255, 257, 259, 261, 263, 265, 267, 269, 271, 281, 283, 285, 287, 289, 291, 293, 295, 297, 303, 309, 313, 317, 323, 331, 333, 337, 345, 347, 349, 351, 353, 357, 359, 361, 365, 369, 373, 379, 381, 387, 391, 393, 395, 397, 399, 401, 403, 405, 407, 411, 413, 415, 417, 419, 423, 425, 427, 429, 431, 433, 435, 437, 439, 441, 443, 445, or 447.

The sense strand and the antisense strand in the present invention is 30 nucleotides or less in length, preferably 18 to 25 nucleotides in length, and more preferably 19 to 21 nucleotides in length.

In the double-stranded RNA according to the embodiment of the present invention, a double-stranded RNA is preferably the combination of the sense strand and the antisense strand, which is selected from the following dsRNAs,
(a) a dsRNA formed from a sense strand including a nucleobase sequence of a nucleotide represented by SEQ ID NO: 1 and an antisense strand including a nucleobase sequence of a nucleotide represented by SEQ ID NO: 2,
(b) a dsRNA formed from a sense strand including a nucleobase sequence of a nucleotide represented by SEQ ID NO: 7 and an antisense strand including a nucleobase sequence of a nucleotide represented by SEQ ID NO: 8,
(c) a dsRNA formed from a sense strand including a nucleobase sequence of a nucleotide represented by SEQ ID NO: 11 and an antisense strand including a nucleobase sequence of a nucleotide represented by SEQ ID NO: 12,
(d) a dsRNA formed from a sense strand including a nucleobase sequence of a nucleotide represented by SEQ ID NO: 13 and an antisense strand including a nucleobase sequence of a nucleotide represented by SEQ ID NO: 14,
(e) a dsRNA formed from a sense strand including a nucleobase sequence of a nucleotide represented by SEQ ID NO: 17 and an antisense strand including a nucleobase sequence of a nucleotide represented by SEQ ID NO: 18,
(f) a dsRNA formed from a sense strand including a nucleobase sequence of a nucleotide represented by SEQ ID NO: 19 and an antisense strand including a nucleobase sequence of a nucleotide represented by SEQ ID NO: 20,
(g) a dsRNA formed from a sense strand including a nucleobase sequence of a nucleotide represented by SEQ ID NO: 25 and an antisense strand including a nucleobase sequence of a nucleotide represented by SEQ ID NO: 26,
(h) a dsRNA formed from a sense strand including a nucleobase sequence of a nucleotide represented by SEQ ID NO: 27 and an antisense strand including a nucleobase sequence of a nucleotide represented by SEQ ID NO: 28,
(i) a dsRNA formed from a sense strand including a nucleobase sequence of a nucleotide represented by SEQ ID NO: 29 and an antisense strand including a nucleobase sequence of a nucleotide represented by SEQ ID NO: 30,
(j) a dsRNA formed from a sense strand including a nucleobase sequence of a nucleotide represented by SEQ ID NO: 31 and an antisense strand including a nucleobase sequence of a nucleotide represented by SEQ ID NO: 32,
(k) a dsRNA formed from a sense strand including a nucleobase sequence of a nucleotide represented by SEQ ID NO: 33 and an antisense strand including a nucleobase sequence of a nucleotide represented by SEQ ID NO: 34,
(1) a dsRNA formed from a sense strand including a nucleobase sequence of a nucleotide represented by SEQ ID NO: 35 and an antisense strand including a nucleobase sequence of a nucleotide represented by SEQ ID NO: 36,
(m) a dsRNA formed from a sense strand including a nucleobase sequence of a nucleotide represented by SEQ ID NO: 37 and an antisense strand including a nucleobase sequence of a nucleotide represented by SEQ ID NO: 38,
(n) a dsRNA formed from a sense strand including a nucleobase sequence of a nucleotide represented by SEQ ID NO: 76 and an antisense strand including a nucleobase sequence of a nucleotide represented by SEQ ID NO: 77,
(o) a dsRNA formed from a sense strand including a nucleobase sequence of a nucleotide represented by SEQ ID NO: 80 and an antisense strand including a nucleobase sequence of a nucleotide represented by SEQ ID NO: 81,
(p) a dsRNA formed from a sense strand including a nucleobase sequence of a nucleotide represented by SEQ ID NO: 84 and an antisense strand including a nucleobase sequence of a nucleotide represented by SEQ ID NO: 85,
(q) a dsRNA formed from a sense strand including a nucleobase sequence of a nucleotide represented by SEQ ID NO: 86 and an antisense strand including a nucleobase sequence of a nucleotide represented by SEQ ID NO: 87,
(r) a dsRNA formed from a sense strand including a nucleobase sequence of a nucleotide represented by SEQ ID NO: 88 and an antisense strand including a nucleobase sequence of a nucleotide represented by SEQ ID NO: 89,
(s) a dsRNA formed from a sense strand including a nucleobase sequence of a nucleotide represented by SEQ ID NO: 92 and an antisense strand including a nucleobase sequence of a nucleotide represented by SEQ ID NO: 93,
(t) a dsRNA formed from a sense strand including a nucleobase sequence of a nucleotide represented by SEQ ID NO: 96 and an antisense strand including a nucleobase sequence of a nucleotide represented by SEQ ID NO: 97,
(u) a dsRNA formed from a sense strand including a nucleobase sequence of a nucleotide represented by SEQ ID NO: 102 and an antisense strand including a nucleobase sequence of a nucleotide represented by SEQ ID NO: 103,
(v) a dsRNA formed from a sense strand including a nucleobase sequence of a nucleotide represented by SEQ ID NO: 104 and an antisense strand including a nucleobase sequence of a nucleotide represented by SEQ ID NO: 105,
(w) a dsRNA formed from a sense strand including a nucleobase sequence of a nucleotide represented by SEQ ID NO: 110 and an antisense strand including a nucleobase sequence of a nucleotide represented by SEQ ID NO: 111,
(x) a dsRNA formed from a sense strand including a nucleobase sequence of a nucleotide represented by SEQ ID NO: 112 and an antisense strand including a nucleobase sequence of a nucleotide represented by SEQ ID NO: 113,
(y) a dsRNA formed from a sense strand including a nucleobase sequence of a nucleotide represented by SEQ ID NO: 114 and an antisense strand including a nucleobase sequence of a nucleotide represented by SEQ ID NO: 115,
(z) a dsRNA formed from a sense strand including a nucleobase sequence of a nucleotide represented by SEQ ID NO: 118 and an antisense strand including a nucleobase sequence of a nucleotide represented by SEQ ID NO: 119,
(aa) a dsRNA formed from a sense strand including a nucleobase sequence of a nucleotide represented by SEQ ID NO: 128 and an antisense strand including a nucleobase sequence of a nucleotide represented by SEQ ID NO: 129,
(ab) a dsRNA formed from a sense strand including a nucleobase sequence of a nucleotide represented by SEQ ID NO: 132 and an antisense strand including a nucleobase sequence of a nucleotide represented by SEQ ID NO: 133,
(ac) a dsRNA formed from a sense strand including a nucleobase sequence of a nucleotide represented by SEQ ID NO: 136 and an antisense strand including a nucleobase sequence of a nucleotide represented by SEQ ID NO: 137,
(ad) a dsRNA formed from a sense strand including a nucleobase sequence of a nucleotide represented by SEQ ID NO: 138 and an antisense strand including a nucleobase sequence of a nucleotide represented by SEQ ID NO: 139,
(ae) a dsRNA formed from a sense strand including a nucleobase sequence of a nucleotide represented by SEQ ID NO: 140 and an antisense strand including a nucleobase sequence of a nucleotide represented by SEQ ID NO: 141,
(af) a dsRNA formed from a sense strand including a nucleobase sequence of a nucleotide represented by SEQ ID NO: 142 and an antisense strand including a nucleobase sequence of a nucleotide represented by SEQ ID NO: 143,
(ag) a dsRNA formed from a sense strand including a nucleobase sequence of a nucleotide represented by SEQ ID NO: 144 and an antisense strand including a nucleobase sequence of a nucleotide represented by SEQ ID NO: 145,
(ah) a dsRNA formed from a sense strand including a nucleobase sequence of a nucleotide represented by SEQ ID NO: 150 and an antisense strand including a nucleobase sequence of a nucleotide represented by SEQ ID NO: 151,
(ai) a dsRNA formed from a sense strand including a nucleobase sequence of a nucleotide represented by SEQ ID NO: 152 and an antisense strand including a nucleobase sequence of a nucleotide represented by SEQ ID NO: 153,
(aj) a dsRNA formed from a sense strand including a nucleobase sequence of a nucleotide represented by SEQ ID NO: 158 and an antisense strand including a nucleobase sequence of a nucleotide represented by SEQ ID NO: 159,
(ak) a dsRNA formed from a sense strand including a nucleobase sequence of a nucleotide represented by SEQ ID NO: 160 and an antisense strand including a nucleobase sequence of a nucleotide represented by SEQ ID NO: 161,
(al) a dsRNA formed from a sense strand including a nucleobase sequence of a nucleotide represented by SEQ ID NO: 164 and an antisense strand including a nucleobase sequence of a nucleotide represented by SEQ ID NO: 165,
(am) a dsRNA formed from a sense strand including a nucleobase sequence of a nucleotide represented by SEQ ID NO: 168 and an antisense strand including a nucleobase sequence of a nucleotide represented by SEQ ID NO: 169,
(an) a dsRNA formed from a sense strand including a nucleobase sequence of a nucleotide represented by SEQ ID NO: 174 and an antisense strand including a nucleobase sequence of a nucleotide represented by SEQ ID NO: 175,
(ao) a dsRNA formed from a sense strand including a nucleobase sequence of a nucleotide represented by SEQ ID NO: 176 and an antisense strand including a nucleobase sequence of a nucleotide represented by SEQ ID NO: 177,
(ap) a dsRNA formed from a sense strand including a nucleobase sequence of a nucleotide represented by SEQ ID NO: 178 and an antisense strand including a nucleobase sequence of a nucleotide represented by SEQ ID NO: 179,
(aq) a dsRNA formed from a sense strand including a nucleobase sequence of a nucleotide represented by SEQ ID NO: 182 and an antisense strand including a nucleobase sequence of a nucleotide represented by SEQ ID NO: 183,
(ar) a dsRNA formed from a sense strand including a nucleobase sequence of a nucleotide represented by SEQ ID NO: 184 and an antisense strand including a nucleobase sequence of a nucleotide represented by SEQ ID NO: 185,
(as) a dsRNA formed from a sense strand including a nucleobase sequence of a nucleotide represented by SEQ ID NO: 186 and an antisense strand including a nucleobase sequence of a nucleotide represented by SEQ ID NO: 187,
(at) a dsRNA formed from a sense strand including a nucleobase sequence of a nucleotide represented by SEQ ID NO: 188 and an antisense strand including a nucleobase sequence of a nucleotide represented by SEQ ID NO: 189,
(au) a dsRNA formed from a sense strand including a nucleobase sequence of a nucleotide represented by SEQ ID NO: 194 and an antisense strand including a nucleobase sequence of a nucleotide represented by SEQ ID NO: 195,
(av) a dsRNA formed from a sense strand including a nucleobase sequence of a nucleotide represented by SEQ ID NO: 196 and an antisense strand including a nucleobase sequence of a nucleotide represented by SEQ ID NO: 197,
(aw) a dsRNA formed from a sense strand including a nucleobase sequence of a nucleotide represented by SEQ ID NO: 198 and an antisense strand including a nucleobase sequence of a nucleotide represented by SEQ ID NO: 199,
(ax) a dsRNA formed from a sense strand including a nucleobase sequence of a nucleotide represented by SEQ ID NO: 200 and an antisense strand including a nucleobase sequence of a nucleotide represented by SEQ ID NO: 201,
(ay) a dsRNA formed from a sense strand including a nucleobase sequence of a nucleotide represented by SEQ ID NO: 202 and an antisense strand including a nucleobase sequence of a nucleotide represented by SEQ ID NO: 203,
(az) a dsRNA formed from a sense strand including a nucleobase sequence of a nucleotide represented by SEQ ID NO: 204 and an antisense strand including a nucleobase sequence of a nucleotide represented by SEQ ID NO: 205,
(ba) a dsRNA formed from a sense strand including a nucleobase sequence of a nucleotide represented by SEQ ID NO: 206 and an antisense strand including a nucleobase sequence of a nucleotide represented by SEQ ID NO: 207,
(bb) a dsRNA formed from a sense strand including a nucleobase sequence of a nucleotide represented by SEQ ID NO: 210 and an antisense strand including a nucleobase sequence of a nucleotide represented by SEQ ID NO: 211,
(bc) a dsRNA formed from a sense strand including a nucleobase sequence of a nucleotide represented by SEQ ID NO: 212 and an antisense strand including a nucleobase sequence of a nucleotide represented by SEQ ID NO: 213,
(bd) a dsRNA formed from a sense strand including a nucleobase sequence of a nucleotide represented by SEQ ID NO: 214 and an antisense strand including a nucleobase sequence of a nucleotide represented by SEQ ID NO: 215,
(be) a dsRNA formed from a sense strand including a nucleobase sequence of a nucleotide represented by SEQ ID NO: 220 and an antisense strand including a nucleobase sequence of a nucleotide represented by SEQ ID NO: 221,
(bf) a dsRNA formed from a sense strand including a nucleobase sequence of a nucleotide represented by SEQ ID NO: 222 and an antisense strand including a nucleobase sequence of a nucleotide represented by SEQ ID NO: 223,
(bg) a dsRNA formed from a sense strand including a nucleobase sequence of a nucleotide represented by SEQ ID NO: 226 and an antisense strand including a nucleobase sequence of a nucleotide represented by SEQ ID NO: 227,
(bh) a dsRNA formed from a sense strand including a nucleobase sequence of a nucleotide represented by SEQ ID NO: 234 and an antisense strand including a nucleobase sequence of a nucleotide represented by SEQ ID NO: 235,
(bi) a dsRNA formed from a sense strand including a nucleobase sequence of a nucleotide represented by SEQ ID NO: 236 and an antisense strand including a nucleobase sequence of a nucleotide represented by SEQ ID NO: 237,
(bj) a dsRNA formed from a sense strand including a nucleobase sequence of a nucleotide represented by SEQ ID NO: 238 and an antisense strand including a nucleobase sequence of a nucleotide represented by SEQ ID NO: 239,
(bk) a dsRNA formed from a sense strand including a nucleobase sequence of a nucleotide represented by SEQ ID NO: 242 and an antisense strand including a nucleobase sequence of a nucleotide represented by SEQ ID NO: 243,
(bl) a dsRNA formed from a sense strand including a nucleobase sequence of a nucleotide represented by SEQ ID NO: 244 and an antisense strand including a nucleobase sequence of a nucleotide represented by SEQ ID NO: 245,
(bm) a dsRNA formed from a sense strand including a nucleobase sequence of a nucleotide represented by SEQ ID NO: 246 and an antisense strand including a nucleobase sequence of a nucleotide represented by SEQ ID NO: 247,
(bn) a dsRNA formed from a sense strand including a nucleobase sequence of a nucleotide represented by SEQ ID NO: 248 and an antisense strand including a nucleobase sequence of a nucleotide represented by SEQ ID NO: 249,
(bo) a dsRNA formed from a sense strand including a nucleobase sequence of a nucleotide represented by SEQ ID NO: 250 and an antisense strand including a nucleobase sequence of a nucleotide represented by SEQ ID NO: 251,
(bp) a dsRNA formed from a sense strand including a nucleobase sequence of a nucleotide represented by SEQ ID NO: 252 and an antisense strand including a nucleobase sequence of a nucleotide represented by SEQ ID NO: 253,
(bq) a dsRNA formed from a sense strand including a nucleobase sequence of a nucleotide represented by SEQ ID NO: 254 and an antisense strand including a nucleobase sequence of a nucleotide represented by SEQ ID NO: 255,
(br) a dsRNA formed from a sense strand including a nucleobase sequence of a nucleotide represented by SEQ ID NO: 256 and an antisense strand including a nucleobase sequence of a nucleotide represented by SEQ ID NO: 257,
(bs) a dsRNA formed from a sense strand including a nucleobase sequence of a nucleotide represented by SEQ ID NO: 258 and an antisense strand including a nucleobase sequence of a nucleotide represented by SEQ ID NO: 259,
(bt) a dsRNA formed from a sense strand including a nucleobase sequence of a nucleotide represented by SEQ ID NO: 260 and an antisense strand including a nucleobase sequence of a nucleotide represented by SEQ ID NO: 261,
(bu) a dsRNA formed from a sense strand including a nucleobase sequence of a nucleotide represented by SEQ ID NO: 262 and an antisense strand including a nucleobase sequence of a nucleotide represented by SEQ ID NO: 263,
(bv) a dsRNA formed from a sense strand including a nucleobase sequence of a nucleotide represented by SEQ ID NO: 264 and an antisense strand including a nucleobase sequence of a nucleotide represented by SEQ ID NO: 265,
(bw) a dsRNA formed from a sense strand including a nucleobase sequence of a nucleotide represented by SEQ ID NO: 266 and an antisense strand including a nucleobase sequence of a nucleotide represented by SEQ ID NO: 267,
(bx) a dsRNA formed from a sense strand including a nucleobase sequence of a nucleotide represented by SEQ ID NO: 268 and an antisense strand including a nucleobase sequence of a nucleotide represented by SEQ ID NO: 269,
(by) a dsRNA formed from a sense strand including a nucleobase sequence of a nucleotide represented by SEQ ID NO: 270 and an antisense strand including a nucleobase sequence of a nucleotide represented by SEQ ID NO: 271,
(bz) a dsRNA formed from a sense strand including a nucleobase sequence of a nucleotide represented by SEQ ID NO: 280 and an antisense strand including a nucleobase sequence of a nucleotide represented by SEQ ID NO: 281,
(ca) a dsRNA formed from a sense strand including a nucleobase sequence of a nucleotide represented by SEQ ID NO: 282 and an antisense strand including a nucleobase sequence of a nucleotide represented by SEQ ID NO: 283,
(cb) a dsRNA formed from a sense strand including a nucleobase sequence of a nucleotide represented by SEQ ID NO: 284 and an antisense strand including a nucleobase sequence of a nucleotide represented by SEQ ID NO: 285,
(cc) a dsRNA formed from a sense strand including a nucleobase sequence of a nucleotide represented by SEQ ID NO: 286 and an antisense strand including a nucleobase sequence of a nucleotide represented by SEQ ID NO: 287,
(cd) a dsRNA formed from a sense strand including a nucleobase sequence of a nucleotide represented by SEQ ID NO: 288 and an antisense strand including a nucleobase sequence of a nucleotide represented by SEQ ID NO: 289,
(ce) a dsRNA formed from a sense strand including a nucleobase sequence of a nucleotide represented by SEQ ID NO: 290 and an antisense strand including a nucleobase sequence of a nucleotide represented by SEQ ID NO: 291,
(cf) a dsRNA formed from a sense strand including a nucleobase sequence of a nucleotide represented by SEQ ID NO: 292 and an antisense strand including a nucleobase sequence of a nucleotide represented by SEQ ID NO: 293,
(cg) a dsRNA formed from a sense strand including a nucleobase sequence of a nucleotide represented by SEQ ID NO: 294 and an antisense strand including a nucleobase sequence of a nucleotide represented by SEQ ID NO: 295,
(ch) a dsRNA formed from a sense strand including a nucleobase sequence of a nucleotide represented by SEQ ID NO: 296 and an antisense strand including a nucleobase sequence of a nucleotide represented by SEQ ID NO: 297,
(ci) a dsRNA formed from a sense strand including a nucleobase sequence of a nucleotide represented by SEQ ID NO: 302 and an antisense strand including a nucleobase sequence of a nucleotide represented by SEQ ID NO: 303,
(cj) a dsRNA formed from a sense strand including a nucleobase sequence of a nucleotide represented by SEQ ID NO: 308 and an antisense strand including a nucleobase sequence of a nucleotide represented by SEQ ID NO: 309,
(ck) a dsRNA formed from a sense strand including a nucleobase sequence of a nucleotide represented by SEQ ID NO: 312 and an antisense strand including a nucleobase sequence of a nucleotide represented by SEQ ID NO: 313,
(cl) a dsRNA formed from a sense strand including a nucleobase sequence of a nucleotide represented by SEQ ID NO: 316 and an antisense strand including a nucleobase sequence of a nucleotide represented by SEQ ID NO: 317,
(cm) a dsRNA formed from a sense strand including a nucleobase sequence of a nucleotide represented by SEQ ID NO: 322 and an antisense strand including a nucleobase sequence of a nucleotide represented by SEQ ID NO: 323,
(cn) a dsRNA formed from a sense strand including a nucleobase sequence of a nucleotide represented by SEQ ID NO: 330 and an antisense strand including a nucleobase sequence of a nucleotide represented by SEQ ID NO: 331,
(co) a dsRNA formed from a sense strand including a nucleobase sequence of a nucleotide represented by SEQ ID NO: 332 and an antisense strand including a nucleobase sequence of a nucleotide represented by SEQ ID NO: 333,
(cp) a dsRNA formed from a sense strand including a nucleobase sequence of a nucleotide represented by SEQ ID NO: 336 and an antisense strand including a nucleobase sequence of a nucleotide represented by SEQ ID NO: 337,
(cq) a dsRNA formed from a sense strand including a nucleobase sequence of a nucleotide represented by SEQ ID NO: 344 and an antisense strand including a nucleobase sequence of a nucleotide represented by SEQ ID NO: 345,
(cr) a dsRNA formed from a sense strand including a nucleobase sequence of a nucleotide represented by SEQ ID NO: 346 and an antisense strand including a nucleobase sequence of a nucleotide represented by SEQ ID NO: 347,
(cs) a dsRNA formed from a sense strand including a nucleobase sequence of a nucleotide represented by SEQ ID NO: 348 and an antisense strand including a nucleobase sequence of a nucleotide represented by SEQ ID NO: 349,
(ct) a dsRNA formed from a sense strand including a nucleobase sequence of a nucleotide represented by SEQ ID NO: 350 and an antisense strand including a nucleobase sequence of a nucleotide represented by SEQ ID NO: 351,
(cu) a dsRNA formed from a sense strand including a nucleobase sequence of a nucleotide represented by SEQ ID NO: 352 and an antisense strand including a nucleobase sequence of a nucleotide represented by SEQ ID NO: 353,
(cv) a dsRNA formed from a sense strand including a nucleobase sequence of a nucleotide represented by SEQ ID NO: 356 and an antisense strand including a nucleobase sequence of a nucleotide represented by SEQ ID NO: 357,
(cw) a dsRNA formed from a sense strand including a nucleobase sequence of a nucleotide represented by SEQ ID NO: 358 and an antisense strand including a nucleobase sequence of a nucleotide represented by SEQ ID NO: 359,
(cx) a dsRNA formed from a sense strand including a nucleobase sequence of a nucleotide represented by SEQ ID NO: 360 and an antisense strand including a nucleobase sequence of a nucleotide represented by SEQ ID NO: 361,
(cy) a dsRNA formed from a sense strand including a nucleobase sequence of a nucleotide represented by SEQ ID NO: 364 and an antisense strand including a nucleobase sequence of a nucleotide represented by SEQ ID NO: 365,
(cz) a dsRNA formed from a sense strand including a nucleobase sequence of a nucleotide represented by SEQ ID NO: 368 and an antisense strand including a nucleobase sequence of a nucleotide represented by SEQ ID NO: 369,
(da) a dsRNA formed from a sense strand including a nucleobase sequence of a nucleotide represented by SEQ ID NO: 372 and an antisense strand including a nucleobase sequence of a nucleotide represented by SEQ ID NO: 373,
(db) a dsRNA formed from a sense strand including a nucleobase sequence of a nucleotide represented by SEQ ID NO: 378 and an antisense strand including a nucleobase sequence of a nucleotide represented by SEQ ID NO: 379,
(dc) a dsRNA formed from a sense strand including a nucleobase sequence of a nucleotide represented by SEQ ID NO: 380 and an antisense strand including a nucleobase sequence of a nucleotide represented by SEQ ID NO: 381,
(dd) a dsRNA formed from a sense strand including a nucleobase sequence of a nucleotide represented by SEQ ID NO: 386 and an antisense strand including a nucleobase sequence of a nucleotide represented by SEQ ID NO: 387,
(de) a dsRNA formed from a sense strand including a nucleobase sequence of a nucleotide represented by SEQ ID NO: 390 and an antisense strand including a nucleobase sequence of a nucleotide represented by SEQ ID NO: 391,
(df) a dsRNA formed from a sense strand including a nucleobase sequence of a nucleotide represented by SEQ ID NO: 392 and an antisense strand including a nucleobase sequence of a nucleotide represented by SEQ ID NO: 393,
(dg) a dsRNA formed from a sense strand including a nucleobase sequence of a nucleotide represented by SEQ ID NO: 394 and an antisense strand including a nucleobase sequence of a nucleotide represented by SEQ ID NO: 395,
(dh) a dsRNA formed from a sense strand including a nucleobase sequence of a nucleotide represented by SEQ ID NO: 396 and an antisense strand including a nucleobase sequence of a nucleotide represented by SEQ ID NO: 397,
(di) a dsRNA formed from a sense strand including a nucleobase sequence of a nucleotide represented by SEQ ID NO: 398 and an antisense strand including a nucleobase sequence of a nucleotide represented by SEQ ID NO: 399,
(dj) a dsRNA formed from a sense strand including a nucleobase sequence of a nucleotide represented by SEQ ID NO: 400 and an antisense strand including a nucleobase sequence of a nucleotide represented by SEQ ID NO: 401,
(dk) a dsRNA formed from a sense strand including a nucleobase sequence of a nucleotide represented by SEQ ID NO: 402 and an antisense strand including a nucleobase sequence of a nucleotide represented by SEQ ID NO: 403,
(dl) a dsRNA formed from a sense strand including a nucleobase sequence of a nucleotide represented by SEQ ID NO: 404 and an antisense strand including a nucleobase sequence of a nucleotide represented by SEQ ID NO: 405,
(dm) a dsRNA formed from a sense strand including a nucleobase sequence of a nucleotide represented by SEQ ID NO: 406 and an antisense strand including a nucleobase sequence of a nucleotide represented by SEQ ID NO: 407,
(dn) a dsRNA formed from a sense strand including a nucleobase sequence of a nucleotide represented by SEQ ID NO: 410 and an antisense strand including a nucleobase sequence of a nucleotide represented by SEQ ID NO: 411,
(do) a dsRNA formed from a sense strand including a nucleobase sequence of a nucleotide represented by SEQ ID NO: 412 and an antisense strand including a nucleobase sequence of a nucleotide represented by SEQ ID NO: 413,
(dp) a dsRNA formed from a sense strand including a nucleobase sequence of a nucleotide represented by SEQ ID NO: 414 and an antisense strand including a nucleobase sequence of a nucleotide represented by SEQ ID NO: 415,
(dq) a dsRNA formed from a sense strand including a nucleobase sequence of a nucleotide represented by SEQ ID NO: 416 and an antisense strand including a nucleobase sequence of a nucleotide represented by SEQ ID NO: 417,
(dr) a dsRNA formed from a sense strand including a nucleobase sequence of a nucleotide represented by SEQ ID NO: 418 and an antisense strand including a nucleobase sequence of a nucleotide represented by SEQ ID NO: 419,
(ds) a dsRNA formed from a sense strand including a nucleobase sequence of a nucleotide represented by SEQ ID NO: 422 and an antisense strand including a nucleobase sequence of a nucleotide represented by SEQ ID NO: 423,
(dt) a dsRNA formed from a sense strand including a nucleobase sequence of a nucleotide represented by SEQ ID NO: 424 and an antisense strand including a nucleobase sequence of a nucleotide represented by SEQ ID NO: 425,
(du) a dsRNA formed from a sense strand including a nucleobase sequence of a nucleotide represented by SEQ ID NO: 426 and an antisense strand including a nucleobase sequence of a nucleotide represented by SEQ ID NO: 427,
(dv) a dsRNA formed from a sense strand including a nucleobase sequence of a nucleotide represented by SEQ ID NO: 428 and an antisense strand including a nucleobase sequence of a nucleotide represented by SEQ ID NO: 429,
(dw) a dsRNA formed from a sense strand including a nucleobase sequence of a nucleotide represented by SEQ ID NO: 430 and an antisense strand including a nucleobase sequence of a nucleotide represented by SEQ ID NO: 431,
(dx) a dsRNA formed from a sense strand including a nucleobase sequence of a nucleotide represented by SEQ ID NO: 432 and an antisense strand including a nucleobase sequence of a nucleotide represented by SEQ ID NO: 433,
(dy) a dsRNA formed from a sense strand including a nucleobase sequence of a nucleotide represented by SEQ ID NO: 434 and an antisense strand including a nucleobase sequence of a nucleotide represented by SEQ ID NO: 435,
(dz) a dsRNA formed from a sense strand including a nucleobase sequence of a nucleotide represented by SEQ ID NO: 436 and an antisense strand including a nucleobase sequence of a nucleotide represented by SEQ ID NO: 437,
(ea) a dsRNA formed from a sense strand including a nucleobase sequence of a nucleotide represented by SEQ ID NO: 438 and an antisense strand including a nucleobase sequence of a nucleotide represented by SEQ ID NO: 439,
(eb) a dsRNA formed from a sense strand including a nucleobase sequence of a nucleotide represented by SEQ ID NO: 440 and an antisense strand including a nucleobase sequence of a nucleotide represented by SEQ ID NO: 441,
(ec) a dsRNA formed from a sense strand including a nucleobase sequence of a nucleotide represented by SEQ ID NO: 442 and an antisense strand including a nucleobase sequence of a nucleotide represented by SEQ ID NO: 443,
(ed) a dsRNA formed from a sense strand including a nucleobase sequence of a nucleotide represented by SEQ ID NO: 444 and an antisense strand including a nucleobase sequence of a nucleotide represented by SEQ ID NO: 445, and
(ee) a dsRNA formed from a sense strand including a nucleobase sequence of a nucleotide represented by SEQ ID NO: 446 and an antisense strand including a nucleobase sequence of a nucleotide represented by SEQ ID NO: 447.

In the double-stranded RNA according to the embodiment of the present invention, a double-stranded RNA is more preferably the combination of the sense strand and the antisense strand, which is selected from the following dsRNAs,
(a) a dsRNA formed from a sense strand including a nucleobase sequence of a nucleotide represented by SEQ ID NO: 1 and an antisense strand including a nucleobase sequence of a nucleotide represented by SEQ ID NO: 2,
(b) a dsRNA formed from a sense strand including a nucleobase sequence of a nucleotide represented by SEQ ID NO: 7 and an antisense strand including a nucleobase sequence of a nucleotide represented by SEQ ID NO: 8,
(e) a dsRNA formed from a sense strand including a nucleobase sequence of a nucleotide represented by SEQ ID NO: 17 and an antisense strand including a nucleobase sequence of a nucleotide represented by SEQ ID NO: 18,
(g) a dsRNA formed from a sense strand including a nucleobase sequence of a nucleotide represented by SEQ ID NO: 25 and an antisense strand including a nucleobase sequence of a nucleotide represented by SEQ ID NO: 26,
(h) a dsRNA formed from a sense strand including a nucleobase sequence of a nucleotide represented by SEQ ID NO: 27 and an antisense strand including a nucleobase sequence of a nucleotide represented by SEQ ID NO: 28,
(j) a dsRNA formed from a sense strand including a nucleobase sequence of a nucleotide represented by SEQ ID NO: 31 and an antisense strand including a nucleobase sequence of a nucleotide represented by SEQ ID NO: 32,
(k) a dsRNA formed from a sense strand including a nucleobase sequence of a nucleotide represented by SEQ ID NO: 33 and an antisense strand including a nucleobase sequence of a nucleotide represented by SEQ ID NO: 34,
(n) a dsRNA formed from a sense strand including a nucleobase sequence of a nucleotide represented by SEQ ID NO: 76 and an antisense strand including a nucleobase sequence of a nucleotide represented by SEQ ID NO: 77,
(p) a dsRNA formed from a sense strand including a nucleobase sequence of a nucleotide represented by SEQ ID NO: 84 and an antisense strand including a nucleobase sequence of a nucleotide represented by SEQ ID NO: 85,
(q) a dsRNA formed from a sense strand including a nucleobase sequence of a nucleotide represented by SEQ ID NO: 86 and an antisense strand including a nucleobase sequence of a nucleotide represented by SEQ ID NO: 87,
(r) a dsRNA formed from a sense strand including a nucleobase sequence of a nucleotide represented by SEQ ID NO: 88 and an antisense strand including a nucleobase sequence of a nucleotide represented by SEQ ID NO: 89,
(s) a dsRNA formed from a sense strand including a nucleobase sequence of a nucleotide represented by SEQ ID NO: 92 and an antisense strand including a nucleobase sequence of a nucleotide represented by SEQ ID NO: 93,
(y) a dsRNA formed from a sense strand including a nucleobase sequence of a nucleotide represented by SEQ ID NO: 114 and an antisense strand including a nucleobase sequence of a nucleotide represented by SEQ ID NO: 115,
(z) a dsRNA formed from a sense strand including a nucleobase sequence of a nucleotide represented by SEQ ID NO: 118 and an antisense strand including a nucleobase sequence of a nucleotide represented by SEQ ID NO: 119,
(ab) a dsRNA formed from a sense strand including a nucleobase sequence of a nucleotide represented by SEQ ID NO: 132 and an antisense strand including a nucleobase sequence of a nucleotide represented by SEQ ID NO: 133,
(ad) a dsRNA formed from a sense strand including a nucleobase sequence of a nucleotide represented by SEQ ID NO: 138 and an antisense strand including a nucleobase sequence of a nucleotide represented by SEQ ID NO: 139,
(ag) a dsRNA formed from a sense strand including a nucleobase sequence of a nucleotide represented by SEQ ID NO: 144 and an antisense strand including a nucleobase sequence of a nucleotide represented by SEQ ID NO: 145,
(aj) a dsRNA formed from a sense strand including a nucleobase sequence of a nucleotide represented by SEQ ID NO: 158 and an antisense strand including a nucleobase sequence of a nucleotide represented by SEQ ID NO: 159,
(ak) a dsRNA formed from a sense strand including a nucleobase sequence of a nucleotide represented by SEQ ID NO: 160 and an antisense strand including a nucleobase sequence of a nucleotide represented by SEQ ID NO: 161,
(al) a dsRNA formed from a sense strand including a nucleobase sequence of a nucleotide represented by SEQ ID NO: 164 and an antisense strand including a nucleobase sequence of a nucleotide represented by SEQ ID NO: 165,
(am) a dsRNA formed from a sense strand including a nucleobase sequence of a nucleotide represented by SEQ ID NO: 168 and an antisense strand including a nucleobase sequence of a nucleotide represented by SEQ ID NO: 169,
(an) a dsRNA formed from a sense strand including a nucleobase sequence of a nucleotide represented by SEQ ID NO: 174 and an antisense strand including a nucleobase sequence of a nucleotide represented by SEQ ID NO: 175,
(ap) a dsRNA formed from a sense strand including a nucleobase sequence of a nucleotide represented by SEQ ID NO: 178 and an antisense strand including a nucleobase sequence of a nucleotide represented by SEQ ID NO: 179,
(aq) a dsRNA formed from a sense strand including a nucleobase sequence of a nucleotide represented by SEQ ID NO: 182 and an antisense strand including a nucleobase sequence of a nucleotide represented by SEQ ID NO: 183,
(ar) a dsRNA formed from a sense strand including a nucleobase sequence of a nucleotide represented by SEQ ID NO: 184 and an antisense strand including a nucleobase sequence of a nucleotide represented by SEQ ID NO: 185,
(as) a dsRNA formed from a sense strand including a nucleobase sequence of a nucleotide represented by SEQ ID NO: 186 and an antisense strand including a nucleobase sequence of a nucleotide represented by SEQ ID NO: 187,
(au) a dsRNA formed from a sense strand including a nucleobase sequence of a nucleotide represented by SEQ ID NO: 194 and an antisense strand including a nucleobase sequence of a nucleotide represented by SEQ ID NO: 195,
(aw) a dsRNA formed from a sense strand including a nucleobase sequence of a nucleotide represented by SEQ ID NO: 198 and an antisense strand including a nucleobase sequence of a nucleotide represented by SEQ ID NO: 199,
(ax) a dsRNA formed from a sense strand including a nucleobase sequence of a nucleotide represented by SEQ ID NO: 200 and an antisense strand including a nucleobase sequence of a nucleotide represented by SEQ ID NO: 201,
(ay) a dsRNA formed from a sense strand including a nucleobase sequence of a nucleotide represented by SEQ ID NO: 202 and an antisense strand including a nucleobase sequence of a nucleotide represented by SEQ ID NO: 203,
(bb) a dsRNA formed from a sense strand including a nucleobase sequence of a nucleotide represented by SEQ ID NO: 210 and an antisense strand including a nucleobase sequence of a nucleotide represented by SEQ ID NO: 211,
(bc) a dsRNA formed from a sense strand including a nucleobase sequence of a nucleotide represented by SEQ ID NO: 212 and an antisense strand including a nucleobase sequence of a nucleotide represented by SEQ ID NO: 213,
(bd) a dsRNA formed from a sense strand including a nucleobase sequence of a nucleotide represented by SEQ ID NO: 214 and an antisense strand including a nucleobase sequence of a nucleotide represented by SEQ ID NO: 215,
(be) a dsRNA formed from a sense strand including a nucleobase sequence of a nucleotide represented by SEQ ID NO: 220 and an antisense strand including a nucleobase sequence of a nucleotide represented by SEQ ID NO: 221,
(bf) a dsRNA formed from a sense strand including a nucleobase sequence of a nucleotide represented by SEQ ID NO: 222 and an antisense strand including a nucleobase sequence of a nucleotide represented by SEQ ID NO: 223,
(bh) a dsRNA formed from a sense strand including a nucleobase sequence of a nucleotide represented by SEQ ID NO: 234 and an antisense strand including a nucleobase sequence of a nucleotide represented by SEQ ID NO: 235,
(bk) a dsRNA formed from a sense strand including a nucleobase sequence of a nucleotide represented by SEQ ID NO: 242 and an antisense strand including a nucleobase sequence of a nucleotide represented by SEQ ID NO: 243,
(bl) a dsRNA formed from a sense strand including a nucleobase sequence of a nucleotide represented by SEQ ID NO: 244 and an antisense strand including a nucleobase sequence of a nucleotide represented by SEQ ID NO: 245,
(bn) a dsRNA formed from a sense strand including a nucleobase sequence of a nucleotide represented by SEQ ID NO: 248 and an antisense strand including a nucleobase sequence of a nucleotide represented by SEQ ID NO: 249,
(bo) a dsRNA formed from a sense strand including a nucleobase sequence of a nucleotide represented by SEQ ID NO: 250 and an antisense strand including a nucleobase sequence of a nucleotide represented by SEQ ID NO: 251,
(bp) a dsRNA formed from a sense strand including a nucleobase sequence of a nucleotide represented by SEQ ID NO: 252 and an antisense strand including a nucleobase sequence of a nucleotide represented by SEQ ID NO: 253,
(bq) a dsRNA formed from a sense strand including a nucleobase sequence of a nucleotide represented by SEQ ID NO: 254 and an antisense strand including a nucleobase sequence of a nucleotide represented by SEQ ID NO: 255,
(br) a dsRNA formed from a sense strand including a nucleobase sequence of a nucleotide represented by SEQ ID NO: 256 and an antisense strand including a nucleobase sequence of a nucleotide represented by SEQ ID NO: 257,
(bs) a dsRNA formed from a sense strand including a nucleobase sequence of a nucleotide represented by SEQ ID NO: 258 and an antisense strand including a nucleobase sequence of a nucleotide represented by SEQ ID NO: 259,
(bt) a dsRNA formed from a sense strand including a nucleobase sequence of a nucleotide represented by SEQ ID NO: 260 and an antisense strand including a nucleobase sequence of a nucleotide represented by SEQ ID NO: 261,
(bu) a dsRNA formed from a sense strand including a nucleobase sequence of a nucleotide represented by SEQ ID NO: 262 and an antisense strand including a nucleobase sequence of a nucleotide represented by SEQ ID NO: 263,
(bv) a dsRNA formed from a sense strand including a nucleobase sequence of a nucleotide represented by SEQ ID NO: 264 and an antisense strand including a nucleobase sequence of a nucleotide represented by SEQ ID NO: 265,
(bz) a dsRNA formed from a sense strand including a nucleobase sequence of a nucleotide represented by SEQ ID NO: 280 and an antisense strand including a nucleobase sequence of a nucleotide represented by SEQ ID NO: 281,
(ca) a dsRNA formed from a sense strand including a nucleobase sequence of a nucleotide represented by SEQ ID NO: 282 and an antisense strand including a nucleobase sequence of a nucleotide represented by SEQ ID NO: 283,
(cb) a dsRNA formed from a sense strand including a nucleobase sequence of a nucleotide represented by SEQ ID NO: 284 and an antisense strand including a nucleobase sequence of a nucleotide represented by SEQ ID NO: 285,
(cc) a dsRNA formed from a sense strand including a nucleobase sequence of a nucleotide represented by SEQ ID NO: 286 and an antisense strand including a nucleobase sequence of a nucleotide represented by SEQ ID NO: 287,
(cd) a dsRNA formed from a sense strand including a nucleobase sequence of a nucleotide represented by SEQ ID NO: 288 and an antisense strand including a nucleobase sequence of a nucleotide represented by SEQ ID NO: 289,
(ce) a dsRNA formed from a sense strand including a nucleobase sequence of a nucleotide represented by SEQ ID NO: 290 and an antisense strand including a nucleobase sequence of a nucleotide represented by SEQ ID NO: 291,
(cg) a dsRNA formed from a sense strand including a nucleobase sequence of a nucleotide represented by SEQ ID NO: 294 and an antisense strand including a nucleobase sequence of a nucleotide represented by SEQ ID NO: 295,
(ch) a dsRNA formed from a sense strand including a nucleobase sequence of a nucleotide represented by SEQ ID NO: 296 and an antisense strand including a nucleobase sequence of a nucleotide represented by SEQ ID NO: 297,
(ci) a dsRNA formed from a sense strand including a nucleobase sequence of a nucleotide represented by SEQ ID NO: 302 and an antisense strand including a nucleobase sequence of a nucleotide represented by SEQ ID NO: 303,
(cj) a dsRNA formed from a sense strand including a nucleobase sequence of a nucleotide represented by SEQ ID NO: 308 and an antisense strand including a nucleobase sequence of a nucleotide represented by SEQ ID NO: 309,
(cl) a dsRNA formed from a sense strand including a nucleobase sequence of a nucleotide represented by SEQ ID NO: 316 and an antisense strand including a nucleobase sequence of a nucleotide represented by SEQ ID NO: 317,
(cq) a dsRNA formed from a sense strand including a nucleobase sequence of a nucleotide represented by SEQ ID NO: 344 and an antisense strand including a nucleobase sequence of a nucleotide represented by SEQ ID NO: 345,
(cs) a dsRNA formed from a sense strand including a nucleobase sequence of a nucleotide represented by SEQ ID NO: 348 and an antisense strand including a nucleobase sequence of a nucleotide represented by SEQ ID NO: 349,
(ct) a dsRNA formed from a sense strand including a nucleobase sequence of a nucleotide represented by SEQ ID NO: 350 and an antisense strand including a nucleobase sequence of a nucleotide represented by SEQ ID NO: 351,
(cu) a dsRNA formed from a sense strand including a nucleobase sequence of a nucleotide represented by SEQ ID NO: 352 and an antisense strand including a nucleobase sequence of a nucleotide represented by SEQ ID NO: 353,
(cv) a dsRNA formed from a sense strand including a nucleobase sequence of a nucleotide represented by SEQ ID NO: 356 and an antisense strand including a nucleobase sequence of a nucleotide represented by SEQ ID NO: 357,
(cw) a dsRNA formed from a sense strand including a nucleobase sequence of a nucleotide represented by SEQ ID NO: 358 and an antisense strand including a nucleobase sequence of a nucleotide represented by SEQ ID NO: 359,
(cx) a dsRNA formed from a sense strand including a nucleobase sequence of a nucleotide represented by SEQ ID NO: 360 and an antisense strand including a nucleobase sequence of a nucleotide represented by SEQ ID NO: 361,
(cy) a dsRNA formed from a sense strand including a nucleobase sequence of a nucleotide represented by SEQ ID NO: 364 and an antisense strand including a nucleobase sequence of a nucleotide represented by SEQ ID NO: 365,
(da) a dsRNA formed from a sense strand including a nucleobase sequence of a nucleotide represented by SEQ ID NO: 372 and an antisense strand including a nucleobase sequence of a nucleotide represented by SEQ ID NO: 373,
(db) a dsRNA formed from a sense strand including a nucleobase sequence of a nucleotide represented by SEQ ID NO: 378 and an antisense strand including a nucleobase sequence of a nucleotide represented by SEQ ID NO: 379,
(dc) a dsRNA formed from a sense strand including a nucleobase sequence of a nucleotide represented by SEQ ID NO: 380 and an antisense strand including a nucleobase sequence of a nucleotide represented by SEQ ID NO: 381,
(de) a dsRNA formed from a sense strand including a nucleobase sequence of a nucleotide represented by SEQ ID NO: 390 and an antisense strand including a nucleobase sequence of a nucleotide represented by SEQ ID NO: 391,
(dg) a dsRNA formed from a sense strand including a nucleobase sequence of a nucleotide represented by SEQ ID NO: 394 and an antisense strand including a nucleobase sequence of a nucleotide represented by SEQ ID NO: 395,
(dh) a dsRNA formed from a sense strand including a nucleobase sequence of a nucleotide represented by SEQ ID NO: 396 and an antisense strand including a nucleobase sequence of a nucleotide represented by SEQ ID NO: 397,
(di) a dsRNA formed from a sense strand including a nucleobase sequence of a nucleotide represented by SEQ ID NO: 398 and an antisense strand including a nucleobase sequence of a nucleotide represented by SEQ ID NO: 399,
(dj) a dsRNA formed from a sense strand including a nucleobase sequence of a nucleotide represented by SEQ ID NO: 400 and an antisense strand including a nucleobase sequence of a nucleotide represented by SEQ ID NO: 401,
(dl) a dsRNA formed from a sense strand including a nucleobase sequence of a nucleotide represented by SEQ ID NO: 404 and an antisense strand including a nucleobase sequence of a nucleotide represented by SEQ ID NO: 405,
(dm) a dsRNA formed from a sense strand including a nucleobase sequence of a nucleotide represented by SEQ ID NO: 406 and an antisense strand including a nucleobase sequence of a nucleotide represented by SEQ ID NO: 407,
(dn) a dsRNA formed from a sense strand including a nucleobase sequence of a nucleotide represented by SEQ ID NO: 410 and an antisense strand including a nucleobase sequence of a nucleotide represented by SEQ ID NO: 411,
(do) a dsRNA formed from a sense strand including a nucleobase sequence of a nucleotide represented by SEQ ID NO: 412 and an antisense strand including a nucleobase sequence of a nucleotide represented by SEQ ID NO: 413,
(dp) a dsRNA formed from a sense strand including a nucleobase sequence of a nucleotide represented by SEQ ID NO: 414 and an antisense strand including a nucleobase sequence of a nucleotide represented by SEQ ID NO: 415,
(dq) a dsRNA formed from a sense strand including a nucleobase sequence of a nucleotide represented by SEQ ID NO: 416 and an antisense strand including a nucleobase sequence of a nucleotide represented by SEQ ID NO: 417,
(dr) a dsRNA formed from a sense strand including a nucleobase sequence of a nucleotide represented by SEQ ID NO: 418 and an antisense strand including a nucleobase sequence of a nucleotide represented by SEQ ID NO: 419,
(dt) a dsRNA formed from a sense strand including a nucleobase sequence of a nucleotide represented by SEQ ID NO: 424 and an antisense strand including a nucleobase sequence of a nucleotide represented by SEQ ID NO: 425,
(du) a dsRNA formed from a sense strand including a nucleobase sequence of a nucleotide represented by SEQ ID NO: 426 and an antisense strand including a nucleobase sequence of a nucleotide represented by SEQ ID NO: 427,
(dv) a dsRNA formed from a sense strand including a nucleobase sequence of a nucleotide represented by SEQ ID NO: 428 and an antisense strand including a nucleobase sequence of a nucleotide represented by SEQ ID NO: 429,
(dw) a dsRNA formed from a sense strand including a nucleobase sequence of a nucleotide represented by SEQ ID NO: 430 and an antisense strand including a nucleobase sequence of a nucleotide represented by SEQ ID NO: 431,
(dx) a dsRNA formed from a sense strand including a nucleobase sequence of a nucleotide represented by SEQ ID NO: 432 and an antisense strand including a nucleobase sequence of a nucleotide represented by SEQ ID NO: 433,
(dy) a dsRNA formed from a sense strand including a nucleobase sequence of a nucleotide represented by SEQ ID NO: 434 and an antisense strand including a nucleobase sequence of a nucleotide represented by SEQ ID NO: 435,
(dz) a dsRNA formed from a sense strand including a nucleobase sequence of a nucleotide represented by SEQ ID NO: 436 and an antisense strand including a nucleobase sequence of a nucleotide represented by SEQ ID NO: 437,
(ea) a dsRNA formed from a sense strand including a nucleobase sequence of a nucleotide represented by SEQ ID NO: 438 and an antisense strand including a nucleobase sequence of a nucleotide represented by SEQ ID NO: 439,
(eb) a dsRNA formed from a sense strand including a nucleobase sequence of a nucleotide represented by SEQ ID NO: 440 and an antisense strand including a nucleobase sequence of a nucleotide represented by SEQ ID NO: 441,
(ec) a dsRNA formed from a sense strand including a nucleobase sequence of a nucleotide represented by SEQ ID NO: 442 and an antisense strand including a nucleobase sequence of a nucleotide represented by SEQ ID NO: 443,
(ed) a dsRNA formed from a sense strand including a nucleobase sequence of a nucleotide represented by SEQ ID NO: 444 and an antisense strand including a nucleobase sequence of a nucleotide represented by SEQ ID NO: 445, and
(ee) a dsRNA formed from a sense strand including a nucleobase sequence of a nucleotide represented by SEQ ID NO: 446 and an antisense strand including a nucleobase sequence of a nucleotide represented by SEQ ID NO: 447.

In the double-stranded RNA according to the embodiment of the present invention, a double-stranded RNA is still more preferably the combination of the sense strand and the antisense strand, which is selected from the following dsRNAs,
(y) a dsRNA formed from a sense strand including a nucleobase sequence of a nucleotide represented by SEQ ID NO: 114 and an antisense strand including a nucleobase sequence of a nucleotide represented by SEQ ID NO: 115,
(z) a dsRNA formed from a sense strand including a nucleobase sequence of a nucleotide represented by SEQ ID NO: 118 and an antisense strand including a nucleobase sequence of a nucleotide represented by SEQ ID NO: 119,
(al) a dsRNA formed from a sense strand including a nucleobase sequence of a nucleotide represented by SEQ ID NO: 164 and an antisense strand including a nucleobase sequence of a nucleotide represented by SEQ ID NO: 165,
(ap) a dsRNA formed from a sense strand including a nucleobase sequence of a nucleotide represented by SEQ ID NO: 178 and an antisense strand including a nucleobase sequence of a nucleotide represented by SEQ ID NO: 179,
(ar) a dsRNA formed from a sense strand including a nucleobase sequence of a nucleotide represented by SEQ ID NO: 184 and an antisense strand including a nucleobase sequence of a nucleotide represented by SEQ ID NO: 185,
(au) a dsRNA formed from a sense strand including a nucleobase sequence of a nucleotide represented by SEQ ID NO: 194 and an antisense strand including a nucleobase sequence of a nucleotide represented by SEQ ID NO: 195,
(bb) a dsRNA formed from a sense strand including a nucleobase sequence of a nucleotide represented by SEQ ID NO: 210 and an antisense strand including a nucleobase sequence of a nucleotide represented by SEQ ID NO: 211,
(bc) a dsRNA formed from a sense strand including a nucleobase sequence of a nucleotide represented by SEQ ID NO: 212 and an antisense strand including a nucleobase sequence of a nucleotide represented by SEQ ID NO: 213,
(bf) a dsRNA formed from a sense strand including a nucleobase sequence of a nucleotide represented by SEQ ID NO: 222 and an antisense strand including a nucleobase sequence of a nucleotide represented by SEQ ID NO: 223,
(bl) a dsRNA formed from a sense strand including a nucleobase sequence of a nucleotide represented by SEQ ID NO: 244 and an antisense strand including a nucleobase sequence of a nucleotide represented by SEQ ID NO: 245,
(bn) a dsRNA formed from a sense strand including a nucleobase sequence of a nucleotide represented by SEQ ID NO: 248 and an antisense strand including a nucleobase sequence of a nucleotide represented by SEQ ID NO: 249,
(br) a dsRNA formed from a sense strand including a nucleobase sequence of a nucleotide represented by SEQ ID NO: 256 and an antisense strand including a nucleobase sequence of a nucleotide represented by SEQ ID NO: 257,
(bs) a dsRNA formed from a sense strand including a nucleobase sequence of a nucleotide represented by SEQ ID NO: 258 and an antisense strand including a nucleobase sequence of a nucleotide represented by SEQ ID NO: 259,
(bv) a dsRNA formed from a sense strand including a nucleobase sequence of a nucleotide represented by SEQ ID NO: 264 and an antisense strand including a nucleobase sequence of a nucleotide represented by SEQ ID NO: 265,
(ca) a dsRNA formed from a sense strand including a nucleobase sequence of a nucleotide represented by SEQ ID NO: 282 and an antisense strand including a nucleobase sequence of a nucleotide represented by SEQ ID NO: 283,
(cc) a dsRNA formed from a sense strand including a nucleobase sequence of a nucleotide represented by SEQ ID NO: 286 and an antisense strand including a nucleobase sequence of a nucleotide represented by SEQ ID NO: 287,
(cd) a dsRNA formed from a sense strand including a nucleobase sequence of a nucleotide represented by SEQ ID NO: 288 and an antisense strand including a nucleobase sequence of a nucleotide represented by SEQ ID NO: 289,
(ce) a dsRNA formed from a sense strand including a nucleobase sequence of a nucleotide represented by SEQ ID NO: 290 and an antisense strand including a nucleobase sequence of a nucleotide represented by SEQ ID NO: 291,
(cg) a dsRNA formed from a sense strand including a nucleobase sequence of a nucleotide represented by SEQ ID NO: 294 and an antisense strand including a nucleobase sequence of a nucleotide represented by SEQ ID NO: 295,
(ci) a dsRNA formed from a sense strand including a nucleobase sequence of a nucleotide represented by SEQ ID NO: 302 and an antisense strand including a nucleobase sequence of a nucleotide represented by SEQ ID NO: 303,
(cl) a dsRNA formed from a sense strand including a nucleobase sequence of a nucleotide represented by SEQ ID NO: 316 and an antisense strand including a nucleobase sequence of a nucleotide represented by SEQ ID NO: 317,
(cq) a dsRNA formed from a sense strand including a nucleobase sequence of a nucleotide represented by SEQ ID NO: 344 and an antisense strand including a nucleobase sequence of a nucleotide represented by SEQ ID NO: 345,
(cs) a dsRNA formed from a sense strand including a nucleobase sequence of a nucleotide represented by SEQ ID NO: 348 and an antisense strand including a nucleobase sequence of a nucleotide represented by SEQ ID NO: 349,
(cu) a dsRNA formed from a sense strand including a nucleobase sequence of a nucleotide represented by SEQ ID NO: 352 and an antisense strand including a nucleobase sequence of a nucleotide represented by SEQ ID NO: 353,
(cv) a dsRNA formed from a sense strand including a nucleobase sequence of a nucleotide represented by SEQ ID NO: 356 and an antisense strand including a nucleobase sequence of a nucleotide represented by SEQ ID NO: 357,
(cw) a dsRNA formed from a sense strand including a nucleobase sequence of a nucleotide represented by SEQ ID NO: 358 and an antisense strand including a nucleobase sequence of a nucleotide represented by SEQ ID NO: 359,
(cx) a dsRNA formed from a sense strand including a nucleobase sequence of a nucleotide represented by SEQ ID NO: 360 and an antisense strand including a nucleobase sequence of a nucleotide represented by SEQ ID NO: 361,
(dg) a dsRNA formed from a sense strand including a nucleobase sequence of a nucleotide represented by SEQ ID NO: 394 and an antisense strand including a nucleobase sequence of a nucleotide represented by SEQ ID NO: 395,
(dh) a dsRNA formed from a sense strand including a nucleobase sequence of a nucleotide represented by SEQ ID NO: 396 and an antisense strand including a nucleobase sequence of a nucleotide represented by SEQ ID NO: 397,
(dn) a dsRNA formed from a sense strand including a nucleobase sequence of a nucleotide represented by SEQ ID NO: 410 and an antisense strand including a nucleobase sequence of a nucleotide represented by SEQ ID NO: 411,
(dp) a dsRNA formed from a sense strand including a nucleobase sequence of a nucleotide represented by SEQ ID NO: 414 and an antisense strand including a nucleobase sequence of a nucleotide represented by SEQ ID NO: 415,
(dq) a dsRNA formed from a sense strand including a nucleobase sequence of a nucleotide represented by SEQ ID NO: 416 and an antisense strand including a nucleobase sequence of a nucleotide represented by SEQ ID NO: 417,
(du) a dsRNA formed from a sense strand including a nucleobase sequence of a nucleotide represented by SEQ ID NO: 426 and an antisense strand including a nucleobase sequence of a nucleotide represented by SEQ ID NO: 427,
(dv) a dsRNA formed from a sense strand including a nucleobase sequence of a nucleotide represented by SEQ ID NO: 428 and an antisense strand including a nucleobase sequence of a nucleotide represented by SEQ ID NO: 429,
(ed) a dsRNA formed from a sense strand including a nucleobase sequence of a nucleotide represented by SEQ ID NO: 444 and an antisense strand including a nucleobase sequence of a nucleotide represented by SEQ ID NO: 445, and
(ee) a dsRNA formed from a sense strand including a nucleobase sequence of a nucleotide represented by SEQ ID NO: 446 and an antisense strand including a nucleobase sequence of a nucleotide represented by SEQ ID NO: 447.

In the double-stranded RNA according to the embodiment of the present invention, a double-stranded RNA is even still more preferably the combination of the sense strand and the antisense strand, which is selected from the following dsRNAs,
(y) a dsRNA formed from a sense strand including a nucleobase sequence of a nucleotide represented by SEQ ID NO: 114 and an antisense strand including a nucleobase sequence of a nucleotide represented by SEQ ID NO: 115,
(z) a dsRNA formed from a sense strand including a nucleobase sequence of a nucleotide represented by SEQ ID NO: 118 and an antisense strand including a nucleobase sequence of a nucleotide represented by SEQ ID NO: 119,
(al) a dsRNA formed from a sense strand including a nucleobase sequence of a nucleotide represented by SEQ ID NO: 164 and an antisense strand including a nucleobase sequence of a nucleotide represented by SEQ ID NO: 165,
(ap) a dsRNA formed from a sense strand including a nucleobase sequence of a nucleotide represented by SEQ ID NO: 178 and an antisense strand including a nucleobase sequence of a nucleotide represented by SEQ ID NO: 179,
(ar) a dsRNA formed from a sense strand including a nucleobase sequence of a nucleotide represented by SEQ ID NO: 184 and an antisense strand including a nucleobase sequence of a nucleotide represented by SEQ ID NO: 185,
(au) a dsRNA formed from a sense strand including a nucleobase sequence of a nucleotide represented by SEQ ID NO: 194 and an antisense strand including a nucleobase sequence of a nucleotide represented by SEQ ID NO: 195,
(bb) a dsRNA formed from a sense strand including a nucleobase sequence of a nucleotide represented by SEQ ID NO: 210 and an antisense strand including a nucleobase sequence of a nucleotide represented by SEQ ID NO: 211,
(bc) a dsRNA formed from a sense strand including a nucleobase sequence of a nucleotide represented by SEQ ID NO: 212 and an antisense strand including a nucleobase sequence of a nucleotide represented by SEQ ID NO: 213,
(bl) a dsRNA formed from a sense strand including a nucleobase sequence of a nucleotide represented by SEQ ID NO: 244 and an antisense strand including a nucleobase sequence of a nucleotide represented by SEQ ID NO: 245,
(bn) a dsRNA formed from a sense strand including a nucleobase sequence of a nucleotide represented by SEQ ID NO: 248 and an antisense strand including a nucleobase sequence of a nucleotide represented by SEQ ID NO: 249,
(br) a dsRNA formed from a sense strand including a nucleobase sequence of a nucleotide represented by SEQ ID NO: 256 and an antisense strand including a nucleobase sequence of a nucleotide represented by SEQ ID NO: 257,
(bs) a dsRNA formed from a sense strand including a nucleobase sequence of a nucleotide represented by SEQ ID NO: 258 and an antisense strand including a nucleobase sequence of a nucleotide represented by SEQ ID NO: 259,
(bv) a dsRNA formed from a sense strand including a nucleobase sequence of a nucleotide represented by SEQ ID NO: 264 and an antisense strand including a nucleobase sequence of a nucleotide represented by SEQ ID NO: 265,
(ca) a dsRNA formed from a sense strand including a nucleobase sequence of a nucleotide represented by SEQ ID NO: 282 and an antisense strand including a nucleobase sequence of a nucleotide represented by SEQ ID NO: 283,
(cc) a dsRNA formed from a sense strand including a nucleobase sequence of a nucleotide represented by SEQ ID NO: 286 and an antisense strand including a nucleobase sequence of a nucleotide represented by SEQ ID NO: 287,
(cd) a dsRNA formed from a sense strand including a nucleobase sequence of a nucleotide represented by SEQ ID NO: 288 and an antisense strand including a nucleobase sequence of a nucleotide represented by SEQ ID NO: 289,
(cg) a dsRNA formed from a sense strand including a nucleobase sequence of a nucleotide represented by SEQ ID NO: 294 and an antisense strand including a nucleobase sequence of a nucleotide represented by SEQ ID NO: 295,
(ci) a dsRNA formed from a sense strand including a nucleobase sequence of a nucleotide represented by SEQ ID NO: 302 and an antisense strand including a nucleobase sequence of a nucleotide represented by SEQ ID NO: 303,
(cl) a dsRNA formed from a sense strand including a nucleobase sequence of a nucleotide represented by SEQ ID NO: 316 and an antisense strand including a nucleobase sequence of a nucleotide represented by SEQ ID NO: 317,
(cs) a dsRNA formed from a sense strand including a nucleobase sequence of a nucleotide represented by SEQ ID NO: 348 and an antisense strand including a nucleobase sequence of a nucleotide represented by SEQ ID NO: 349,
(cu) a dsRNA formed from a sense strand including a nucleobase sequence of a nucleotide represented by SEQ ID NO: 352 and an antisense strand including a nucleobase sequence of a nucleotide represented by SEQ ID NO: 353,
(cv) a dsRNA formed from a sense strand including a nucleobase sequence of a nucleotide represented by SEQ ID NO: 356 and an antisense strand including a nucleobase sequence of a nucleotide represented by SEQ ID NO: 357,
(cw) a dsRNA formed from a sense strand including a nucleobase sequence of a nucleotide represented by SEQ ID NO: 358 and an antisense strand including a nucleobase sequence of a nucleotide represented by SEQ ID NO: 359,
(cx) a dsRNA formed from a sense strand including a nucleobase sequence of a nucleotide represented by SEQ ID NO: 360 and an antisense strand including a nucleobase sequence of a nucleotide represented by SEQ ID NO: 361,
(dg) a dsRNA formed from a sense strand including a nucleobase sequence of a nucleotide represented by SEQ ID NO: 394 and an antisense strand including a nucleobase sequence of a nucleotide represented by SEQ ID NO: 395,
(dh) a dsRNA formed from a sense strand including a nucleobase sequence of a nucleotide represented by SEQ ID NO: 396 and an antisense strand including a nucleobase sequence of a nucleotide represented by SEQ ID NO: 397,
(dn) a dsRNA formed from a sense strand including a nucleobase sequence of a nucleotide represented by SEQ ID NO: 410 and an antisense strand including a nucleobase sequence of a nucleotide represented by SEQ ID NO: 411,
(dp) a dsRNA formed from a sense strand including a nucleobase sequence of a nucleotide represented by SEQ ID NO: 414 and an antisense strand including a nucleobase sequence of a nucleotide represented by SEQ ID NO: 415,
(dq) a dsRNA formed from a sense strand including a nucleobase sequence of a nucleotide represented by SEQ ID NO: 416 and an antisense strand including a nucleobase sequence of a nucleotide represented by SEQ ID NO: 417,
(du) a dsRNA formed from a sense strand including a nucleobase sequence of a nucleotide represented by SEQ ID NO: 426 and an antisense strand including a nucleobase sequence of a nucleotide represented by SEQ ID NO: 427,
(dv) a dsRNA formed from a sense strand including a nucleobase sequence of a nucleotide represented by SEQ ID NO: 428 and an antisense strand including a nucleobase sequence of a nucleotide represented by SEQ ID NO: 429,
(ed) a dsRNA formed from a sense strand including a nucleobase sequence of a nucleotide represented by SEQ ID NO: 444 and an antisense strand including a nucleobase sequence of a nucleotide represented by SEQ ID NO: 445, and
(ee) a dsRNA formed from a sense strand including a nucleobase sequence of a nucleotide represented by SEQ ID NO: 446 and an antisense strand including a nucleobase sequence of a nucleotide represented by SEQ ID NO: 447.

In the double-stranded RNA according to the embodiment of the present invention, a double-stranded RNA is particularly preferably the combination of the sense strand and the antisense strand, which is selected from the following dsRNAs,
(y) a dsRNA formed from a sense strand including a nucleobase sequence of a nucleotide represented by SEQ ID NO: 114 and an antisense strand including a nucleobase sequence of a nucleotide represented by SEQ ID NO: 115,
(al) a dsRNA formed from a sense strand including a nucleobase sequence of a nucleotide represented by SEQ ID NO: 164 and an antisense strand including a nucleobase sequence of a nucleotide represented by SEQ ID NO: 165,
(ar) a dsRNA formed from a sense strand including a nucleobase sequence of a nucleotide represented by SEQ ID NO: 184 and an antisense strand including a nucleobase sequence of a nucleotide represented by SEQ ID NO: 185,
(bb) a dsRNA formed from a sense strand including a nucleobase sequence of a nucleotide represented by SEQ ID NO: 210 and an antisense strand including a nucleobase sequence of a nucleotide represented by SEQ ID NO: 211,
(bc) a dsRNA formed from a sense strand including a nucleobase sequence of a nucleotide represented by SEQ ID NO: 212 and an antisense strand including a nucleobase sequence of a nucleotide represented by SEQ ID NO: 213,
(br) a dsRNA formed from a sense strand including a nucleobase sequence of a nucleotide represented by SEQ ID NO: 256 and an antisense strand including a nucleobase sequence of a nucleotide represented by SEQ ID NO: 257,
(cc) a dsRNA formed from a sense strand including a nucleobase sequence of a nucleotide represented by SEQ ID NO: 286 and an antisense strand including a nucleobase sequence of a nucleotide represented by SEQ ID NO: 287,
(cd) a dsRNA formed from a sense strand including a nucleobase sequence of a nucleotide represented by SEQ ID NO: 288 and an antisense strand including a nucleobase sequence of a nucleotide represented by SEQ ID NO: 289,
(cl) a dsRNA formed from a sense strand including a nucleobase sequence of a nucleotide represented by SEQ ID NO: 316 and an antisense strand including a nucleobase sequence of a nucleotide represented by SEQ ID NO: 317,
(cs) a dsRNA formed from a sense strand including a nucleobase sequence of a nucleotide represented by SEQ ID NO: 348 and an antisense strand including a nucleobase sequence of a nucleotide represented by SEQ ID NO: 349,
(cu) a dsRNA formed from a sense strand including a nucleobase sequence of a nucleotide represented by SEQ ID NO: 352 and an antisense strand including a nucleobase sequence of a nucleotide represented by SEQ ID NO: 353,
(cw) a dsRNA formed from a sense strand including a nucleobase sequence of a nucleotide represented by SEQ ID NO: 358 and an antisense strand including a nucleobase sequence of a nucleotide represented by SEQ ID NO: 359,
(cx) a dsRNA formed from a sense strand including a nucleobase sequence of a nucleotide represented by SEQ ID NO: 360 and an antisense strand including a nucleobase sequence of a nucleotide represented by SEQ ID NO: 361,
(dh) a dsRNA formed from a sense strand including a nucleobase sequence of a nucleotide represented by SEQ ID NO: 396 and an antisense strand including a nucleobase sequence of a nucleotide represented by SEQ ID NO: 397,
(dp) a dsRNA formed from a sense strand including a nucleobase sequence of a nucleotide represented by SEQ ID NO: 414 and an antisense strand including a nucleobase sequence of a nucleotide represented by SEQ ID NO: 415,
(dq) a dsRNA formed from a sense strand including a nucleobase sequence of a nucleotide represented by SEQ ID NO: 416 and an antisense strand including a nucleobase sequence of a nucleotide represented by SEQ ID NO: 417, and
(ee) a dsRNA formed from a sense strand including a nucleobase sequence of a nucleotide represented by SEQ ID NO: 446 and an antisense strand including a nucleobase sequence of a nucleotide represented by SEQ ID NO: 447.

As another aspect, in the double-stranded RNA according to the embodiment of the present invention, a double-stranded RNA is more preferably the combination of the sense strand and the antisense strand, which is selected from the following dsRNAs,
(a) a dsRNA formed from a sense strand including a nucleobase sequence of a nucleotide represented by SEQ ID NO: 1 and an antisense strand including a nucleobase sequence of a nucleotide represented by SEQ ID NO: 2,
(b) a dsRNA formed from a sense strand including a nucleobase sequence of a nucleotide represented by SEQ ID NO: 7 and an antisense strand including a nucleobase sequence of a nucleotide represented by SEQ ID NO: 8,
(c) a dsRNA formed from a sense strand including a nucleobase sequence of a nucleotide represented by SEQ ID NO: 11 and an antisense strand including a nucleobase sequence of a nucleotide represented by SEQ ID NO: 12,
(e) a dsRNA formed from a sense strand including a nucleobase sequence of a nucleotide represented by SEQ ID NO: 17 and an antisense strand including a nucleobase sequence of a nucleotide represented by SEQ ID NO: 18,
(f) a dsRNA formed from a sense strand including a nucleobase sequence of a nucleotide represented by SEQ ID NO: 19 and an antisense strand including a nucleobase sequence of a nucleotide represented by SEQ ID NO: 20,
(g) a dsRNA formed from a sense strand including a nucleobase sequence of a nucleotide represented by SEQ ID NO: 25 and an antisense strand including a nucleobase sequence of a nucleotide represented by SEQ ID NO: 26,
(h) a dsRNA formed from a sense strand including a nucleobase sequence of a nucleotide represented by SEQ ID NO: 27 and an antisense strand including a nucleobase sequence of a nucleotide represented by SEQ ID NO: 28, and
(k) a dsRNA formed from a sense strand including a nucleobase sequence of a nucleotide represented by SEQ ID NO: 33 and an antisense strand including a nucleobase sequence of a nucleotide represented by SEQ ID NO: 34,
In the double-stranded RNA according to the embodiment of the present invention, a double-stranded RNA is still more preferably the combination of the sense strand and the antisense strand, which is selected from the following dsRNAs,
(b) a dsRNA formed from a sense strand including a nucleobase sequence of a nucleotide represented by SEQ ID NO: 7 and an antisense strand including a nucleobase sequence of a nucleotide represented by SEQ ID NO: 8,
(e) a dsRNA formed from a sense strand including a nucleobase sequence of a nucleotide represented by SEQ ID NO: 17 and an antisense strand including a nucleobase sequence of a nucleotide represented by SEQ ID NO: 18,
(f) a dsRNA formed from a sense strand including a nucleobase sequence of a nucleotide represented by SEQ ID NO: 19 and an antisense strand including a nucleobase sequence of a nucleotide represented by SEQ ID NO: 20,
(g) a dsRNA formed from a sense strand including a nucleobase sequence of a nucleotide represented by SEQ ID NO: 25 and an antisense strand including a nucleobase sequence of a nucleotide represented by SEQ ID NO: 26,
(h) a dsRNA formed from a sense strand including a nucleobase sequence of a nucleotide represented by SEQ ID NO: 27 and an antisense strand including a nucleobase sequence of a nucleotide represented by SEQ ID NO: 28, and
(k) a dsRNA formed from a sense strand including a nucleobase sequence of a nucleotide represented by SEQ ID NO: 33 and an antisense strand including a nucleobase sequence of a nucleotide represented by SEQ ID NO: 34,

As yet another aspect, in the double-stranded RNA according to the embodiment of the present invention, a double-stranded RNA is more preferably the combination of the sense strand and the antisense strand, which is selected from the following dsRNAs,
(y) a dsRNA formed from a sense strand including a nucleobase sequence of a nucleotide represented by SEQ ID NO: 114 and an antisense strand including a nucleobase sequence of a nucleotide represented by SEQ ID NO: 115,
(al) a dsRNA formed from a sense strand including a nucleobase sequence of a nucleotide represented by SEQ ID NO: 164 and an antisense strand including a nucleobase sequence of a nucleotide represented by SEQ ID NO: 165,
(br) a dsRNA formed from a sense strand including a nucleobase sequence of a nucleotide represented by SEQ ID NO: 256 and an antisense strand including a nucleobase sequence of a nucleotide represented by SEQ ID NO: 257,
(cd) a dsRNA formed from a sense strand including a nucleobase sequence of a nucleotide represented by SEQ ID NO: 288 and an antisense strand including a nucleobase sequence of a nucleotide represented by SEQ ID NO: 289,
(cl) a dsRNA formed from a sense strand including a nucleobase sequence of a nucleotide represented by SEQ ID NO: 316 and an antisense strand including a nucleobase sequence of a nucleotide represented by SEQ ID NO: 317,
(cu) a dsRNA formed from a sense strand including a nucleobase sequence of a nucleotide represented by SEQ ID NO: 352 and an antisense strand including a nucleobase sequence of a nucleotide represented by SEQ ID NO: 353,
(cw) a dsRNA formed from a sense strand including a nucleobase sequence of a nucleotide represented by SEQ ID NO: 358 and an antisense strand including a nucleobase sequence of a nucleotide represented by SEQ ID NO: 359,
(dh) a dsRNA formed from a sense strand including a nucleobase sequence of a nucleotide represented by SEQ ID NO: 396 and an antisense strand including a nucleobase sequence of a nucleotide represented by SEQ ID NO: 397,
(dq) a dsRNA formed from a sense strand including a nucleobase sequence of a nucleotide represented by SEQ ID NO: 416 and an antisense strand including a nucleobase sequence of a nucleotide represented by SEQ ID NO: 417, and
(ee) a dsRNA formed from a sense strand including a nucleobase sequence of a nucleotide represented by SEQ ID NO: 446 and an antisense strand including a nucleobase sequence of a nucleotide represented by SEQ ID NO: 447.

In the double-stranded RNA according to the embodiment of the present invention, a double-stranded RNA is still more preferably the combination of the sense strand and the antisense strand, which is selected from the following dsRNAs,
(al) a dsRNA formed from a sense strand including a nucleobase sequence of a nucleotide represented by SEQ ID NO: 164 and an antisense strand including a nucleobase sequence of a nucleotide represented by SEQ ID NO: 165,
(br) a dsRNA formed from a sense strand including a nucleobase sequence of a nucleotide represented by SEQ ID NO: 256 and an antisense strand including a nucleobase sequence of a nucleotide represented by SEQ ID NO: 257,
(cd) a dsRNA formed from a sense strand including a nucleobase sequence of a nucleotide represented by SEQ ID NO: 288 and an antisense strand including a nucleobase sequence of a nucleotide represented by SEQ ID NO: 289,
(cu) a dsRNA formed from a sense strand including a nucleobase sequence of a nucleotide represented by SEQ ID NO: 352 and an antisense strand including a nucleobase sequence of a nucleotide represented by SEQ ID NO: 353,
(dh) a dsRNA formed from a sense strand including a nucleobase sequence of a nucleotide represented by SEQ ID NO: 396 and an antisense strand including a nucleobase sequence of a nucleotide represented by SEQ ID NO: 397,
(dq) a dsRNA formed from a sense strand including a nucleobase sequence of a nucleotide represented by SEQ ID NO: 416 and an antisense strand including a nucleobase sequence of a nucleotide represented by SEQ ID NO: 417, and
(ee) a dsRNA formed from a sense strand including a nucleobase sequence of a nucleotide represented by SEQ ID NO: 446 and an antisense strand including a nucleobase sequence of a nucleotide represented by SEQ ID NO: 447.

In the double-stranded RNA according to the embodiment of the present invention, a double-stranded RNA is even still more preferably the combination of the sense strand and the antisense strand, which is selected from the following dsRNAs,
(al) a dsRNA formed from a sense strand including a nucleobase sequence of a nucleotide represented by SEQ ID NO: 164 and an antisense strand including a nucleobase sequence of a nucleotide represented by SEQ ID NO: 165, and
(cu) a dsRNA formed from a sense strand including a nucleobase sequence of a nucleotide represented by SEQ ID NO: 352 and an antisense strand including a nucleobase sequence of a nucleotide represented by SEQ ID NO: 353,

A nucleobase of a nucleotide is a pyrimidine compound or a purine compound having a heterocycle, which is a constituent component of any nucleic acid, and includes an adenine (A), a guanine (G), a cytosine (C), a thymine (T), or a uracil (U). In the present specification, each of "G", "g", "C", "c", "A", "a", "U", "u", and "T" generally represents a nucleobase, a nucleoside, a nucleotide, or a nucleotide mimetic, which contains a guanine, a cytosine, an adenine, a uracil, or thymine as a base.

The term "sequence" or "nucleobase sequence" used in the present specification means a succession or order of a nucleobase, and/or a nucleotide, and/or a nucleoside, and described with a succession of letters using the standard nucleotide nomenclature and the notation for modified nucleotides described in the present specification.

In the present specification, the term "nucleotide" can include a modified nucleotide, a modified nucleotide mimetic, an abasic site, and a surrogate substitution moiety.

The sense strand and/or the antisense strand may optionally and independently contain an additional 1, 2, 3, 4, 5, or 6 nucleotides (extension) at the 3' end or the 5' end, or both at the 3' end and the 5' end of the core sequence. In a case where the additional nucleotides of the antisense strand is present, the additional nucleotides of the antisense strand may or may not be complementary to the corresponding sequence in the YTHDC1 mRNA. In a case where the additional nucleotides of the sense strand is present, the additional nucleotides of the sense strand may or may not be complementary to the corresponding sequence in the YTHDC1 mRNA. In a case where the additional nucleotides of the antisense strand is present, the additional nucleotides of the antisense strand may or may not be complementary to the additional nucleotides that may be present in the corresponding sense strand.

In the present specification, the extension includes 1, 2, 3, 4, 5, or 6 nucleotides located at the 5' end and/or 3' end of the core sequence of the sense strand and/or the core sequence of the antisense strand. The extension nucleotides on the sense strand may or may not be complementary to the nucleotides of the corresponding antisense strand (the core sequence nucleotide or the extension nucleotide). Conversely, the extension nucleotides on the antisense strand may or may not be complementary to the nucleotides of the corresponding sense strand (the core sequence nucleotide or the extension nucleotide). In some embodiments, both the sense strand and the antisense strand of the double-stranded RNA include 3' extension and 5' extension. In some embodiments, one or more 3' extension nucleotides in one strand form base pairs with one or more 5' extension nucleotides in the other strand. In another embodiment, one or more 3' extension nucleotides in one strand do not form a base pair with one or more 5' extension nucleotides in the other strand. In some embodiments, the double-stranded RNA has an antisense strand having a 3' extension and a sense strand having a 5' extension.

In some embodiments, the sense strand and the antisense strand of the double-stranded RNA described in the present specification contain the same number of nucleotides. In some embodiments, the sense strand and the antisense strand of the double-stranded RNA described in the present specification contain different numbers of nucleotides. In some embodiments, the 5' end of the sense strand and the 3' end of the antisense strand of the double-stranded RNA form a blunt end. In some embodiments, the 3' end of the sense strand and the 5' end of the antisense strand of the double-stranded RNA form a blunt end. In some embodiments, both ends of the double-stranded RNA form blunt ends. In some embodiments, neither end of the double-stranded RNA is a blunt end. In some embodiments, the 5' end of the sense strand and the 3' end of the antisense strand of the double-stranded RNA form a frayed end. In some embodiments, the 3' end of the sense strand and the 5' end of the antisense strand of the double-stranded RNA form a frayed end. In some embodiments, both ends of the double-stranded RNA form frayed ends. In some embodiments, neither end of the double-stranded RNA is frayed end. In the present specification, the frayed end means an end of a double-stranded RNA molecule in which the terminal nucleotides of the two annealed strands form a pair (that is, do not form an overhang) but are not complementary (that is, form a non-complementary pair). In the present specification, an overhang is an extension of one or more unpaired nucleotides located at the end of one strand of a double-stranded RNA molecule. The unpaired nucleotides may be on the sense strand or the antisense strand, resulting in a 3' overhang or a 5' overhang. In some embodiments, the double-stranded RNA molecule includes a blunt end and a frayed end, includes a blunt end and a 5' overhang end, includes a blunt end and a 3' overhang end, includes an frayed end and a 5' overhang end, includes an frayed end and a 3' overhang end, includes two 5' overhang ends, includes two 3' overhang ends, includes a 5' overhang end and a 3' overhang end, includes two frayed ends, or includes two blunt ends.

In the present specification, the "modified nucleotide" is a nucleotide other than a ribonucleotide (2'-hydroxyl nucleotide). Non-limiting examples of modified nucleotides include deoxynucleotides, nucleotide mimetics, abasic nucleotides, 2'-modified nucleotides, 3' to 3' linkages (inverted) nucleotides, non-natural base-containing nucleotides, bridged nucleotides, and locked nucleotides. A non-limiting examples of a 2'-modified nucleotide (that is, a nucleotide having a group other than the hydroxyl group at the 2'-position of the 5-membered sugar ring) include a 2'-O-methyl nucleotide (in the present specification, represented as a lowercase letter "n" in a nucleotide sequence), a 2'-deoxy-2'-fluoronucleotide (represented as Nf in the present specification, but also referred to as 2'-fluoronucleotide in the present specification), a 2'-deoxynucleotide, and a 2'-methoxyethyl (2'-O-2-methoxyethyl) nucleotide. It is not necessary for all positions in a given compound to be uniformly modified. Two or more modifications can be incorporated in a single double-stranded RNA, or two or more modifications can be incorporated in a single nucleotide of the double-stranded RNA. The sense strand and the antisense strand of the double-stranded RNA can be synthesized and/or modified by a method known in the art. Modification of one nucleotide is independent of modification of another nucleotide.

In some embodiments, one or more nucleotides of the double-stranded RNA are linked by non-standard linkages or backbones (that is, modified internucleoside linkages or modified internucleoside backbones). In some embodiments, the modified internucleoside linkage is a non-phosphate-containing covalent bonding internucleoside linkage. Non-limiting examples of modified internucleoside linkages or modified internucleoside backbones include phosphorothioates, 5'-phosphorothioate group (represented in the present specification as a lowercase letter "s" before a nucleotide, as in sN, sn, or sNf), and chiral phosphorothioates.

In some embodiments, the double-stranded RNA contains one or more modified nucleotides and one or more modified internucleoside linkages. In some embodiments, a 2'-modified nucleotide is combined with a modified internucleoside linkage. For example, in some embodiments, the sense strand of the double-stranded RNA can contain 1, 2, 3, or 4 phosphorothioate linkages, the antisense strand of the double-stranded RNA can contain 1, 2, 3, or 4 phosphorothioate linkages; or both the sense strand and the antisense strand can independently contain 1, 2, 3, or 4 phosphorothioate linkages.

The pharmaceutical composition containing the double-stranded RNA according to the embodiment of the present invention may be designed using a drug delivery system (DDS) technology for delivery to a target tissue or cells of interest. Methods of design and production for delivering a double-stranded RNA (for example, siRNA) to a target tissue or cells of interest are known in the art. To improve, for example, stability, bioavailability, and utility as therapeutic methods, a method of producing a double-stranded RNA having a modification (for example, a chemical modification) that enables these properties is also known (Yuko Ito, STI Horizon, Vol. 5, No. 4, 2019, https://doi.org/10.15108/stih.00196). Furthermore, methods of prescribing and delivering a pharmaceutical composition containing a double-stranded RNA to a living body are also well known in the art. Examples thereof include, but are not limited to, the methods described in Signal Transduction and Targeted Therapy, volume 5, Article number: 101, 2020.

The pharmaceutical composition containing the double-stranded RNA according to the embodiment of the present invention may or may not contain, for example, lipid nanoparticles (LNP) or N-acetylgalactosamine (GalNAc). In a specific embodiment, the pharmaceutical composition preferably includes lipid nanoparticles (LNP) or N-acetylgalactosamine (GalNAc).

In some embodiments, the double-stranded RNA can be expressed from a transcriptional unit inserted into a vector. The recombinant vector can be a DNA plasmid, a non-viral vector, or a viral vector. The expression viral vector of the double-stranded RNA can be constructed based on, but is not limited to, an adeno-associated virus, a retrovirus, an adenovirus, or an alphavirus. The recombinant vector capable of expressing the double-stranded RNA can be delivered as described above, and allows the double-stranded RNA to be expressed transiently or stably. For example, such a vector can contain 1) a transcription initiation region, 2) optionally a transcription termination region, and 3) a nucleic acid sequence encoding at least one strand of a double-stranded RNA, and the sequence may be linked to the initiation region and the termination region to allow expression and/or delivery of the double-stranded RNA.

The double-stranded RNA according to the embodiment of the present invention may be encoded in the above-described recombinant expression vector, and in a case where the double-stranded RNA is encoded in the recombinant expression vector, the double-stranded RNA is encoded preferably in an adeno-associated virus vector, a retrovirus vector, an adenovirus vector, or an alphavirus vector, and more preferably in an adeno-associated virus vector.

The "adeno-associated virus" is a single-stranded DNA virus, which belongs to the family Parvoviridae and consists of about 4,700 bases, has no envelope, and has a capsid of a regular icosahedral structure having a diameter of 20 to 30 nm. Since the adeno-associated virus recognizes heparan sulfate proteoglycan which is a universal component of the cell membrane to infect a cell, the range of hosts thereof is wide.

Any gene or nucleic acid can be inserted into the genome of the adeno-associated virus. Therefore, it is possible to introduce a gene or nucleic acid of interest by infecting a target cell with a genetically modified adeno-associated virus, and the adeno-associated virus thus can be used as a vector for gene transfer. Furthermore, since the "adeno-associated virus vector" is non-pathogenic and enables the gene transfer into a terminally differentiated non-dividing cell, it is expected to be applied to gene therapy.

It is known that adeno-associated viruses have different infection directivity to tissues and cells depending on the difference in serotype (Ozawa et al., Drug Delivery System, Vol. 22, No. 6, pp. 643 to 650, 2007).

The substance contained in the pharmaceutical composition containing the double-stranded RNA according to the embodiment of the present invention is preferably an adeno-associated virus type 5, an adeno-associated virus type 7, an adeno-associated virus type 8, or an adeno-associated virus type 9, and more preferably an adeno-associated virus type 8.

The double-stranded RNA according to the embodiment of the present invention can be combined with one or more additional (that is, second, third, or the like) therapeutic agents. Examples of second therapeutic agent include a nucleic acid analog compound or a double-stranded RNA for an HBV RNA. Examples of additional therapeutic agents include low-molecular-weight molecule compounds, antibodies, vaccines, and the like. The double-stranded RNA, with or without combination of one or more additional agents, can be combined with one or more pharmaceutically acceptable carriers to form a pharmaceutical composition.

The pharmaceutically acceptable carrier includes an excipient, a diluent, a bulking agent, a disintegrating agent, a stabilizer, a preservative, a buffer, an emulsifier, a fragrance, a colorant, a sweetener, a thickening agent, a taste improving agent, a dissolution auxiliary agent, and other additives. In a case where one or more of such carriers are used, it is possible to prepare double-stranded RNA having forms such as a tablet, a capsule, a powdered drug, a syrup, a granule, a pill, a suspension, an emulsion, a powder preparation, a suppository, an eye drop, a nasal drop, an ear drop, a patch, an ointment, an injection agent, a liquid drug, a troche, and an elixir. It is preferable to prepare the double-stranded RNA in the form of a suspension or an injection agent.

These double-stranded RNAs can be administered in a number of ways depending on whether topical or systemic treatment is desired. For example, intravenous administration, intraarterial administration, subcutaneous administration, intraperitoneal administration, subdermal administration (for example, through an implanted device), or intraparenchymal administration can be performed.

In addition, the dose and the frequency of administration of the double-stranded RNA according to the embodiment of the present invention can be appropriately selected depending on the sex, weight, age, severity, symptom, and the like of the patient. In general, for adults, a daily dose of 0.01 to 1,000 mg/kg may be dividedly administered one to several times by drip infusion or subcutaneous administration.

The double-stranded RNA according to the embodiment of the present invention is a double-stranded RNA for treating hepatitis B in a subject, and is useful as a double-stranded RNA including an inhibitor for the expression of YTHDC1.

The pharmaceutical composition containing the double-stranded RNA according to the embodiment of the present invention can be used in a method for treating infectious diseases of HBV, diseases, or abnormalities caused by HBV infection. Such a method includes administering the double-stranded RNA described in the present specification to a subject (for example, a human or animal subject).

### <Use application of double-stranded RNA>

The double-stranded RNA according to the embodiment of the present invention is useful in the medical treatment or prevention of HBV infection.

The double-stranded RNA according to the embodiment of the present invention can inhibit the production of an HBV protein, particularly the HBs antigen. In addition, the HBV gene expression (the HBV DNA production) can be inhibited. Therefore, the double-stranded RNA according to the embodiment of the present invention is useful for the medical treatment or prevention of HBV infection.

The present invention relates to the use of the double-stranded RNA according to the embodiment of the present invention for inhibiting the production of the HBs antigen.

The present invention relates to the use of the double-stranded RNA according to the embodiment of the present invention for inhibiting the DNA production of HBV.

The present invention relates to the use of the double-stranded RNA according to the embodiment of the present invention for inhibiting the gene expression of HBV.

The present invention relates to the use of the double-stranded RNA according to the embodiment of the present invention for the medical treatment or prevention of HBV infection.

The disease associated with HBV infection includes progressive liver fibrosis, inflammation, and necrosis, which progress to liver cirrhosis, end-stage liver disease, and hepatocellular carcinoma.

Next, the present invention will be described with reference to Test Examples; however, the present invention is not limited thereto.

### Examples

### [Test Example 1]

### (Screening of unmodified dsRNA for YTHDC1)

HepG2.2.15 cells obtained by introducing the HBV genome into a hepatoblastoma cell line HepG2 (Proc Natl Acad Sci USA, Vol. 84, pp. 1005 to 1009, 1987), which are cells continuously producing viruses, were used, and the knockdown effect of designed unmodified dsRNA on YTHDC1 mRNA and the anti-HBV activity of designed unmodified dsRNA was evaluated by the following procedure.

The following medium was used as a cell culture medium.

DMEM/F-12, GlutaMAX (manufactured by Invitrogen Corporation) + 5 µg/mL insulin (manufactured by FUJIFILM Wako Pure Chemical Corporation) + 1% penicillin/streptomycin (manufactured by FUJIFILM Wako Pure Chemical Corporation) + 10 mmol/L HEPES (manufactured by FUJIFILM Wako Pure Chemical Corporation) + 50 µmol/L hydrocortisone (manufactured by Sigma-Aldrich Co., LLC) + 10% FBS (manufactured by Equitech-Bio Inc.)

The dsRNAs used for the transfection have the sequences shown in Table 1 (designated by duplex number #1 to duplex number #31). In addition, Negative Control No. 1 siRNA (manufactured by Thermo Fisher Scientific, Inc.) was used as a negative control dsRNA. HepG2.2.15 cells were suspended in the above medium to prepare a HepG2.2.15 cell suspension of 2 × 10⁵ cells/mL. The dsRNA was diluted with Nuclease-Free Water (not DEPC-Treated) (manufactured by Thermo Fisher Scientific, Inc.) to prepare a 500 nmol/L dsRNA solution. The prepared dsRNA solution was further diluted with a serum-free medium (manufactured by Thermo Fisher Scientific, Inc.) to prepare a 100 nmol/L dsRNA solution. 0.3 µL of Lipofectamine RNAiMAX Transfection Reagent (manufactured by Thermo Fisher Scientific, Inc.) and 4.7 µL of a serum-free medium were added to 5 µL of this dsRNA solution and incubated at room temperature for 5 minutes. 10 µL of this dsRNA-Lipofectamine RNAiMAX Transfection Reagent mixture was diluted with 40 µL of a cell culture medium to adjust the volume to 50 µL. This diluted mixture was mixed with 50 µL of the above-described HepG2.2.15 cell suspension, seeded on a 96-well microtiter plate, and incubated in a 5% CO₂ incubator at 37°C for 3 days. Next, the medium was exchanged at 100 µL/well with the above-described cell culture medium, and incubation was further carried out at 37°C for 3 days in a 5% CO₂ incubator. Then, the culture supernatant was collected, and the cell viability was measured using CellTiter96 AQueous One Solution Cell Proliferation Assay (manufactured by Promega Corporation) (Table 2). After measuring the cell viability, the cells were washed with phosphate buffered saline (100 µL/well, manufactured by Fujifilm Wako Pure Chemical Corporation), and total RNA was extracted using NucleoSpin 96 RNA (manufactured by Macherey-Nagel GmbH & Co. KG). 7 µL of the extracted total RNA was reverse-transcribed using a PrimeScript RT Reagent Kit with gDNA Eraser (manufactured by Takara Bio Inc.) to prepare cDNA. YTHDC1 and glyceraldehyde-3-phosphate dehydrogenase (GAPDH) as a housekeeping gene, in the prepared cDNA, were quantified by a real-time PCR method, the YTHDC1 expression level corrected by GAPDH was calculated, and then the relative value (%) of the expression level of YTHDC1 in each of the unmodified dsRNA-added wells to the expression level of YTHDC1 in the negative control dsRNA-added well was calculated (Table 2). In addition, the amount of the HBs antigen in the collected culture supernatant was measured using AlphaLISA Hepatitis B Virus Surface Antigen (HBsAg) Kit (manufactured by PerkinElmer, Inc.). The relative value (%) in each of the unmodified dsRNA-added wells to the negative control dsRNA-added well was calculated (Table 2).

**[Table 1]**

| Sequences of sense strands and antisense strands of unmodified dsRNA for YTHDC1 | | | | | |
|---|---|---|---|---|---|
| Duplex number | SEQ ID NO | Sequence of sense strand (5' to 3') | SEQ ID NO | Sequence of antisense strand (5' to 3') | Position on YTHDC1 cDNA (SEQ ID NO: 63) |
| #1 | 1 | GGAGAAAGAUGGAGAACUU | 2 | AAGUUCUCCAUCUUUCUCC | 385-403 |
| #2 | 3 | GCUCAUCUGUUAGCAAUAA | 4 | UUAUUGCUAACAGAUGAGC | 588-606 |
| #3 | 5 | GUCAGCCACAGAGUAUAAA | 6 | UUUAUACUCUGUGGCUGAC | 634-652 |
| #4 | 7 | CAGCCACAGAGUAUAAAAA | 8 | UUUUUAUACUCUGUGGCUG | 636-654 |
| #5 | 9 | CCUCCAGAGAACCUUAUAA | 10 | UUAUAAGGUUCUCUGGAGG | 726-744 |
| #6 | 11 | GGAGGAAGAAGAAGAAUAU | 12 | AUAUUCUUCUUCUUCCUCC | 1096-1114 |
| #7 | 13 | GCAGAUCAAACCAGUAAAC | 14 | GUUUACUGGUUUGAUCUGC | 1334-1352 |
| #8 | 15 | GUCAGAGAGAGUGGAAAAU | 16 | AUUUUCCACUCUCUCUGAC | 1517-1535 |
| #9 | 17 | GGGUUUGCAAGACUUUCUU | 18 | AAGAAAGUCUUGCAAACCC | 1541-1559 |
| #10 | 19 | GCAGGAAUGAGUGCUAAAA | 20 | UUUUAGCACUCAUUCCUGC | 1604-1622 |
| #11 | 21 | UGGGAGGUGUCUUUAAAAU | 22 | AUUUUAAAGACACCUCCCA | 1626-1644 |
| #12 | 23 | ACGUGAUGGACAGGAAAUU | 24 | AAUUUCCUGUCCAUCACGU | 1732-1750 |
| #13 | 25 | CCAGCUUUGUCUUCUGUUU | 26 | AAACAGAAGACAAAGCUGG | 1768-1786 |
| #14 | 27 | GCGUCGACCAGAAGAUUAU | 28 | AUAAUCUUCUGGUCGACGC | 1912-1930 |
| #15 | 29 | AGUGGACAGACGAUUUUCA | 30 | UGAAAAUCGUCUGUCCACU | 2023-2041 |
| #16 | 31 | UUCGCCGAGAUGUGUUUUU | 32 | AAAAACACAUCUCGGCGAA | 2046-2064 |
| #17 | 33 | UCGCCGAGAUGUGUUUUUA | 34 | UAAAAACACAUCUCGGCGA | 2047-2065 |
| #18 | 35 | CCACAUGAAGCAAGAUACA | 36 | UGUAUCUUGCUUCAUGUGG | 2237-2255 |
| #19 | 37 | UGCACACGGUUCAGUAAAA | 38 | UUUUACUGAACCGUGUGCA | 2885-2903 |
| #20 | 39 | GUAGCAGCCUCUUGUUUUU | 40 | AAAAACAAGAGGCUGCUAC | 2965-2983 |
| #21 | 41 | GGCACACUUUAGCAGAAAU | 42 | AUUUCUGCUAAAGUGUGCC | 3386-3404 |
| #22 | 43 | UGGCCUGAAAAGAACAACA | 44 | UGUUGUUCUUUUCAGGCCA | 3890-3908 |
| #23 | 45 | UUGUGGUAGAGAGGAAAAA | 46 | UUUUUCCUCUCUACCACAA | 4054-4072 |
| #24 | 47 | CCUGGCCUGAGAUUUUUAA | 48 | UUAAAAAUCUCAGGCCAGG | 4721-4739 |
| #25 | 49 | UGGCCUGAGAUUUUUAAAC | 50 | GUUUAAAAAUCUCAGGCCA | 4723-4741 |
| #26 | 51 | GGCUUAAGCAGAGGAUUUU | 52 | AAAAUCCUCUGCUUAAGCC | 4758-4776 |
| #27 | 53 | AAUCCGGUCAGGGAUUUUA | 54 | UAAAAUCCCUGACCGGAUU | 5179-5197 |
| #28 | 55 | UCCGGUCAGGGAUUUUAUU | 56 | AAUAAAAUCCCUGACCGGA | 5181-5199 |
| #29 | 57 | UGGCAGGAGAGAGACUAUU | 58 | AAUAGUCUCUCUCCUGCCA | 5361-5379 |
| #30 | 59 | GCAGGAGAGAGACUAUUUU | 60 | AAAAUAGUCUCUCUCCUGC | 5363-5381 |
| #31 | 61 | CUGUGACACACAUGGAUAA | 62 | UUAUCCAUGUGUGUCACAG | 5857-5875 |

**[Table 2]**

| Evaluation of activity of unmodified dsRNA for YTHDC1 | | | |
|---|---|---|---|
| Duplex number | Cell viability % | Relative expression level of YTHDC1 mRNA % | Relative amount of HBs antigen % |
| #1 | 68.7±7.1 | 46.3±3.4 | 3.1±0.6 |
| #2 | 62.6±7.5 | 109.6±17.3 | 11.2±0.6 |
| #3 | 55.7±1.9 | 92.0±1.2 | 15.8±1.1 |
| #4 | 89.7±1.6 | 43.1±6.5 | 3.2±0.3 |
| #5 | 14.9±2.3 | 130.9±14.8 | 5.6±0.7 |
| #6 | 74.7±3.1 | 68.7±2.4 | 5.2±0.1 |
| #7 | 65.3±4.1 | 51.6±11.2 | 21.4±0.9 |
| #8 | 79.7±2.5 | 77.1±6.6 | 4.6±0.3 |
| #9 | 158.2±6.7 | 14.5±2.3 | 3.1±0.2 |
| #10 | 123.4±12.4 | 56.2±7.7 | 3.7±0.1 |
| #11 | 137.4±7.1 | 89.0±15.5 | 11.8±1.7 |
| #12 | 93.7±1.9 | 87.2±14.3 | 3.0±0.4 |
| #13 | 129.1±10.0 | 13.4±0.7 | 3.9±0.5 |
| #14 | 116.0±9.6 | 45.4±5.5 | 2.8±0.2 |
| #15 | 174.4±5.9 | 64.6±7.5 | 148.8±2.6 |
| #16 | 160.6±3.2 | 25.5±1.1 | 13.2±1.0 |
| #17 | 167.8±7.4 | 15.5±1.7 | 3.9±0.4 |
| #18 | 22.1±7.7 | 69.6±3.1 | 3.2±0.7 |
| #19 | 92.4±3.6 | 61.9±3.4 | 21.8±1.6 |
| #20 | 99.6±9.2 | 70.3±1.8 | 9.4±0.6 |
| #21 | 75.2±7.1 | 220.8±5.1 | 10.5±1.3 |
| #22 | 145.3±3.2 | 161.4±26.2 | 20.8±2.2 |
| #23 | 101.5±6.3 | 201.6±31.3 | 17.1±0.5 |
| #24 | 92.6±11.1 | 159.8±37.7 | 43.0±8.8 |
| #25 | 170.0±10.4 | 117.7±17.0 | 7.7±1.2 |
| #26 | 107.2±11.0 | 106.0±16.8 | 16.2±0.9 |
| #27 | 123.7±7.6 | 102.6±12.2 | 40.5±4.3 |
| #28 | 118.2±14.0 | 97.2±23.8 | 31.4±4.2 |
| #29 | 110.5±2.7 | 482.4±48.7 | 11.8±1.6 |
| #30 | 139.9±13.2 | 102.2±25.2 | 34.4±1.1 |
| #31 | 94.5±0.9 | 247.1±45.0 | 15.1±1.3 |

By the dsRNAs of the duplex numbers #1, #4, #6, #7, #9, #10, #13, #14, #15, #16, #17, #18, and #19 among the unmodified dsRNAs for YTHDC1, the expression level of YTHDC1 mRNA was decreased by 30% or more. Among them, for the duplex numbers #1, #4, #9, #13, #14, #16, and #17, the expression level of YTHDC1 mRNA was decreased by 50% or more. Furthermore, by these 7 types of dsRNAs, a significant decrease in cell viability (cell viability of less than 50%) was not observed, while the amount of HBs antigen in the culture supernatant was all decreased by about 90%.

### [Test Example 2]

### (Evaluation of activity of unmodified dsRNA for YTHDC1)

Using HepG2.2.15 cells, the activities of dsRNAs of duplex numbers #1, #4, #6, #9, #10, #13, #14, and #17 found in Test Example 1 were evaluated by the following procedure.

The following medium was used as a cell culture medium.

DMEM/F-12, GlutaMAX + 5 µg/mL insulin + 1% penicillin/streptomycin + 10 mmol/L HEPES + 50 µmol/L hydrocortisone + 10% FBS

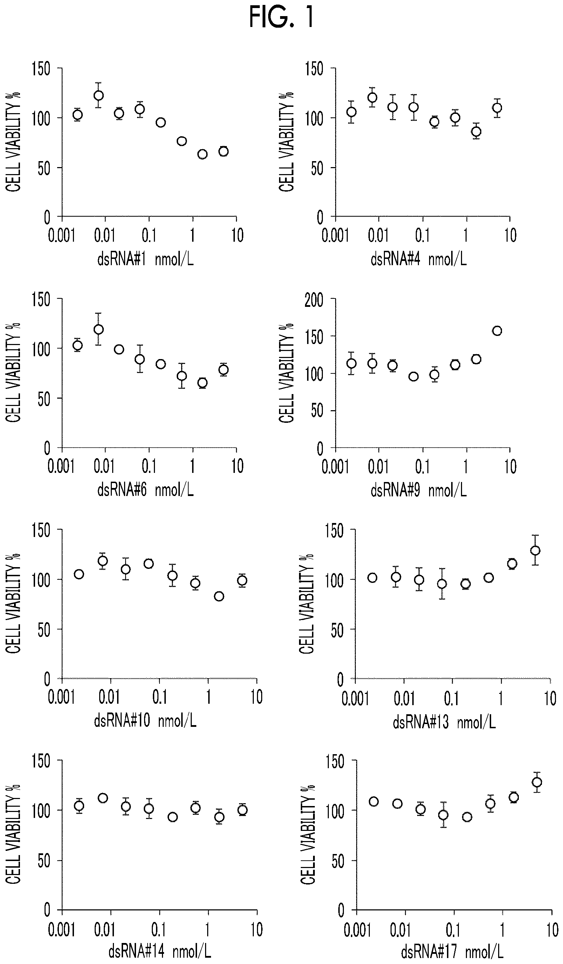

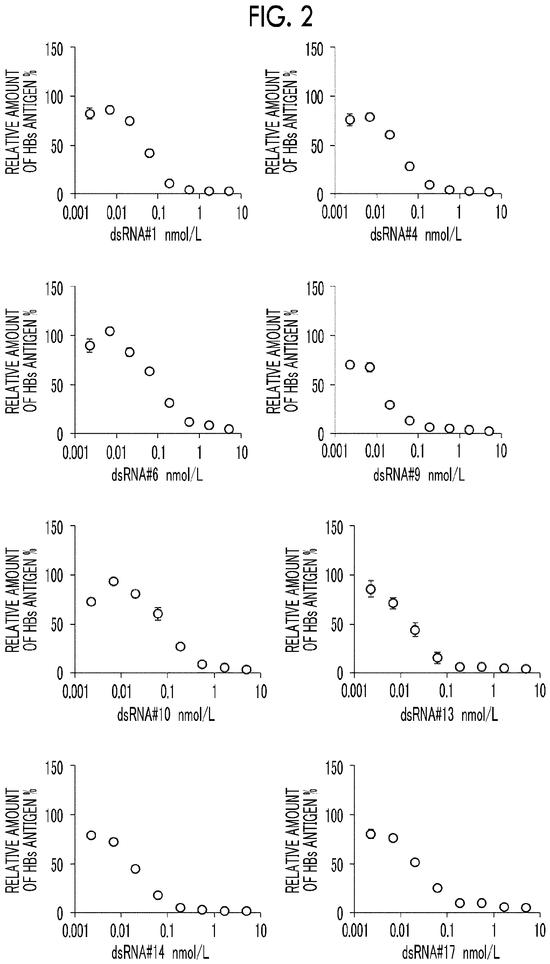

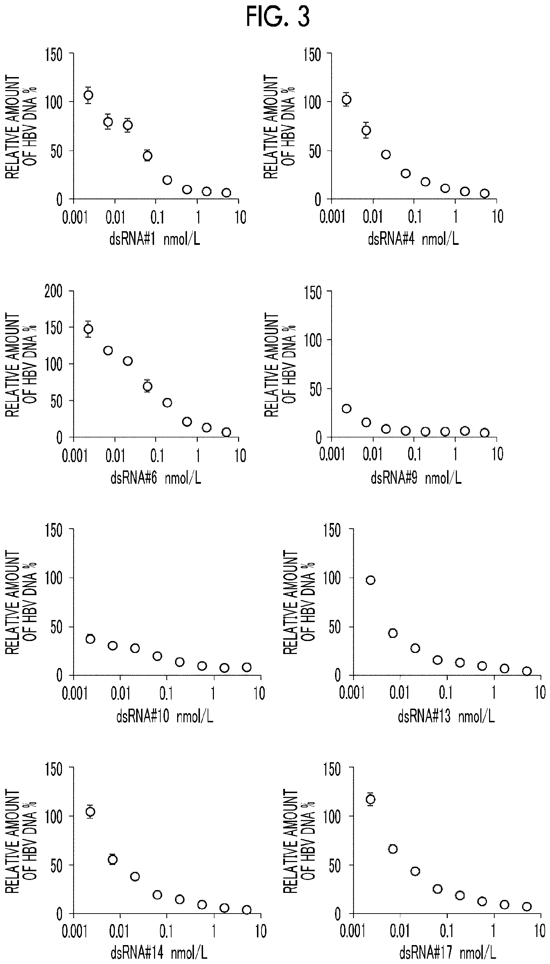

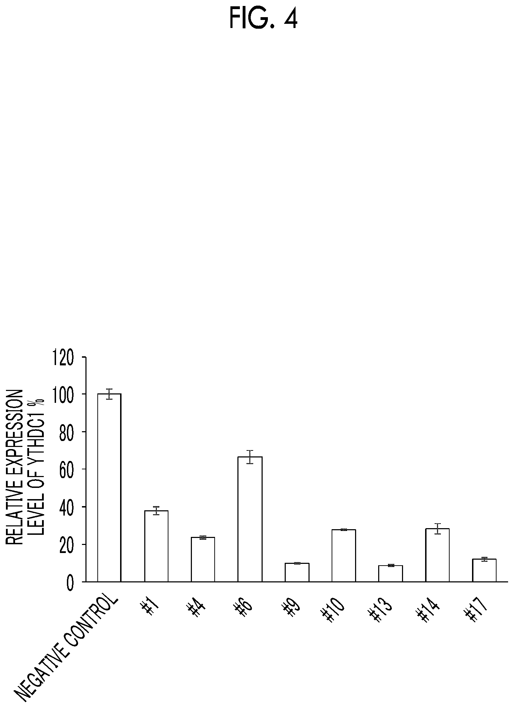

The dsRNAs used for the transfection have the sequences shown in Table 1 (designated by the duplex numbers #1, #4, #6, #9, #10, #13, #14, and #17). In addition, Negative Control No. 1 siRNA was used as a negative control dsRNA.
(1) HepG2.2.15 cells were suspended in the above medium to prepare a HepG2.2.15 cell suspension of 2 × 10⁵ cells/mL. The dsRNA was diluted with Nuclease-Free Water (not DEPC-Treated) to prepare dsRNA solutions of 3-fold dilution series from 0.229 to 500 nmol/L. These prepared dsRNA solutions were further diluted with a serum-free medium to prepare a dsRNA solution of 3-fold dilution series from 0.0457 to 100 nmol/L. 0.3 µL of Lipofectamine RNAiMAX Transfection Reagent and 4.7 µL of a serum-free medium were added to each concentration of 5 µL of dsRNA solutions and incubated at room temperature for 5 minutes. 10 µL of this dsRNA-Lipofectamine RNAiMAX Transfection Reagent mixture was diluted with 40 µL of a cell culture medium to adjust the volume to 50 µL. This diluted mixture was mixed with 50 µL of the above-described HepG2.2.15 cell suspension, seeded on a 96-well microtiter plate, and incubated in a 5% CO₂ incubator at 37°C for 3 days. Next, the medium was exchanged at 100 µL/well with the above-described cell culture medium, and incubation was further carried out at 37°C for 3 days in a 5% CO₂ incubator. Then, the culture supernatant was collected, and the cell viability was measured using CellTiter96 AQueous One Solution Cell Proliferation Assay (Fig. 1). The amount of the HBs antigen in the collected culture supernatant was measured using AlphaLISA Hepatitis B Virus Surface Antigen (HBsAg) Kit. In addition, the collected culture supernatant was treated with a mixture of Buffer AL (manufactured by Qiagen) and Proteinase K (manufactured by Thermo Fisher Scientific, Inc.), and the DNA of HBV (the HBV DNA) in the obtained solution was quantified by a real-time PCR method. Regarding each of the markers, the relative value (%) in each of the dsRNA-added wells in each concentration to the negative control dsRNA-added well was calculated (Figs. 2 and 3). Furthermore, regarding each of the markers, a 50% inhibitory concentration (IC50) was calculated (Tables 3 and 4).
(2) HepG2.2.15 cells were suspended in the above medium to prepare a HepG2.2.15 cell suspension of 2 × 10⁵ cells/mL. The dsRNA was diluted with Nuclease-Free Water (not DEPC-Treated) to prepare a 500 nmol/L dsRNA solution. This prepared dsRNA solution was further diluted with a serum-free medium to prepare a 100 nmol/L dsRNA solution. 3 µL of Lipofectamine RNAiMAX Transfection Reagent and 47 µL of a serum-free medium were added to 50 µL of this dsRNA solution and incubated at room temperature for 5 minutes. 100 µL of this dsRNA-Lipofectamine RNAiMAX Transfection Reagent mixture was diluted with 400 µL of a cell culture medium to adjust the volume to 500 µL. This diluted mixture was mixed with 500 µL of the above-described HepG2.2.15 cell suspension, seeded on a 12-well microtiter plate, and incubated in a 5% CO₂ incubator at 37°C for 3 days. Next, the medium was exchanged at 1,000 µL/well with the above-described cell culture medium, and incubation was further carried out at 37°C for 3 days in a 5% CO₂ incubator. The culture supernatant was removed, the cells were then washed with 1,000 µL/well phosphate buffered saline, and total RNA was extracted using an RNeasy Mini Kit (manufactured by Qiagen N.V.). 800 ng of the extracted total RNA was reverse-transcribed using a PrimeScript RT Reagent Kit with gDNA Eraser to prepare cDNA. YTHDC1 and GAPDH as a housekeeping gene, in the prepared cDNA, were quantified by a real-time PCR method, the YTHDC1 expression level corrected by GAPDH was calculated, and then the relative value (%) of the expression level of YTHDC1 in each of the unmodified dsRNA-added wells to the expression level of YTHDC1 in the negative control dsRNA-added well was calculated (Fig. 4).

In the duplex number #1, a slight decrease in cell viability was observed at a concentration of 1.67 nmol/L or more, while in the duplex numbers #4, #6, #9, #10, #13, #14, and #17, no effect on cell viability was observed in each concentration (Fig. 1). By each of the dsRNAs of the duplex numbers #1, #4, #6, #9, #10, #13, #14, and #17, the amount of the HBs antigen was decreased in a dose-dependent manner, and a reducing effect of 90% or more was observed in all of the HBs antigens as maximum drug efficacy (Fig. 2). The IC50 values of the HBs antigen-reducing effects by respective dsRNAs were all less than 0.1 nmol/L (Table 3). In addition, HBV DNA was decreased in a dose-dependent manner by each dsRNA, and a reducing effect of 90% or more was observed in all of the HBV DNA as the maximum drug efficacy (Fig. 3). The IC50 values of the HBV DNA-reducing effects by respective dsRNAs were all less than 0.2 nmol/L (Table 4). Then, the expression level of YTHDC1 was reduced to 50% or less by the dsRNAs of the duplex numbers #1, #4, #9, #10, #13, #14, and #17, and in particular, by the dsRNAs of the duplex numbers #4, #9, #10, #13, #14 and #17, the expression level of YTHDC1 was reduced to 70% or less (Fig. 4).

**[Table 3]**

| IC50 value of HBs antigen-reducing effect by unmodified dsRNA | |
|---|---|
| Duplex number | IC50 nmol/L |
| #1 | 0.0478 |
| #4 | 0.0298 |
| #6 | 0.0986 |
| #9 | 0.0114 |
| #10 | 0.0879 |
| #13 | 0.0163 |
| #14 | 0.0168 |
| #17 | 0.0220 |

**[Table 4]**

| IC50 value of HBV DNA-reducing effect by unmodified dsRNA | |
|---|---|
| Duplex number | IC50 nmol/L |
| #1 | 0.0519 |
| #4 | 0.0174 |
| #6 | 0.164 |
| #9 | <0.00229 |
| #10 | <0.00229 |
| #13 | 0.00602 |
| #14 | 0.00976 |

### [Test Example 3]

### (Screening of modified dsRNA for YTHDC1)

Using HepG2.2.15 cells, the knockdown effect on YTHDC1 mRNA and anti-HBV activity of the designed modified dsRNA were evaluated by the following procedure.

The following medium was used as a cell culture medium.

DMEM/F-12, GlutaMAX + 5 µg/mL insulin + 1% penicillin/streptomycin + 10 mmol/L HEPES + 50 µmol/L hydrocortisone + 10% FBS

The modified dsRNAs used for the transfection have the sequences shown in Table 5 (designated by the duplex number #32 to the duplex number #224). In addition, a modified dsRNA for firefly luciferase (the sequence shown in Table 5, designated by the duplex number #225) was used as a negative control dsRNA. HepG2.2.15 cells were suspended in the above medium to prepare a HepG2.2.15 cell suspension of 2 × 10⁵ cells/mL. The dsRNA was diluted with Nuclease-Free Water (not DEPC-Treated) to prepare a 500 nmol/L dsRNA solution. This prepared dsRNA solution was further diluted with a serum-free medium to prepare a 100 nmol/L dsRNA solution. 0.3 µL of Lipofectamine RNAiMAX Transfection Reagent and 4.7 µL of a serum-free medium were added to 5 µL of this dsRNA solution and incubated at room temperature for 5 minutes. 10 µL of this dsRNA-Lipofectamine RNAiMAX Transfection Reagent mixture was diluted with 40 µL of a cell culture medium to adjust the volume to 50 µL. This diluted mixture was mixed with 50 µL of the above-described HepG2.2.15 cell suspension, seeded on a 96-well microtiter plate, and incubated in a 5% CO₂ incubator at 37°C for 3 days. Next, the medium was exchanged at 100 µL/well with the above-described cell culture medium, and incubation was further carried out at 37°C for 3 days in a 5% CO₂ incubator. Then, the culture supernatant was collected, and the cell viability was measured using CellTiter96 AQueous One Solution Cell Proliferation Assay (Table 6). After measuring the cell viability, the cells were lysed with ISOGENII (manufactured by Nippon Gene Co., LTD.). A total RNA was extracted from the cell lysate using a DIRECT-zol-96 MagBead RNA Kit (manufactured by ZYMO RESEARCH). 7 µL of the extracted total RNA was reverse-transcribed using a PrimeScript RT Reagent Kit with gDNA Eraser to prepare cDNA. YTHDC1 and GAPDH in the prepared cDNA were quantified by a real-time PCR method, the YTHDC1 expression level corrected by GAPDH was calculated, and then the relative value (%) of the expression level of YTHDC1 in each of the dsRNA-added wells to the expression level of YTHDC1 in the negative control dsRNA-added well was calculated (Table 6). In addition, the amount of the HBs antigen in the collected culture supernatant was measured using AlphaLISA Hepatitis B Virus Surface Antigen (HBsAg) Kit. The relative value (%) in each of the dsRNA-added wells to the negative control dsRNA-added well was calculated (Table 6).

By 123 types of dsRNAs of the duplex numbers #38, #40, #42, #43, #44, #46, #48, #51, #52, #55, #56, #57, #59, #64, #66, #68, #69, #70, #71, #72, #75, #76, #79, #80, #82, #84, #87, #88, #89, #91, #92, #93, #94, #97, #98, #99, #100, #101, #102, #103, #105, #106, #107, #110, #111, #113, #117, #118, #119, #121, #122, #123, #124, #125, #126, #127, #128, #129, #130, #131, #132, #133, #134, #135, #140, #141, #142, #143, #144, #145, #146, #147, #148, #151, #154, #156, #158, #161, #165, #166, #168, #172, #173, #174, #175, #176, #178, #179, #180, #182, #184, #186, #189, #190, #193, #195, #196, #197, #198, #199, #200, #201, #202, #203, #205, #206, #207, #208, #209, #211, #212, #213, #214, #215, #216, #217, #218, #219, #220, #221, #222, and #223 among the modified dsRNAs for YTHDC1, the expression level of YTHDC1 mRNA was reduced by 30% or more. In addition, no decrease in cell viability (cell viability of less than 70%) by these 123 types of dsRNAs was observed.

By 85 types of dsRNAs of the duplex numbers #38, #42, #43, #44, #46, #57, #59, #66, #69, #72, #79, #82, #84, #87, #89, #91, #92, #93, #97, #99, #100, #101, #105, #106, #107, #110, #111, #117, #121, #122, #124, #125, #126, #127, #128, #129, #130, #131, #132, #140, #141, #142, #143, #144, #145, #147, #148, #151, #154, #158, #172, #174, #175, #176, #178, #179, #180, #182, #186, #189, #190, #195, #197, #198, #199, #200, #202, #203, #205, #206, #207, #208, #209, #212, #213, #214, #215, #216, #217, #218, #219, #220, #221, #222, and #223 among these 123 types of dsRNAs, the expression level of YTHDC1 mRNA was reduced by 50% or more.

By 36 types of dsRNAs of the duplex numbers #57, #59, #82, #89, #92, #97, #105, #106, #111, #122, #124, #128, #129, #132, #141, #143, #144, #145, #147, #151, #158, #172, #174, #176, #178, #179, #180, #197, #198, #205, #207, #208, #213, #214, #222, and #223 among these 85 types of dsRNAs, the expression level of YTHDC1 mRNA was reduced by 70% or more.

Furthermore, by 33 types of dsRNAs of the duplex numbers #57, #59, #82, #89, #92, #97, #105, #106, #122, #124, #128, #129, #132, #141, #143, #144, #147, #151, #158, #174, #176, #178, #179, #180, #197, #198, #205, #207, #208, #213, #214, #222, and #223 among these 36 types of dsRNAs, the HBs antigens in the culture supernatant were all decreased by 50% or more.

Among them, by 17 types of dsRNAs of the duplex numbers #57, #82, #92, #105, #106, #128, #143, #144, #158, #174, #176, #179, #180, #198, #207, #208, and #223, the HBs antigens in the culture supernatant were all decreased by 70% or more. In addition, by 15 types of dsRNAs of the duplex numbers #57, #82, #92, #105, #106, #143, #144, #158, #174, #179, #180, #198, #207, #208, and #223, the HBV DNAs in the culture supernatant were all reduced by 50% or more.

**[Table 5]**

| **Sequences of sense strands and antisense strands of modified dsRNA for YTHDC1** | | | | | |
|---|---|---|---|---|---|
| **Duplex number** | **SEQ ID NO** | **Sequence of sense strand (5' to 3')** | **SEQ ID NO** | **Sequence of antisense strand (5' to 3')** | **Position on YTHDC1 cDNA (SEQ ID NO: 63)** |
| #32 | 64 | a s u s G f a u g A f A f C f u g U f a u a a U f s c s a | 65 | u s G f s a u u u a u a c a g u u C f a u c a u s c s u | 417-436 |
| #33 | 66 | a s a s A f g u g A f U f C f g a A f u g g a A f s u s a | 67 | usAf suuccauucga uCfacuuususu | 480-499 |
| #34 | 68 | a s g s U f g a u C f G f A f a u G f g a a u C f s u s a | 69 | usAfsgauuccauuc gAfucacususu | 482-501 |
| #35 | 70 | u s g s G f a a u C f U f A f c u G f a u a c C f s a s a | 71 | usUfsgguaucagua gAfuuccasusu | 492-511 |
| #36 | 72 | gsgsAfaucUfAfCfu gAfuaccAfsasa | 73 | u s U f s u g g u a u c a g u a G f a u u c c s a s u | 493-512 |
| #37 | 74 | u s c s U f a c u G f A f U f a c C f a a a c G f s a s a | 75 | u s U f s c g u u u g g u a u c A f g u a g a s u s u | 497-516 |
| #38 | 76 | c s u s A f c u g A f U f A f c c A f a a c g A f s c s a | 77 | u s G f s u c g u u u g g u a u C f a g u a g s a s u | 498-517 |
| $39 | 78 | usasCfugaUfAfCfc aAfacgaCfsasa | 79 | usUfsgucguuuggu aUfcaguasgsa | 499-518 |
| #40 | 80 | a s c s U f g a u A f C f C f a a A f c g a c A f s a s a | 81 | u s U f s u g u c g u u u g g u A f u c a g u s a s g | 500-519 |
| #41 | 82 | a s c s G f a c a A f A f A f g c C f u u c u G f s u s a | 83 | usAfscagaaggcuu uUfgucgususu | 511-530 |
| #42 | 84 | a s c s A f a c u G f G f U f u u C f u a a g C f s c s a | 85 | u s G f s g c u u a g a a a c c A f g u u g u s c s u | 538-557 |
| #43 | 86 | u s c s A f u c u G f U f U f a g C f a a u a A f s c s a | 87 | u s G f s u u u a u u g c u a a c A f g a u g a s g s c | 563-582 |
| #44 | 88 | a s a s U f a g u U f A f G f u a C f a a a a G f s g s a | 89 | u s C f s c u u u u g u a c u a A f c u a u u s c s u | 586-605 |
| #45 | 90 | g s a s A f a c a A f G f C f g u C f u a g a U f s g s a | 91 | u s C f s a u c u a g a c g c u U f g u u u c s u s u | 651-670 |
| #46 | 92 | a s a s A f c a a G f C f G f u c U f a g a u G f s c s a | 93 | u s G f s c a u c u a g a c g c U f u g u u u s c s u | 652-671 |
| #47 | 94 | a s a s C f a a g C f G f U f c u A f g a u g C f s u s a | 95 | u s A f s g c a u c u a g a c g C f u u g u u s u s c | 653-672 |
| #48 | 96 | g s c s G f u c u A f G f A f u g C f u g a u C f s g s a | 97 | u s C f s g a u c a g c a u c u A f g a c g c s u s u | 658-677 |
| #49 | 98 | u s c s U f a g a U f G f C f u g A f u c g g A f s a s a | 99 | u s U f s u c c g a u c a g c a U f c u a g a s c s g | 661-680 |
| #50 | 100 | c s u s A f g a u G f C f U f g a U f c g g a A f s a s a | 101 | u s U f s u u c c g a u c a g c A f u c u a g s a s c | 662-681 |
| #51 | 102 | c s u s G f a u c G f G f A f a a A f u u c g U f s c s a | 103 | u s G f s a c g a a u u u u c c G f a u c a g s c s a | 669-688 |
| #52 | 104 | u s g s A f u c g G f A f A f a a U f u c g u C f s u s a | 105 | u s A f s g a c g a a u u u u c C f g a u c a s g s c | 670-689 |
| #53 | 106 | a s u s C f g g a A f A f A f u u C f g u c u A f s u s a | 107 | u s A f s u a g a c g a a u u u U f c c g a u s c s a | 672-691 |
| #54 | 108 | u s c s G f g a a A f A f U f u c G f u c u a U f s c s a | 109 | u s G f s a u a g a c g a a u u U f u c c g a s u s c | 673-692 |
| #55 | 110 | g s g s A f a a a U f U f C f g u C f u a u c A f s a s a | 111 | u s U f s u g a u a g a c g a a U f u u u c c s g s a | 675-694 |
| #56 | 112 | a s a s A f u u c G f U f C f u a U f c a a g U f s a s a | 113 | u s U f s a c u u g a u a g a c G f a a u u u s u s c | 678-697 |
| #57 | 114 | a s u s U f c g u C f U f A f u c A f a g u a G f s u s a | 115 | u s A f s c u a c u u g a u a g A f c g a a u s u s u | 680-699 |
| #58 | 116 | u s c s G f u c u A f U f C f a a G f u a g u G f s c s a | 117 | u s G f s c a c u a c u u g a u A f g a c g a s a s u | 682-701 |
| #59 | 118 | u s g s A f a a a A f A f C f c u G f u g u c C f s g s a | 119 | u s C f s g g a c a c a g g u u U f u u u c a s g s g | 727-746 |
| #60 | 120 | a s a s A f a c c U f G f U f g u C f c g g a A f s a s a | 121 | u s U f s u u c c g g a c a c a G f g u u u u s u s u | 731-750 |
| #61 | 122 | c s c s G f g a a A f A f G f g g A f u c c u G f s a s a | 123 | u s U f s c a g g a u c c c u u U f u c c g g s a s c | 742-761 |
| #62 | 124 | g s a s A f a g g A f G f G f g c C f a a a u C f s u s a | 125 | u s A f s g a u u u g g c c c u C f c u u u c s a s g | 758-777 |
| #63 | 126 | c s a s A f a u c U f C f C f u a C f g c c a G f s a s a | 127 | u s U f s c u g g c g u a g g a G f a u u u g s g s c | 769-788 |
| #64 | 128 | a s a s U f c u c C f U f A f c g C f c a g a U f s g s a | 129 | u s C f s a u c u g g c g u a g G f a g a u u s u s g | 771-790 |
| #65 | 130 | c s c s U f a c g C f C f A f g a U f g g u u C f s u s a | 131 | u s A f s g a a c c a u c u g g C f g u a g g s a s g | 776-795 |
| #66 | 132 | g s a s A f u u g G f G f C f u u G f a a g u G f s g s a | 133 | u s C f s c a c u u c a a g c c C f a a u u c s u s c | 798-817 |
| #67 | 134 | g s g s G f c u u G f A f A f g u G f g a u a G f s a s a | 135 | u s U f s c u a u c c a c u u c A f a g c c c s a s a | 803-822 |
| #68 | 136 | c s u s U f g a a G f U f G f g a U f a g a c G f s u s a | 137 | usAfscgucuauccca cUfucaagscsc | 806-825 |
| #69 | 138 | a s g s A f c g u G f C f A f a g C f a g a u C f s c s a | 139 | u s G f s g a u c u g c u u g c A f c g u c u s a s u | 818-837 |
| #70 | 140 | g s a s C f g u g C f A f A f g c A f g a u c C f s a s a | 141 | u s U f s g g a u c u g c u u g C f a c g u c s u s a | 819-838 |
| #71 | 142 | a s u s G f a u u A f U f G f a c A f c u c g A f s a s a | 143 | u s U f s u c g a g u g u c a u A f a u c a u s u s u | 1122-1141 |
| #72 | 144 | u s g s A f u u a U f G f A f c a C f u c g a A f s g s a | 145 | u s C f s u u c g a g u g u c a U f a a u c a s u s u | 1123-1142 |
| #73 | 146 | g s a s U f u a u G f A f C f a c U f c g a a G f s u s a | 147 | u s A f s c u u c g a g u g u c A f u a a u c s a s u | 1124-1143 |
| #74 | 148 | a s u s U f a u g A f C f A f c u C f g a a g U f s g s a | 149 | u s C f s a c u u c g a g u g u C f a u a a u s c s a | 1125-1144 |
| #75 | 150 | u s u s A f u g a C f A f C f u c G f a a g u G f s a s a | 151 | u s U f s c a c u u c g a g u g U f c a u a a s u s c | 1126-1145 |
| #76 | 152 | a s u s G f a c a C f U f C f g a A f g u g a G f s g s a | 153 | usCfscucacuucga gUfgucausasa | 1128-1147 |
| #77 | 154 | c s u s C f g a a G f U f G f a g G f c c a g U f s g s a | 155 | u s C f s a c u g g c c u c a c U f u c g a g s u s g | 1134-1153 |
| #78 | 156 | a s g s A f g a g C f U f A f g a G f g c a u A f s u s a | 157 | u s A f s u a u g c c u c u a g C f u c u c u s u s c | 1242-1261 |
| #79 | 158 | g s g s U f c c a C f G f C f u c C f c u g u A f s a s a | 159 | u s U f s u a c a g g g a g c g U f g g a c c s a s u | 1413-1432 |
| #80 | 160 | g s u s C f c a c G f C f U f c c C f u g u a A f s a s a | 161 | u s U f s u u a c a g g g a g c G f u g g a c s c s a | 1414-1433 |
| #81 | 162 | a s a s A f u c u U f G f C f a u U f u a g a U f s c s a | 163 | u s G f s a u c u a a a u g c a A f g a u u u s a s a | 1444-1463 |
| #82 | 164 | g s g s G f u u u G f C f A f a g A f c u u u C f s u s a | 165 | u s A f s g a a a g u c u u g c A f a a c c c s u s u | 1514-1533 |
| #83 | 166 | a s a s U f c a c A f U f C f a c G f g a g g A f s u s a | 167 | u s A f s u c c u c c g u g a u G f u g a u u s c s u | 1536-1555 |
| #84 | 168 | u s c s A f c g g A f G f G f a u C f u c c u A f s u s a | 169 | usAfsuaggagaucc uCfcgugasusg | 1543-1562 |
| #85 | 170 | c s a s C f g g a G f G f A f u c U f c c u a U f s a s a | 171 | u s U f s a u a g g a g a u c c U f c c g u g s a s u | 1544-1563 |
| #86 | 172 | a s c s G f g a g G f A f U f c u C f c u a u A f s c s a | 173 | u s G f s u a u a g g a g a u c C f u c c g u s g s a | 1545-1564 |
| #87 | 174 | g s a s U f c u c C f U f A f u a C f a c u g G f s g s a | 175 | u s C f s c c a g u g u a u a g G f a g a u c s c s u | 1551-1570 |
| #88 | 176 | g s g s A f u u u G f C f A f g g C f g u g a A f s u s a | 177 | u s A f s u u c a c g c c u g c A f a a u c c s a s g | 1623-1642 |
| #89 | 178 | g s c s G f u g a A f U f U f a c C f c u u c A f s c s a | 179 | u s G f s u g a a g g g u a a u U f c a c g c s c s u | 1633-1652 |
| #90 | 180 | g s a s A f u u a C f C f C f u u C f a c u a A f s g s a | 181 | u s C f s u u a g u g a a g g g U f a a u u c s a s c | 1637-1656 |
| #91 | 182 | a s u s U f a c c C f U f U f c a C f u a a g U f s c s a | 183 | u s G f s a c u u a g u g a a g G f g u a a u s u s c | 1639-1658 |
| #92 | 184 | u s a s C f c c u U f C f A f c u A f a g u c G f s g s a | 185 | u s C f s c g a c u u a g u g a A f g g g u a s a s u | 1641-1660 |
| #93 | 186 | u s c s A f c u a A f G f U f c g G f c u c a U f s c s a | 187 | u s G f s a u g a g c c g a c u U f a g u g a s a s g | 1647-1666 |
| #94 | 188 | c s a s C f u a a G f U f C f g g C f u c a u C f s u s a | 189 | u s A f s g a u g a g c c g a c U f u a g u g s a s a | 1648-1667 |
| #95 | 190 | g s u s C f g g c U f C f A f u c U f c a c c A f s a s a | 191 | u s U f s u g g u g a g a u g a G f c c g a c s u s u | 1654-1673 |
| #96 | 192 | a s c s C f a a u C f C f U f u g G f a a u g A f s a s a | 193 | u s U f s u c a u u c c a a g g A f u u g g u s g s a | 1667-1686 |
| #97 | 194 | a s u s A f a a c C f A f G f u a A f a g a u C f s g s a | 195 | u s C f s g a u c u u u a c u g G f u u u a u s g s u | 1686-1705 |
| #98 | 196 | a s a s C f c a g U f A f A f a g A f u c g g A f s c s a | 197 | u s G f s u c c g a u c u u u a C f u g g u u s u s a | 1689-1708 |
| #99 | 198 | a s g s U f a a a G f A f U f c g G f a c g u G f s a s a | 199 | u s U f s c a c g u c c g a u c U f u u a c u s g s g | 1693-1712 |
| #100 | 200 | g s u s A f a a g A f U f C f g g A f c g u g A f s u s a | 201 | u s A f s u c a c g u c c g a u C f u u u a c s u s g | 1694-1713 |
| #101 | 202 | u s a s A f a g a U f C f G f g a C f g u g a U f s g s a | 203 | u s C f s a u c a c g u c c g a U f c u u u a s c s u | 1695-1714 |
| #102 | 204 | a s a s G f a u c G f G f A f c g U f g a u g G f s a s a | 205 | u s U f s c c a u c a c g u c c G f a u c u u s u s a | 1697-1716 |
| #103 | 206 | a s g s A f u c g G f A f C f g u G f a u g g A f s c s a | 207 | u s G f s u c c a u c a c g u c C f g a u c u s u s u | 1698-1717 |
| #104 | 208 | a s c s G f u g a U f G f G f a c A f g g a a A f s u s a | 209 | u s A f s u u u c c u g u c c a U f c a c g u s c s c | 1705-1724 |
| #105 | 210 | c s c s C f g a u G f A f A f a g U f a u u g A f s c s a | 211 | u s G f s u c a a u a c u u u c A f u c g g g s g s g | 1763-1782 |
| #106 | 212 | c s c s G f a u g A f A f A f g u A f u u g a C f s u s a | 213 | u s A f s g u c a a u a c u u u C f a u c g g s g s g | 1764-1783 |
| #107 | 214 | g s u s A f u u g A f C f U f u g U f a u c a G f s g s a | 215 | u s C f s c u g a u a c a a g u C f a a u a c s u s u | 1773-1792 |
| #108 | 216 | u s a s U f u g a C f U f U f g u A f u c a g G f s u s a | 217 | u s A f s c c u g a u a c a a g U f c a a u a s c s u | 1774-1793 |
| #109 | 218 | u s g s U f a u c A f G f G f u c A f u u c a U f s a s a | 219 | u s U f s a u g a a u g a c c u G f a u a c a s a s g | 1782-1801 |
| #110 | 220 | u s c s A f u u c A f U f A f a a A f u g c g U f s c s a | 221 | u s G f s a c g c a u u u u a u G f a a u g a s c s c | 1791-1810 |
| #111 | 222 | a s u s U f c a u A f A f A f a u G f c g u c A f s c s a | 223 | u s G f s u g a c g c a u u u u A f u g a a u s g s a | 1793-1812 |
| #112 | 224 | a s u s A f a a a U f G f C f g u C f a c a a G f s a s a | 225 | u s U f s c u u g u g a c g c a U f u u u a u s g s a | 1797-1816 |
| #113 | 226 | a s u s G f c g u C f A f C f a a G f a g a a G f s a s a | 227 | u s U f s c u u c u c u u g u g A f c g c a u s u s u | 1802-1821 |
| #114 | 228 | g s c s G f u c a C f A f A f g a G f a a g a A f s u s a | 229 | u s A f s u u c u u c u c u u g U f g a c g c s a s u | 1804-1823 |
| #115 | 230 | g s a s U f c a c G f A f G f g a C f g u c c A f s u s a | 231 | u s A f s u g g a c g u c c u c G f u g a u c s g s g | 1836-1855 |
| #116 | 232 | g s a s C f g u c C f A f U f c c C f g u c g A f s g s a | 233 | u s C f s u c g a c g g g a u g G f a c g u c s c s u | 1845-7864 |
| #117 | 234 | c s g s U f c c a U f C f C f c g U f c g a g A f s a s a | 235 | u s U f s u c u c g a c g g g a U f g g a c g s u s c | 1847-1866 |
| #118 | 236 | a s u s C f c c g U f C f G f a g A f a c c a G f s u s a | 237 | u s A f s c u g g u u c u c g a C f g g g a u s g s g | 1852-7871 |
| #119 | 238 | c s c s G f u c g A f G f A f a c C f a g u c C f s g s a | 239 | u s C f s g g a c u g g u u c u C f g a c g g s g s a | 1855-1874 |
| #120 | 240 | u s c s G f a g a A f C f C f a g U f c c g g G f s a s a | 241 | u s U f s c c c g g a c u g g u U f c u c g a s c s g | 1858-1877 |
| #121 | 242 | g s g s A f u g u G f G f G f a a G f g c g u C f s g s a | 243 | u s C f s g a c g c c u u c c c A f c a u c c s c s g | 1873-1892 |
| #122 | 244 | g s u s G f g g a A f G f G f c g U f c g a c C f s a s a | 245 | u s U f s g g u c g a c g c c u U f c c c a c s a s u | 1877-1896 |
| #123 | 246 | g s g s C f g u c G f A f C f c a G f a a g a U f s u s a | 247 | u s A f s a u c u u c u g g u c G f a c g c c s u s u | 7884-7903 |
| #124 | 248 | gscsGfucgAfCfCfa gAfagauUfsasa | 249 | u s U f s a a u c u u c u g g u C f g a c g c s c s u | 1885-7904 |
| #125 | 250 | c s g s U f c g a C f C f A f g a A f g a u u A f s u s a | 251 | u s A f s u a a u c u u c u g g U f c g a c g s c s c | 1886-1905 |
| #126 | 252 | g s u s C f g a c C f A f G f a a G f a u u a U f s g s a | 253 | u s C f s a u a a u c u u c u g G f u c g a c s g s c | 1887-1906 |
| #127 | 254 | u s c s G f a c c A f G f A f a g A f u u a u G f s a s a | 255 | u s U f s c a u a a u c u u c u G f g u c g a s c s g | 1888-1907 |
| #128 | 256 | a s g s A f a a c C f A f A f g g A f u u g a C f s u s a | 257 | u s A f s g u c a a u c c u u g G f u u u c u s u s u | 1923-7942 |
| #129 | 258 | a s a s A f c c a A f G f G f a u U f g a c u A f s u s a | 259 | u s A f s u a g u c a a u c c u U f g g u u u s c s u | 1925-1944 |
| #130 | 260 | a s a s C f c a a G f G f A f u u G f a c u a U f s c s a | 261 | u s G f s a u a g u c a a u c c U f u g g u u s u s c | 1926-1945 |
| #131 | 262 | g s u s A f u u u A f A f A f g g A f u c c a C f s g s a | 263 | u s C f s g u g g a u c c u u u A f a a u a c s c s c | 1969-1988 |
| #132 | 264 | a s u s U f u a a A f G f G f a u C f c a c g A f s u s a | 265 | u s A f s u c g u g g a u c c u U f u a a a u s a s c | 1971-7990 |
| #133 | 266 | u s u s U f a a a G f G f A f u c C f a c g a U f s a s a | 267 | u s U f s a u c g u g g a u c c U f u u a a a s u s a | 1972-1991 |
| #134 | 268 | u s u s A f a a g G f A f U f c c A f c g a u A f s c s a | 269 | u s G f s u a u c g u g g a u c C f u u u a a s a s u | 1973-1992 |
| #135 | 270 | u s a s A f a g g A f U f C f c a C f g a u a C f s c s a | 271 | u s G f s g u a u c g u g g a u C f c u u u a s a s a | 1974-1993 |
| #136 | 272 | a s g s G f a u c C f A f C f g a U f a c c a G f s g s a | 273 | u s C f s c u g g u a u c g u g G f a u c c u s u s u | 1977-1996 |
| #137 | 274 | a s u s C f c a c G f A f U f a c C f a g g a A f s g s a | 275 | u s C f s u u c c u g g u a u c G f u g g a u s c s c | 1980-7.999 |
| #138 | 276 | u s c s C f a c g A f U f A f c c A f g g a a G f s u s a | 277 | u s A f s c u u c c u g g u a u C f g u g g a s u s c | 1981-2000 |
| #139 | 278 | a s g s A f c g a U f U f U f u c A f g g a g U f s u s a | 279 | usAfsacuccugaaa aUfcgucusgsu | 2003-2022 |
| #140 | 280 | a s c s G f a u u U f U f C f a g G f a g u u C f s g s a | 281 | u s C f s g a a c u c c u g a a A f a u c g u s c s u | 2005-2024 |
| #141 | 282 | c s g s A f u u u U f C f A f g g A f g u u c G f s c s a | 283 | u s G f s c g a a c u c c u g a A f a a u c g s u s c | 2006-2025 |
| #142 | 284 | g s a s U f u u u C f A f G f g a G f u u c g C f s c s a | 285 | u s G f s g c g a a c u c c u g A f a a a u c s g s u | 2007-2026 |
| #143 | 286 | g s g s A f g u u C f G f C f c g A f g a u g U f s g s a | 287 | u s C f s a c a u c u c g g c g A f a c u c c s u s g | 2015-2034 |
| #144 | 288 | a s g s U f u c g C f C f G f a g A f u g u g U f s u s a | 289 | u s A f s a c a c a u c u c g g C f g a a c u s c s c | 2017-2036 |
| #145 | 290 | g s u s U f c g c C f G f A f g a U f g u g u U f s u s a | 291 | u s A f s a a c a c a u c u c g G f c g a a c s u s c | 2018-2037 |
| #146 | 292 | u s u s C f g c c G f A f G f a u G f u g u u U f s u s a | 293 | u s A f s a a a c a c a u c u c G f g c g a a s c s u | 2019-2038 |
| #147 | 294 | c s g s C f c g a G f A f U f g u G f u u u u U f s a s a | 295 | u s U f s a a a a a c a c a u c U f c g g c g s a s a | 2021-2040 |
| #148 | 296 | u s g s U f u u u U f A f A f a u G f g g u c C f s u s a | 297 | u s A f s g g a c c c a u u u a A f a a a c a s c s a | 2031-2050 |
| #149 | 298 | u s g s G f g u c C f U f A f c a A f u g a u U f s a s a | 299 | u s U f s a a u c a u u g u a g G f a c c c a s u s u | 2041-2060 |
| #150 | 300 | g s g s G f u c c U f A f C f a a U f g a u u A f s u s a | 301 | u s A f s u a a u c a u u g u a G f g a c c c s a s u | 2042-2061 |
| #151 | 302 | g s g s U f c c u A f C f A f a u G f a u u a U f s g s a | 303 | u s C f s a u a a u c a u u g u A f g g a c c s c s a | 2043-2062 |
| #152 | 304 | u s u s U f c a u A f A f C f a u G f g g a c C f s a s a | 305 | u s U f s g g u c c c a u g u u A f u g a a a s u s u | 2069-2088 |
| #153 | 306 | a s u s A f a a c G f A f G f u a C f a u g a U f s u s a | 307 | u s A f s a u c a u g u a c u c G f u u u a u s c s u | 2232-2251 |
| #154 | 308 | usasAfacgAfGfUfa cAfugauUfsasa | 309 | u s U f s a a u c a u g u a c u C f g u u u a s u s c | 2233-2252 |
| #155 | 310 | a s a s A f c g a G f U f A f c a U f g a u u A f s u s a | 311 | u s A f s u a a u c a u g u a c U f c g u u u s a s u | 2234-2253 |
| #156 | 312 | a s a s C f g a g U f A f C f a u G f a u u a U f s g s a | 313 | u s C f s a u a a u c a u g u a C f u c g u u s u s a | 2235-2254 |
| #157 | 314 | u s g s A f u u a U f G f A f u a U f g a g g G f s u s a | 315 | u s A f s c c c u c a u a u c a U f a a u c a s u s g | 2245-2264 |
| #158 | 316 | g s a s U f g a u U f U f C f c u U f c g u c G f s c s a | 317 | usGfscgacgaagga aAfucaucscsa | 2264-2283 |
| #159 | 318 | a s u s G f a u u U f C f C f u u C f g u c g C f s a s a | 319 | u s U f s g c g a c g a a g g a A f a u c a u s c s c | 2265-2284 |
| #160 | 320 | usgsAfuuuCfCfUfu cGfucgcAfscsa | 321 | u s G f s u g c g a c g a a g g A f a a u c a s u s c | 2266-2285 |
| #161 | 322 | a s u s U f u c c U f U f C f g u C f g c a c A f s c s a | 323 | u s G f s u g u g c g a c g a a G f g a a a u s c s a | 2268-2287 |
| #162 | 324 | u s u s C f g u c G f C f A f c a C f a a g c U f s g s a | 325 | u s C f s a g c u u g u g u g c G f a c g a a s g s g | 2274-2293 |
| #163 | 326 | g s a s G f a a g U f A f G f a c C f c c g u G f s a s a | 327 | u s U f s c a c g g g g u c u a C f u u c u c s c s g | 2305-2324 |
| #164 | 328 | a s g s U f a g a C f C f C f c g U f g a a a G f s a s a | 329 | u s U f s c u u u c a c g g g g U f c u a c u s u s c | 2309-2328 |
| #165 | 330 | a s a s C f g a g A f G f C f g a G f a c c g C f s c s a | 331 | u s G f s g c g g u c u c g c u C f u c g u u s c s c | 2334-2353 |
| #166 | 332 | a s c s G f a g a G f C f G f a g A f c c g c C f s c s a | 333 | u s G f s g g c g g u c u c g c U f c u c g u s u s c | 2335-2354 |
| #167 | 334 | a s g s A f g c g A f G f A f c c G f c c c u A f s g s a | 335 | u s C f s u a g g g c g g u c u C f g c u c u s c s g | 2338-2357 |
| #168 | 336 | g s a s C f c g c C f C f U f a g A f g a u a A f s c s a | 337 | u s G f s u u a u c u c u a g g G f c g g u c s u s c | 2345-2364 |
| #169 | 338 | a s c s C f g c c C f U f A f g a G f a u a a C f s a s a | 339 | u s U f s g u u a u c u c u a g G f g c g g u s c s u | 2346-2365 |
| #170 | 340 | g s c s C f c u a G f A f G f a u A f a c a g A f s c s a | 341 | u s G f s u c u g u u a u c u c U f a g g g c s g s g | 2349-2368 |
| #171 | 342 | c s c s C f u a g A f G f A f u a A f c a g a C f s g s a | 343 | u s C f s g u c u g u u a u c u C f u a g g g s c s g | 2350-2369 |
| #172 | 344 | a s g s A f g a u A f A f C f a g A f c g a g A f s c s a | 345 | u s G f s u c u c g u c u g u u A f u c u c u s a s g | 2354-2373 |
| #173 | 346 | a s u s A f a c a G f A f C f g a G f a c a g A f s g s a | 347 | u s C f s u c u g u c u c g u c U f g u u a u s c s u | 2358-2377 |
| #174 | 348 | g s a s G f c g a G f A f U f a g A f g g a c G f s u s a | 349 | u s A f s c g u c c u c u a u c U f c g c u c s u s c | 2375-2394 |
| #175 | 350 | g s c s G f a g a U f A f G f a g G f a c g u G f s a s a | 351 | u s U f s c a c g u c c c u c u a U f c u c g c s u s c | 2377-2396 |
| #176 | 352 | a s g s A f g a g C f G f A f u u A f u g u g A f s u s a | 353 | u s A f s u c a c a u a a u c g C f u c u c u s u s u | 2408-2427 |
| #177 | 354 | g s a s G f a g c G f A f U f u a U f g u g a U f s c s a | 355 | u s G f s a u c a c a u a a u c G f c u c u c s u s u | 2409-2428 |
| #178 | 356 | a s g s A f g c g A f U f U f a u G f u g a u C f s g s a | 357 | u s C f s g a u c a c a u a a u C f g c u c u s c s u | 2410-2429 |
| #179 | 358 | g s a s G f c g a U f U f A f u g U f g a u c G f s a s a | 359 | u s U f s c g a u c a c a u a a U f c g c u c s u s c | 2411-2430 |
| #180 | 360 | a s g s C f g a u U f A f U f g u G f a u c g A f s g s a | 361 | u s C f s u c g a u c a c a u a A f u c g c u s c s u | 2412-2431 |
| #181 | 362 | c s g s A f u u a U f G f U f g a U f c g a g A f s c s a | 363 | u s G f s u c u c g a u c a c a U f a a u c g s c s u | 2414-2433 |
| #182 | 364 | g s g s A f g a g A f G f G f u c G f a u a u A f s g s a | 365 | u s C f s u a u a u c g a c c u C f u c u c c s c s c | 2443-2462 |
| #183 | 366 | g s g s U f c g a U f A f U f a g A f a g a u A f s a s a | 367 | u s U f s u a u c u u c u a u a U f c g a c c s u s c | 2450-2469 |
| #184 | 368 | c s c s A f a g c A f U f G f c a G f u a u c A f s u s a | 369 | u s A f s u g a u a c u g c a u G f c u u g g s a s a | 2612-2631 |
| #185 | 370 | c s a s A f g c a U f G f C f a g U f a u c a U f s a s a | 371 | u s U f s a u g a u a c u g c a U f g c u u g s g s a | 2613-2632 |
| #186 | 372 | g s g s A f a a a A f C f U f c a A f a u c c G f s c s a | 373 | u s G f s c g g a u u u g a g u U f u u u c c s a s g | 2637-2656 |
| #187 | 374 | a s a s A f a c u C f A f A f a u C f c g c c A f s a s a | 375 | u s U f s u g g c g g a u u u g A f g u u u u s u s c | 2640-2659 |
| #188 | 376 | a s a s A f u c c G f C f C f a a A f a a u c C f s a s a | 377 | u s U f s g g a u u u u u g g c G f g a u u u s g s a | 2647-2666 |
| #189 | 378 | u s u s U f u a u U f G f G f g g C f a g a a U f s g s a | 379 | u s C f s a u u c u g c c c c a A f u a a a a s g s u | 2687-2706 |
| #190 | 380 | usus U f a u u G f G f G f g c A f g a a u G f s g s a | 381 | u s C f s c a u u c u g c c c c A f a u a a a s a s g | 2688-2707 |
| #191 | 382 | a s g s U f c c a A f G f A f a u G f u a g a U f s a s a | 383 | u s U f s a u c u a c a u u c u U f g g a c u s g s u | 2709-2728 |
| #192 | 384 | u s c s U f u a u A f G f U f g u G f u u c a U f s c s a | 385 | u s G f s a u g a a c a c a c u A f u a a g a s a s c | 2749-2768 |
| #193 | 386 | u s a s U f a g u G f U f G f u u C f a u c c U f s a s a | 387 | u s U f s a g g a u g a a c a c A f c u a u a s a s g | 2752-2771 |
| #194 | 388 | a s u s A f g u g U f G f U f u c A f u c c u A f s g s a | 389 | u s C f s u a g g a u g a a c a C f a c u a u s a s a | 2753-2772 |
| #195 | 390 | u s g s U f u u c U f A f C f a u G f u a g u U f s g s a | 391 | u s C f s a a c u a c a u g u a G f a a a c a s c s a | 2842-2861 |
| #196 | 392 | u s c s U f a c a U f G f U f a g U f u g c a C f s a s a | 393 | u s U f s g u g c a a c u a c a U f g u a g a s a s a | 2846-2865 |
| #197 | 394 | a s c s A f u g u A f G f U f u g C f a c a c G f s g s a | 395 | u s C f s c g u g u g c a a c u A f c a u g u s a s g | 2849-2868 |
| #198 | 396 | g s u s A f g u u G f C f A f c a C f g g u u C f s a s a | 397 | u s U f s g a a c c g u g u g c A f a c u a c s a s u | 2853-2872 |
| #199 | 398 | g s u s U f g c a C f A f C f g g U f u c a g U f s a s a | 399 | u s U f s a c u g a a c c g u g U f g c a a c s u s a | 2856-2875 |
| #200 | 400 | u s u s G f c a c A f C f G f g u U f c a g u A f s a s a | 401 | u s U f s u a c u g a a c c g u G f u g c a a s c s u | 2857-2876 |
| #201 | 402 | a s c s A f c g g U f U f C f a g U f a a a a A f s u s a | 403 | u s A f s u u u u u a c u g a a C f c g u g u s g s c | 2861-2880 |
| #202 | 404 | g s u s A f u g a U f G f G f u u U f g a c u G f s u s a | 405 | u s A f s c a g u c a a a c c a U f c a u a c s u s u | 2909-2928 |
| #203 | 406 | a s g s U f g u u G f U f A f g c A f g c c u C f s u s a | 407 | u s A f s g a g g c u g c u a c A f a c a c u s g s c | 2932-2951 |
| #204 | 408 | a s g s A f c a u U f G f C f u a U f c u a a U f s u s a | 409 | u s A f s a u u a g a u a g c a A f u g u c u s u s c | 3041-3060 |
| #205 | 410 | u s c s C f u a u G f U f U f c a G f u a g c G f s u s a | 411 | u s A f s c g c u a c u g a a c A f u a g g a s a s u | 3082-3101 |
| #206 | 412 | c s c s U f a u g U f U f C f a g U f a g c g U f s g s a | 413 | u s C f s a c g c u a c u g a a C f a u a g g s a s a | 3083-3102 |
| #207 | 414 | u s a s U f g u u C f A f G f u a G f c g u g G f s u s a | 415 | u s A f s c c a c g c u a c u g A f a c a u a s g s g | 3085-3104 |
| #208 | 416 | u s g s U f u c a G f U f A f g c G f u g g u U f s g s a | 417 | u s C f s a a c c a c g c u a c U f g a a c a s u s a | 3087-3106 |
| #209 | 418 | g s u s U f c a g U f A f G f c g U f g g u u G f s a s a | 419 | u s U f s c a a c c a c g c u a C f u g a a c s a s u | 3088-3107 |
| #210 | 420 | u s a s G f c g u G f G f U f u g A f u g c u A f s u s a | 421 | u s A f s u a g c a u c a a c c A f c g c u a s c s u | 3094-3113 |
| #211 | 422 | g s g s A f a u g U f U f C f a c A f a c u u G f s c s a | 423 | usGfscaaguuguga aCfauuccsasa | 3149-3168 |
| #212 | 424 | a s u s G f u u c A f C f A f a c U f u g c g U f s g s a | 425 | u s C f s a c g c a a g u u g u G f a a c a u s u s c | 3152-3171 |
| #213 | 426 | u s g s U f u c a C f A f A f c u U f g c g u G f s c s a | 427 | u s G f s c a c g c a a g u u g U f g a a c a s u s u | 3153-3172 |
| #214 | 428 | g s u s U f c a c A f A f C f u u G f c g u g C f s g s a | 429 | u s C f s g c a c g c a a g u u G f u g a a c s a s u | 3154-3173 |
| #215 | 430 | c s a s C f a a c U f U f G f c g U f g c g u G f s g s a | 431 | u s C f s c a c g c a c g c a a G f u u g u g s a s a | 3157-3176 |
| #216 | 432 | a s c s A f a c u U f G f C f g u G f c g u g G f s c s a | 433 | u s G f s c c a c g c a c g c a A f g u u g u s g s a | 3158-3177 |
| #217 | 434 | c s g s U f g c g U f G f G f c a G f c g g a A f s g s a | 435 | u s C f s u u c c g c u g c c a C f g c a c g s c s a | 3166-3185 |
| #218 | 436 | g s g s C f a g c G f G f A f a g A f c g a u U f s c s a | 437 | u s G f s a a u c g u c u u c c G f c u g c c s a s c | 3173-3192 |
| #219 | 438 | g s c s A f g c g G f A f A f g a C f g a u u C f s c s a | 439 | u s G f s g a a u c g u c u u c C f g c u g c s c s a | 3174-3193 |
| #220 | 440 | a s g s C f g g a A f G f A f c g A f u u c c C f s a s a | 441 | u s U f s g g g a a u c g u c u U f c c g c u s g s c | 3176-3195 |
| #221 | 442 | a s a s G f a c g A f U f U f c c C f a u a u U f s c s a | 443 | u s G f s a a u a u g g g a a u C f g u c u u s c s c | 3181-3200 |
| #222 | 444 | a s g s A f c g a U f U f C f c c A f u a u u C f s u s a | 445 | u s A f s g a a u a u g g g a a U f c g u c u s u s c | 3182-3201 |
| #223 | 446 | g s a s C f g a u U f C f C f c a U f a u u c U f s a s a | 447 | u s U f s a g a a u a u g g g a A f u c g u c s u s u | 3183-3202 |
| #224 | 448 | a s c s G f a u u C f C f C f a u A f u u c u A f s c s a | 449 | u s G f s u a g a a u a u g g g A f a u c g u s c s u | 3184-3203 |
| #225 | 450 | c s u s U f a c g C f U f G f a g U f a c u u C f g a | 451 | u s C f s g a a g u a c u c a g C f g u a a g s u s u | - |

**[Table 6]**

| **Evaluation of activity of modified dsRNA for YTHDC1** | | | | |
|---|---|---|---|---|
| **Duplex number** | **Cell viability %** | **Relative expression level of YTHDCl mRNA %** | **Relative amount of HBs antigen %** | **Relative amount of HBV DNA %** |
| #32 | 55.4±5.2 | 54.8±3.7 | 21.1±2.6 | 54±2.9 |
| #33 | 63.7±0.5 | 92.2±6 | 30.5±1.2 | 77.3±8.1 |
| #34 | 71.8±4.4 | 75.8±33.1 | 37±4.8 | 70.4±11.7 |
| #35 | 100.5±8.6 | 80.3±5.3 | 143.1±7.9 | 148.1±17.8 |
| #36 | 63.8±5 | 36.5±3.5 | 22.2±6.4 | 40.8±9.1 |
| #37 | 101.5±3.2 | 94.5±68 | 37.9±3.1 | 58.5±10.4 |
| #38 | 103±15.8 | 33.3±2.7 | 30.8±8.1 | 42.7±7.7 |
| #39 | 83.2±7.1 | 170.3±173.8 | 17.8±2.8 | 36.6±8.2 |
| #40 | 88.6±5.9 | 62.8±4.2 | 31.4±5.8 | 62.7±16 |
| #41 | 49.3±2.9 | 57.8±3.1 | 27±0.7 | 60±4.2 |
| #42 | 71.3±3.1 | 36.9±4 | 25.4±0.5 | 44±4.6 |
| #43 | 88.7±6.9 | 49.6±5.2 | 40.8±4.6 | 65.5±13.7 |
| #44 | 94±3.2 | 44.4±2.6 | 29.3±7.5 | 50.2±9.6 |
| #45 | 71±3.4 | 161.2±165.3 | 36.2±2.7 | 40.8±6.3 |
| #46 | 97.1±2.6 | 45.2±1 | 20.3±3.3 | 43.2±8.8 |
| #47 | 108.2±6.4 | 147.5±172.9 | 100.4±5.9 | 103.5±40.2 |
| #48 | 106.9±5.2 | 61.6±72.9 | 2±7.9±4.2 | 45.1±6.7 |
| #49 | 65.1±2.4 | 42.6±3.6 | 11.3±1.7 | 34.5±7.4 |
| #50 | 80.3±8.6 | 72.1±18.5 | 16.4±3.1 | 24±2.2 |
| #51 | 101.6±4.1 | 57.8±11.6 | 18.7±1.4 | 40.7±9.3 |
| #52 | 79.1±2.9 | 56.1±2 | 13.3±6.5 | 42.2±2.8 |
| #53 | 43±0.6 | 66.6±4±7.7 | 8.6±0.4 | 34.8±9.6 |
| #54 | 31.2±1.9 | 95.7±7.5 | 11.3+0.4 | 44.9±21.1 |
| #55 | 97.4±8.7 | 56.1±33.7 | 21.7±2.5 | 32.2±9.9 |
| #56 | 124.7±8.4 | 52.1±8.3 | 38.7±2.5 | 91.3±29 |
| #57 | 106.8±6.3 | 22.9±1.7 | 19±2.5 | 35.2±9.5 |
| # 58 | 114.1±3 | 80.3±48.7 | 123.8±14.8 | 134.9±9.1 |
| #59 | 101.1±1.7 | 29.3±0.4 | 34.6±3 | 46.2±11.2 |
| #60 | 79.4±0.5 | 104±4.6 | 58.9±10.3 | 27.4±8.2 |
| #61 | 102.4±2.4 | 90.2±2.4 | 50.3±8.7 | 31.3±3.9 |
| #62 | 77.8±2.8 | 83.3±8.8 | 54.2±5.1 | 66.3±1.1 |
| #63 | 79.4±5.2 | 70.4±0.5 | 33.1±1.9 | 29±0.4 |
| #64 | 105.7±5.6 | 59.1±5.4 | 90.3±9.7 | 178.7±6.5 |
| #65 | 87.7±2.1 | 86.9±46.3 | 51.3±6.5 | 34.2±8.5 |
| #66 | 111.1±6.3 | 37.2±0.3 | 71.6±7 | 42±3 |
| #67 | 88.2±6.1 | 78.6±1.5 | 56.9±4.2 | 40.4±1.6 |
| #68 | 89±5.8 | 59.6±2.2 | 55.3±6.6 | 123.8±21.3 |
| #69 | 91.9±3.2 | 38.7±2.6 | 38.4±6.6 | 26.4±0.9 |
| #70 | 91.3±4.1 | 58.5±3.3 | 77.3±5.3 | 44.3±2.8 |
| #71 | 80±7.2 | 58.7±4.7 | 40.9±2.4 | 39.8±0.8 |
| #72 | 74.2±5.6 | 42±3.6 | 26.4±7 | 34.2±4.1 |
| #73 | 56.5±2 | 57.9±12.6 | 32.1±2 | 55.7±37.5 |
| #74 | 71.4±4.3 | 81.2±45.6 | 28.9±3.8 | 30±1.5 |
| #75 | 76±1.8 | 50.1±6.4 | 19.6±1.6 | 26+2.2 |
| #76 | 108±11.2 | 61.9±2.9 | 103.6±5.9 | 63.6±11.8 |
| #77 | 71.7±6.4 | 104.6±7.8 | 43.6±4.5 | 114.3±12.9 |
| #78 | 95.7±11.9 | 114.7±4.6 | 71.3±2.9 | 64.6±5 |
| #79 | 94.6±6.5 | 41.3±1.9 | 21.2±2 | 32.1±4.8 |
| #80 | 108.1±5.2 | 55.3±3.9 | 99.9±5.8 | 66.8±14.3 |
| #81 | 57.2±8.8 | 122.8±8.7 | 52.8±3.2 | 49.8±0.8 |
| #82 | 91±9.9 | 25±1.9 | 13.7±2.4 | 27.1±4.3 |
| #83 | 96.2±8.8 | 81.1±3 | 83.6±8.1 | 51.6±15.6 |
| #84 | 72.8±4.4 | 38.9±7.4 | 29±1.2 | 26.9±6.2 |
| #85 | 23.8±1.3 | 59.5±6.7 | 32.3±1 | 47.6±1 |
| #86 | 87.7±4.9 | 87±6.1 | 112±4.5 | 87.7±3.2 |
| #87 | 107.6±2.5 | 37.8±4.2 | 90.3±5.9 | 65.1±3.8 |
| #88 | 72.9±4.8 | 59.9±3.4 | 34.4±3.6 | 59.1±12.5 |
| #89 | 83.1±4.9 | 24.2±1.9 | 47.6±5.1 | 42.6±2.1 |
| #90 | 56.4±4.3 | 26.9±1.3 | 27±4.7 | 41.4±1.7 |
| #91 | 95.6±4.6 | 41.2±5 | 89.2±1.9 | 78.9±1.8 |
| #92 | 96.5±7.6 | 24.8±1.1 | 29.4±1.7 | 37.8±1.1 |
| #93 | 71.5±1 | 43.3±1.2 | 27±1.3 | 40.8±1.9 |
| #94 | 101.2±3.6 | 67.5±3.9 | 37.6±4.1 | 38.1±6.9 |
| #95 | 57.6±2.7 | 74±7.9 | 39±2.2 | 36.4±2.6 |
| #96 | 60.1±1.9 | 39.2±3.7 | 24.2±3.8 | 41.4±3 |
| #97 | 98.9±1.3 | 25.5±1 | 47.6±2.6 | 33.9±0.5 |
| #98 | 90.5±3.8 | 51.7±1 | 29.1±3 | 28±1.5 |
| #99 | 86.3±1.7 | 42.9±1.7 | 36.1±4.1 | 38.8±1.8 |
| #100 | 106.6±7.1 | 46.6±3.6 | 66.2±4.5 | 96.8±13.1 |
| #101 | 109.2±1.2 | 42.9±3.5 | 56.1±5.2 | 39±6.8 |
| #102 | 106.9±2.9 | 67.3±14.3 | 87.4±5.3 | 86.5±6.2 |
| #103 | 96.6±2.2 | 51±15.1 | 51.4±1.5 | 62.5±6.2 |
| #104 | 73±4 | 94±3.5 | 13.9±4.2 | 35.4±1.9 |
| #105 | 103.2±4.1 | 15.4±2.4 | 27.9±3 | 35.9±2 |
| #106 | 86.5±7.2 | 26.9±1.9 | 28.6±2.5 | 41.9±3.3 |
| #107 | 93.2±3.6 | 32.7±1.2 | 55±9.3 | 44.1±3.4 |
| #108 | 40.1±2.6 | 78.1±7 | 33.9±1.4 | 83.8±17.6 |
| #109 | 41.5±0.9 | 46.8±9.4 | 10.1±0.8 | 40.7±0.7 |
| #110 | 99.7±5.4 | 32.4±1.3 | 42±6.5 | 57.7±3.9 |
| #111 | 100.3±9.4 | 28.8±0.6 | 65.3±2.1 | 67.3±5.3 |
| #112 | 66.1±7 | 109.4±3.9 | 31.4±4.2 | 34.1±5.2 |
| #113 | 79.3±5.6 | 59±6.4 | 154.7±6 | 65.8±22.9 |
| #114 | 69±0.6 | 52.3±3.3 | 17.7±6.4 | 31.3±9.2 |
| #115 | 110.5±2.8 | 75.7±3.8 | 64.8±2.8 | 85.8±8.7 |
| #116 | 116.5±9.8 | 75.7±30.4 | 165.5±18.2 | 91.8±6.8 |
| #117 | 85.2±9.7 | 43.8±5.9 | 50.9±3.3 | 42.7±1 |
| #118 | 98.5±6.1 | 53.4±1.8 | 107.1±5.7 | 71.8±10 |
| #119 | 89±5.7 | 55.5±6 | 90.3±34.5 | 71.4±15.4 |
| #120 | 98.1±5.7 | 89.3±3.5 | 63.2±5.1 | 179.9±13.9 |
| #121 | 116.6±7.6 | 38.7±2.1 | 52.3±10.5 | 78.8±7.8 |
| #122 | 129.7±10.7 | 27.6+2.1 | 49.5±4.5 | 37.1±4.6 |
| #123 | 100.1±5.1 | 60±4.3 | 136±23.2 | 76.3±6.6 |
| #124 | 112.8±6.4 | 26.8±1.2 | 37.6±4 | 32.1±3.7 |
| #125 | 108.9±4.2 | 40.1±1.2 | 41.8±8.5 | 36.24,3 |
| #126 | 116±3.3 | 31.6±0.9 | 86.8±14.4 | 77.6±4.6 |
| #127 | 98.7±13.5 | 34.1±3.2 | 80.5±20.7 | 69.5±11 |
| #128 | 85.8±6.9 | 27±2.3 | 21.7±5 | 56.8±3 |
| #129 | 123.6±8.8 | 25.3±0.2 | 33.6±6.9 | 31.3±10.2 |
| #130 | 80.1±7.2 | 32.2±2 | 19±4.1 | 41±4.4 |
| #131 | 130.9±3.4 | 30.4±0.7 | 123±3.4 | 69.7±10.8 |
| #132 | 116.8±4 | 28.1±4 | 48.9±10.8 | 38.9±4.5 |
| #133 | 80.7±3.7 | 57.1±1.1 | 34.7±5.2 | 49.4±4.2 |
| #134 | 116±8.1 | 50.8±1.5 | 91.9±18.5 | 75.2±9.5 |
| #135 | 113.1±5.8 | 59.9±3.7 | 182.9±20.5 | 127±4.2 |
| #136 | 115±15.3 | 74.1±1.7 | 152.1±3.4 | 95.8±3.7 |
| #137 | 84.1±12.1 | 74.6±4.8 | 142±14.9 | 149±8.3 |
| #138 | 78.3±12.9 | 258.2±274.8 | 72.3±8.8 | 154.8±17.1 |
| #139 | 82.2±11.5 | 126.4±110.6 | 20.7±1.4 | 63.2±6.2 |
| #140 | 128.7±4.9 | 32±2.8 | 67.9±2.1 | 50.2±0.3 |
| #141 | 120.5±5.7 | 21.7±2 | 35.3±8.8 | 43±3.1 |
| #142 | 94.6±12.2 | 37.3±5.6 | 34.5±7.6 | 47.8±3.6 |
| #143 | 90.4±5.7 | 26.1±1.8 | 23.7±2.2 | 37.2±5.8 |
| #144 | 102.9±4.4 | 17.5±1.5 | 21.1±5.8 | 34.7±6.3 |
| #145 | 126.9±9.8 | 26.2±1.2 | 202.3±202.6 | 53.9±4.5 |
| #146 | 110.1±3.5 | 56.7±6.7 | 138.5±3 | 94.9±11.6 |
| #147 | 93.3±3.4 | 12.9±2.3 | 33.1±2.9 | 37.9±1.8 |
| #148 | 83.8±0.6 | 32.3±3.8 | 32.4±13.5 | 46.5±2.7 |
| #149 | 107.3±8 | 79±9.6 | 37.9±6.1 | 71.6±6 |
| #150 | 121.7±11 | 76.1±3.5 | 112.3±1.9 | 118.3±3.7 |
| #151 | 101.2±6.2 | 28.4±4.8 | 45.2±7.3 | 45±2.2 |
| #152 | 116.5±3.3 | 74.2±8.4 | 84.2±4 | 83.2±0.9 |
| #153 | 46±3.1 | 33±3.5 | 37.3±3.1 | 46±9.5 |
| #154 | 75.5±3 | 36.8±2.2 | 25.7±5.7 | 32.6±5.6 |
| #155 | 66±1 | 54.7±6.5 | 54.2±2.4 | 60.4±12.1 |
| #156 | 101.3±10.3 | 56.4±7.4 | 85.9±2.7 | 93.2±12.2 |
| #157 | 27.3±2.1 | 88±3.8 | 38.4±3.2 | 78.6±5.2 |
| #158 | 87.8±2.2 | 21.5±1.8 | 20.7±2.2 | 37±2.8 |
| #159 | 56.2±2.1 | 21.4±3.1 | 10.3±7 | 41.4±5.3 |
| #160 | 55±1.9 | 24.8±1.1 | 5.6±2.5 | 38.2±1.8 |
| #161 | 105.8±4.1 | 52.5±4.4 | 193.9±23.2 | 95.7±10.3 |
| #162 | 64.2±2.2 | 69±3.2 | 50.6±4.5 | 72.8±15.5 |
| #163 | 58.3±0.7 | 89.7±7.8 | 19.6±0.6 | 49.4±4.9 |
| #164 | 62±5.1 | 139.8±23.6 | 28.1±3.7 | 45±3.6 |
| #165 | 85.3±11.4 | 63.8±6.8 | 95.3±12.3 | 109.2±5 |
| #166 | 74.7±7.6 | 51.5±3.2 | 49.7±10.1 | 73.9±4.8 |
| #167 | 53.4±4.3 | 39.4±3.6 | 27.6±11.8 | 59.5±6.8 |
| #168 | 91.9±8.8 | 66.9±5.9 | 125.8±11.8 | 93.1±3.9 |
| #169 | 114.8±6.5 | 87.8±6.6 | 25.5±8.4 | 56.4±3 |
| #170 | 64.8±7.5 | 39.3±4.6 | 20±8.3 | 54.4±3.6 |
| #171 | 52.4±4.1 | 19.2±1.3 | 33±1.7 | 48.1±11.5 |
| #172 | 114.5±1.4 | 29.4±1.3 | 52.5±7.6 | 52.9±4.9 |
| #173 | 78.6±1.3 | 50.6±0.8 | 15±8.2 | 39.1±6.1 |
| #174 | 97.8±4.4 | 24.3±1.2 | 26.1±3.2 | 34.3±5.6 |
| #175 | 89.2±6.8 | 41.8±1.9 | 25.1±3 | 40.9±4.1 |
| #176 | 79.8±0.7 | 16.4±1.1 | 23.1±4.1 | 51.2±6.3 |
| #177 | 67.5±4.2 | 54.3±3.7 | 83.2±7.7 | 89±8.4 |
| #178 | 107.5±0.6 | 27.3±0.7 | 37.4±7.5 | 41.4±1.7 |
| #179 | 107.9±1.2 | 14.5±0.7 | 16±6.1 | 27.3±3 |
| #180 | 80.1±1.4 | 24.2±1.4 | 28.52 | 35.4±2.7 |
| #181 | 57.2±1.1 | 36±4.6 | 29±5.5 | 45.9±2.7 |
| #182 | 75±2.1 | 33.8±2.8 | 11.4±2.4 | 28.6±2.1 |
| #183 | 66.4±0.8 | 39±1.1 | 18.9±1.3 | 41.3±2.6 |
| #184 | 127.5±9.1 | 50.2±3 | 96.3±11.5 | 101.7±23 |
| #185 | 43.4±1.9 | 84.2±4.6 | 40.5±4.9 | 53.2±4.7 |
| #186 | 91.5±2.6 | 30.1±0.7 | 32.2±2.6 | 42±4 |
| #187 | 68.1±0.8 | 42.3±1.8 | 32.9±49.9 | 27.7±1.8 |
| #188 | 114±2.8 | 55.6±4.5 | 72.1±8.1 | 66.1±2.1 |
| #189 | 102.7±8.6 | 45.8±1.6 | 35.4±0.8 | 38.2±1.7 |
| #190 | 118.4±5.5 | 39.3±1.1 | 48.4±1.4 | 42.2±6.5 |
| #191 | 46.9±3.6 | 63.8±7.4 | 8.4±1.1 | 50.5±7.1 |
| #192 | 62±1.7 | 59.5±0.7 | 20.8±1 | 41±6.3 |
| #193 | 79.3±6.6 | 50.9±0.7 | 33.5±3.3 | 47.4±3.8 |
| #194 | 54.2±2.1 | 50±1.7 | 79.2±8.4 | 46.6±2.8 |
| #195 | 85.5±4.6 | 46.8±3 | 73±6.4 | 64.5±4.5 |
| #196 | 71.2±4.7 | 64.5±3.3 | 97.3±12.4 | 47.5±6 |
| #197 | 130.5±0.4 | 24±1.8 | 49±6.2 | 44.9±4.3 |
| #198 | 106.1±2.4 | 22.9±1.4 | 17.5±4.5 | 30.6±3.2 |
| #199 | 107.4±1.9 | 33.1±1.2 | 86.6±8.7 | 38.1±2.3 |
| #200 | 111.9±6.3 | 38.1±2.8 | 134.7±7.4 | 63.5±2 |
| #201 | 90.1±5.5 | 59.1±1.5 | 42.7±5.5 | 40.7±4.7 |
| #202 | 82.9±5.9 | 38.5±4.2 | 58.7±6.4 | 55.4±5.1 |
| #203 | 87.6±6.3 | 31±1.6 | 18.1±5.6 | 55.1±2.4 |
| #204 | 23.3±2.2 | 54±1 | 18.9±5.5 | 77.6±2.2 |
| #205 | 120.6±5.4 | 25.2±0.6 | 39.7±3.1 | 50.6±1.3 |
| #206 | 110.1±6.3 | 35.1±14.7 | 35.6±6.6 | 42.4±3.4 |
| #207 | 108.6±0.2 | 28.3±2.1 | 28.7±3.3 | 38±5.6 |
| #208 | 112.3±6.7 | 28.2±2 | 26.8±2.9 | 49.9±2.7 |
| #209 | 97.5±5.1 | 45±1.9 | 82.5±7.4 | 50±6.5 |
| #210 | 40.3±4.2 | 42.6±3.4 | 20.6±3.5 | 47.4±8.9 |
| #211 | 102.3±2.3 | 66.5±0.7 | 110.2±9.1 | 98.4±1.3 |
| #212 | 90.6±6.1 | 31.6±2.8 | 42.5±4 | 58.8±6.9 |
| #213 | 96.4±0.6 | 26.3±2.3 | 45.2±6.6 | 45.7±5.9 |
| #214 | 110±5.1 | 23.3±1 | 42.2±6.1 | 41.7±8.7 |
| #215 | 136±1.8 | 41.6±0.5 | 10.9±4.2 | 27.8±6.7 |
| #216 | 108±6.5 | 30.5±2.7 | 63.2±3 | 54.4±4.5 |
| #217 | 84±0.5 | 34.1±2.3 | 14.9±0.7 | 37±8.7 |
| #248 | 103.7±8 | 39.8±1.7 | 5 9.5±1.5 | 40.3±8.2 |
| #219 | 75.2±3.6 | 46.8±1 | 131.1±14.8 | 87.5±9.3 |
| #220 | 115.3±10.9 | 33.8±5.6 | 33.5±1.3 | 46.2±4.6 |
| #221 | 86.1±9.1 | 44.2±2.9 | 40.5±9.4 | 53.4±2.8 |
| #222 | 88.7+0.6 | 27±2 | 37.1±3.9 | 44.2±5.7 |
| #223 | 106.8±4.7 | 22.7±1.4 | 27.1±8.5 | 34.1±4 |
| #224 | 45.6±3.8 | 38.2±2.5 | 33.2±5.2 | 50.6±5 |

### [Test Example 4]

### (Evaluation of activity of modified dsRNA for YTHDC1)

Using HepG2.2.15 cells, the activities of 10 types of dsRNAs of the duplex numbers #57, #82, #128, #144, #158, #176, #179, #198, #208, and #223 among the modified dsRNAs found in Test Example 3 were evaluated by the following procedure.

The following medium was used as a cell culture medium.

DMEM/F-12, GlutaMAX + 5 µg/mL insulin + 1% penicillin/streptomycin + 10 mmol/L HEPES + 50 µmol/L hydrocortisone + 10% FBS

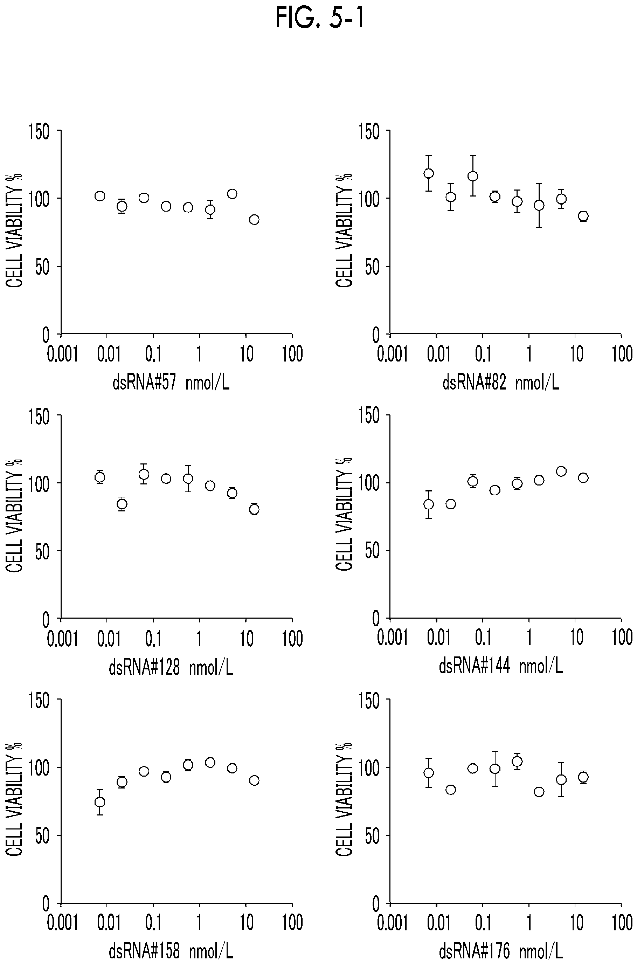

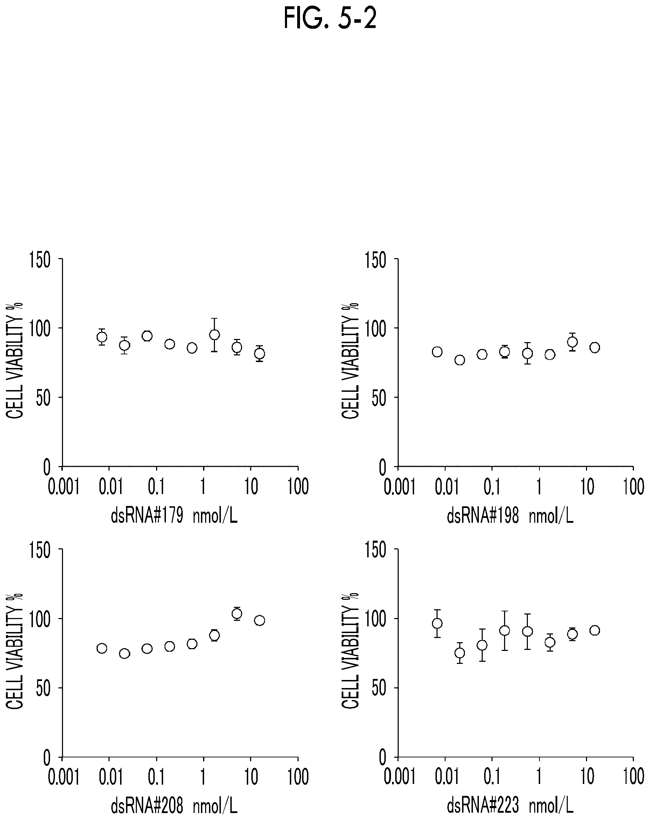

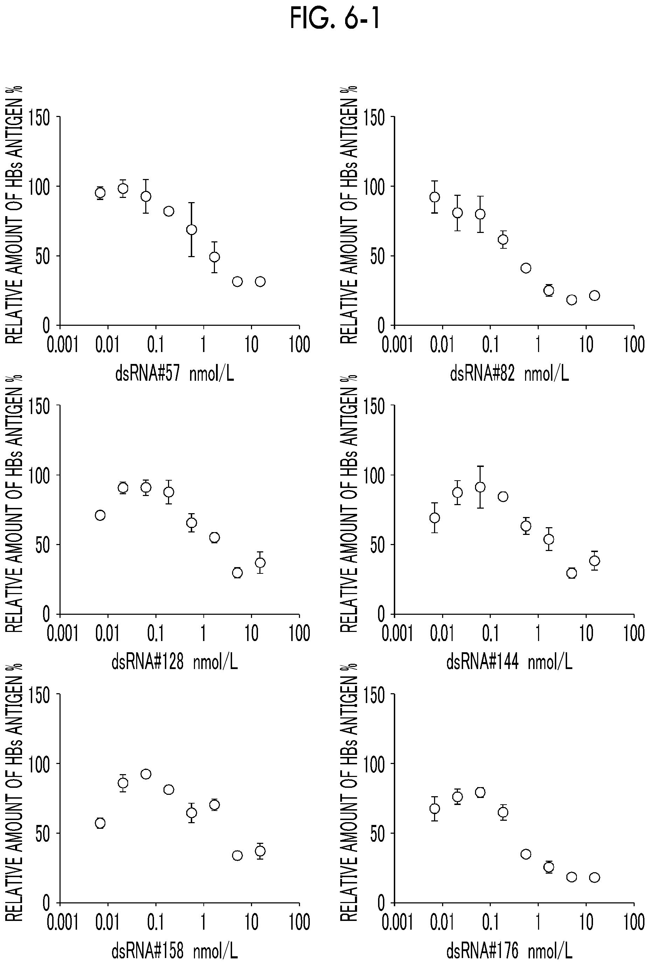

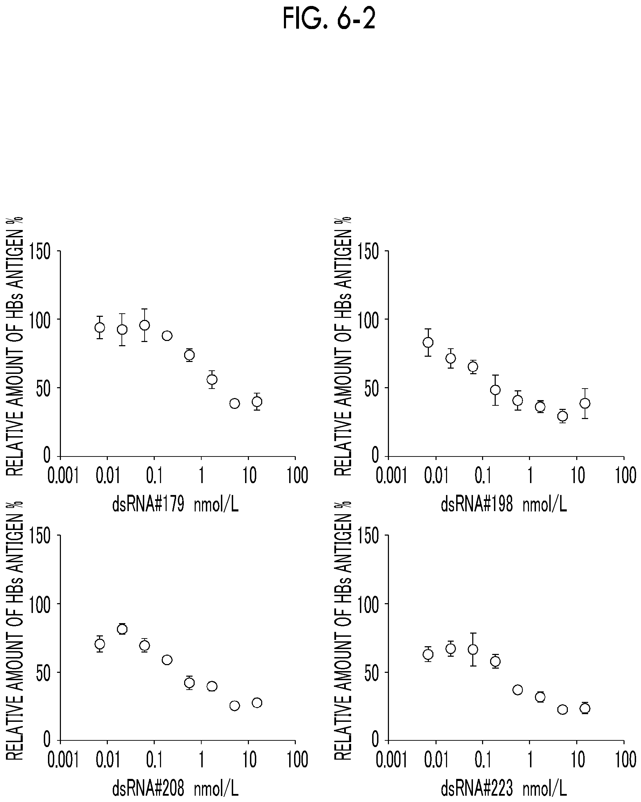

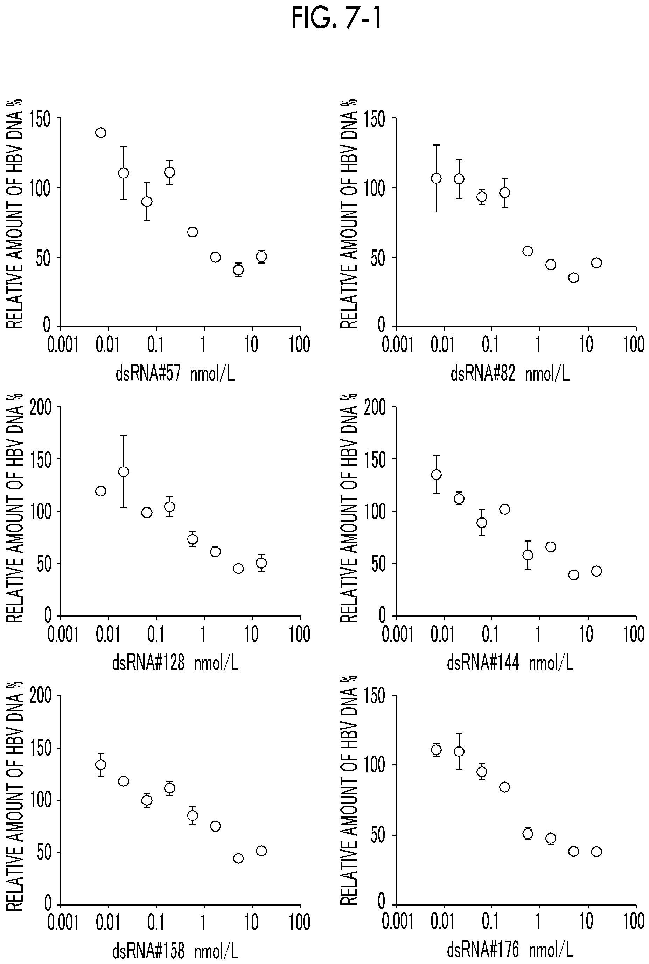

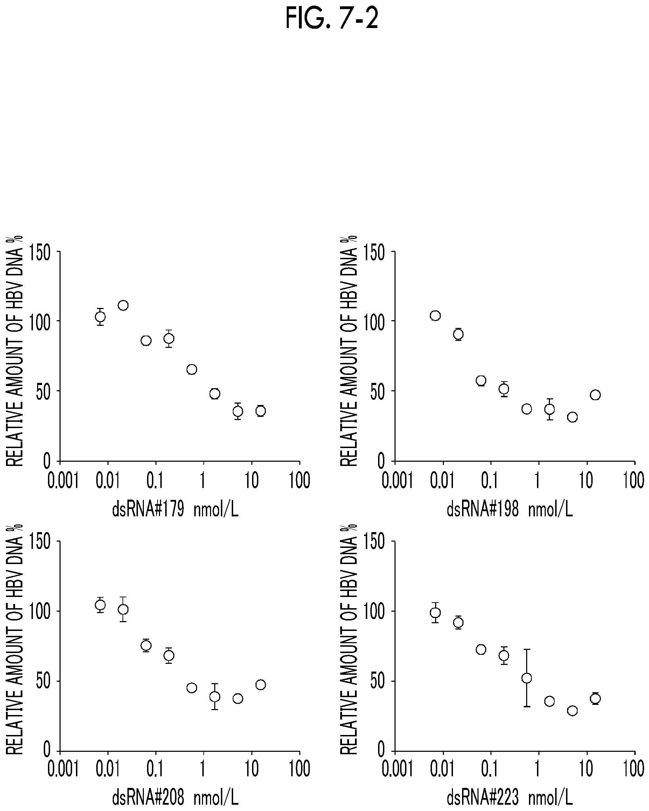

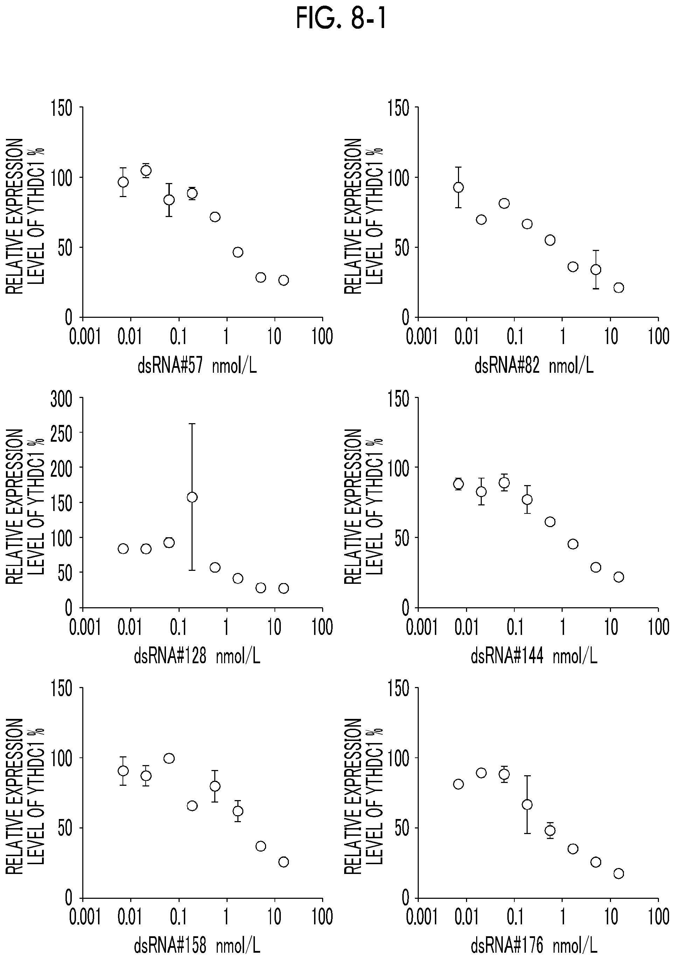

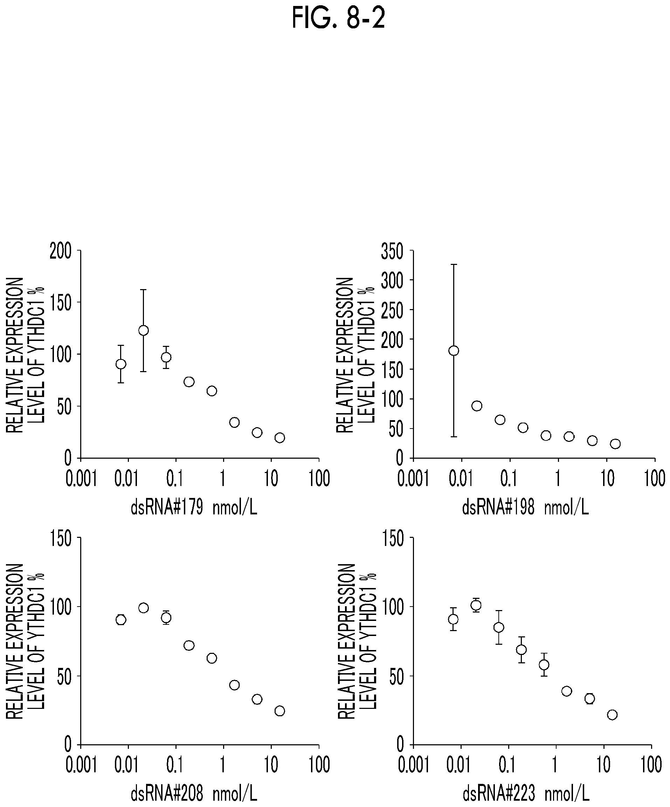

The dsRNAs used for the transfection have the sequences shown in Table 5 (designated by the duplex numbers #57, #82, #128, #144, #158, #176, #179, #198, #208, and #223). In addition, a modified dsRNA for firefly luciferase (the sequence shown in Table 5, designated by the duplex number #225) was used as a negative control dsRNA. HepG2.2.15 cells were suspended in the above medium to prepare a HepG2.2.15 cell suspension of 2 × 10⁵ cells/mL. The dsRNA was diluted with Nuclease-Free Water (not DEPC-Treated) to prepare dsRNA solutions of 3-fold dilution series from 0.686 to 1,500 nmol/L. These prepared dsRNA solutions were further diluted with a serum-free medium to prepare a dsRNA solution of 3-fold dilution series from 0.137 to 300 nmol/L. 0.3 µL of Lipofectamine RNAiMAX Transfection Reagent and 4.7 µL of a serum-free medium were added to each concentration of 5 µL of dsRNA solutions and incubated at room temperature for 5 minutes. 10 µL of this dsRNA-Lipofectamine RNAiMAX Transfection Reagent mixture was diluted with 40 µL of a cell culture medium to adjust the volume to 50 µL. This diluted mixture was mixed with 50 µL of the above-described HepG2.2.15 cell suspension, seeded on a 96-well microtiter plate, and incubated in a 5% CO₂ incubator at 37°C for 3 days. Next, the medium was exchanged at 100 µL/well with the above-described cell culture medium, and incubation was further carried out at 37°C for 3 days in a 5% CO₂ incubator. Then, the culture supernatant was collected, and the cell viability was measured using CellTiter96 AQueous One Solution Cell Proliferation Assay (Fig. 5). The amount of the HBs antigen in the collected culture supernatant was measured using AlphaLISA Hepatitis B Virus Surface Antigen (HBsAg) Kit. The relative value (%) in each of the dsRNA-added wells to the negative control dsRNA-added well was calculated (Fig. 6). Furthermore, the collected culture supernatant was treated with a mixture of Buffer AL and Proteinase K, and the DNA of HBV (the HBV DNA) in the obtained solution was quantified by a real-time PCR method. The relative value (%) in each of the dsRNA-added wells to the negative control dsRNA-added well was calculated (Fig. 7). In addition, after measuring the cell viability, the cells were lysed with ISOGENII. A total RNA was extracted from the cell lysate using a DIRECT-zol-96 MagBead RNA Kit. 7 µL of the extracted total RNA was reverse-transcribed using a PrimeScript RT Reagent Kit with gDNA Eraser to prepare cDNA. YTHDC1 and GAPDH in the prepared cDNA were quantified by a real-time PCR method, the YTHDC1 expression level corrected by GAPDH was calculated, and then the relative value (%) of the expression level of YTHDC1 in each of the dsRNA-added wells to the expression level of YTHDC1 in the negative control dsRNA-added well was calculated (Fig. 8).

No effect on cell viability was observed in any of concentrations for 10 types of dsRNAs of the duplex numbers #57, #82, #128, #144, #158, #176, #179, #198, #208, and #223 (Fig. 5). By each dsRNA, the amount of the HBs antigen was decreased generally in a dose-dependent manner, and a reducing effect of 60% or more was observed in all of the HBs antigens as maximum drug efficacy (Fig. 6). Among them, a reducing effect of 70% or more was observed in all of the HBs antigens as maximum drug efficacy by the dsRNA of the duplex numbers #82, #128, #144, #176, #198, #208, and #223, and in particular, a reducing effect of 80% or more was observed in all of the HBs antigens as maximum drug efficacy by the dsRNA of the duplex numbers #82 and #176. The IC50 values of the HBs antigen-reducing effects by respective dsRNAs were all less than 5 nmol/L (Table 7). In addition, HBV DNA was decreased generally in a dose-dependent manner by each dsRNA, and a reducing effect of 50% or more was observed in all of the HBV DNAs as the maximum drug efficacy (Fig. 7). The IC50 values of the HBV DNA-reducing effects by respective dsRNAs were all less than 5 nmol/L (Table 8). Then, by respective dsRNAs, the expression level of YTHDC1 was decreased generally in a dose-dependent manner, and all of the expression levels of YTHDC1 were decreased to 70% or less as the maximum drug efficacy (Fig. 8). The IC50 values of the YTHDC1 expression level-reducing effects by respective dsRNAs were all less than 5 nmol/L (Table 9).

**[Table 7]**

| IC50 value of HBs antigen-reducing effect by modified dsRNA | |
|---|---|
| Duplex number | IC50 nmol/L |
| #57 | 1.5798 |
| #82 | 0.3454 |
| #128 | 2.0756 |
| #144 | 1.9795 |
| #158 | 3.0665 |
| #176 | 0.3181 |
| #179 | 2.4261 |
| #198 | 0.1660 |
| #208 | 0.3372 |
| #223 | 0.2840 |

**[Table 8]**

| IC50 value of HBV DNA-reducing effect by modified dsRNA | |
|---|---|
| Duplex number | IC50 nmol/L |
| #57 | 1.6451 |
| #82 | 0.8969 |
| #128 | 3.5857 |
| #144 | 3.1911 |
| #158 | 4.0624 |
| #176 | 0.7587 |
| #179 | 1.4665 |
| #198 | 0.2062 |
| #208 | 0.4445 |
| #223 | 0.6486 |

**[Table 9]**

| IC50 value of YTHDC1 expression level-reducing effect by unmodified dsRNA | |
|---|---|
| Duplex number | IC50 nmol/L |
| #57 | 1.4243 |
| #82 | 0.7395 |
| #128 | 0.9279 |
| #144 | 1.2107 |
| #158 | 2.8179 |
| #176 | 0.4941 |
| #179 | 0.9436 |
| #198 | 0.2052 |
| #208 | 1.1558 |
| #223 | 0.8891 |

The double-stranded RNA according to the embodiment of the present invention exhibits an excellent anti-HBV activity and is useful as an anti-hepatitis B virus agent.

### [Sequence list]

International application 20F01304W1JP21035781_29.app based on International Patent Cooperation Treaty

## Claims

1. A double-stranded RNA that inhibits production of a hepatitis B virus protein,
wherein the double-stranded RNA is formed from a sense strand and an antisense strand, and
the sense strand includes a nucleobase sequence of nucleotide, which is represented by any one of SEQ ID NOs: 1, 7, 11, 13, 17, 19, 25, 27, 29, 31, 33, 35, 37, 76, 80, 84, 86, 88, 92, 96, 102, 104, 110, 112, 114, 118, 128, 132, 136, 138, 140, 142, 144, 150, 152, 158, 160, 164, 168, 174, 176, 178, 182, 184, 186, 188, 194, 196, 198, 200, 202, 204, 206, 210, 212, 214, 220, 222, 226, 234, 236, 238, 242, 244, 246, 248, 250, 252, 254, 256, 258, 260, 262, 264, 266, 268, 270, 280, 282, 284, 286, 288, 290, 292, 294, 296, 302, 308, 312, 316, 322, 330, 332, 336, 344, 346, 348, 350, 352, 356, 358, 360, 364, 368, 372, 378, 380, 386, 390, 392, 394, 396, 398, 400, 402, 404, 406, 410, 412, 414, 416, 418, 422, 424, 426, 428, 430, 432, 434, 436, 438, 440, 442, 444, or 446, or includes a nucleobase sequence having a sequence identity of 90% or more with the nucleobase sequence.

2. The double-stranded RNA according to claim 1,
wherein the antisense strand includes a nucleobase sequence of nucleotide, which is represented by any one of SEQ ID NOs: 2, 8, 12, 14, 18, 20, 26, 28, 30, 32, 34, 36, 38, 77, 81, 85, 87, 89, 93, 97, 103, 105, 111, 113, 115, 119, 129, 133, 137, 139, 141, 143, 145, 151, 153, 159, 161, 165, 169, 175, 177, 179, 183, 185, 187, 189, 195, 197, 199, 201, 203, 205, 207, 211, 213, 215, 221, 223, 227, 235, 237, 239, 243, 245, 247, 249, 251, 253, 255, 257, 259, 261, 263, 265, 267, 269, 271, 281, 283, 285, 287, 289, 291, 293, 295, 297, 303, 309, 313, 317, 323, 331, 333, 337, 345, 347, 349, 351, 353, 357, 359, 361, 365, 369, 373, 379, 381, 387, 391, 393, 395, 397, 399, 401, 403, 405, 407, 411, 413, 415, 417, 419, 423, 425, 427, 429, 431, 433, 435, 437, 439, 441, 443, 445, or 447, or includes a nucleobase sequence having a sequence identity of 90% or more with the nucleobase sequence.

3. The double-stranded RNA according to claim 1 or claim 2,
wherein a combination of the sense strand and the antisense strand is selected from the following dsRNAs,
(a) a dsRNA formed from a sense strand including a nucleobase sequence of a nucleotide represented by SEQ ID NO: 1 and an antisense strand including a nucleobase sequence of a nucleotide represented by SEQ ID NO: 2,
(b) a dsRNA formed from a sense strand including a nucleobase sequence of a nucleotide represented by SEQ ID NO: 7 and an antisense strand including a nucleobase sequence of a nucleotide represented by SEQ ID NO: 8,
(c) a dsRNA formed from a sense strand including a nucleobase sequence of a nucleotide represented by SEQ ID NO: 11 and an antisense strand including a nucleobase sequence of a nucleotide represented by SEQ ID NO: 12,
(d) a dsRNA formed from a sense strand including a nucleobase sequence of a nucleotide represented by SEQ ID NO: 13 and an antisense strand including a nucleobase sequence of a nucleotide represented by SEQ ID NO: 14,
(e) a dsRNA formed from a sense strand including a nucleobase sequence of a nucleotide represented by SEQ ID NO: 17 and an antisense strand including a nucleobase sequence of a nucleotide represented by SEQ ID NO: 18,
(f) a dsRNA formed from a sense strand including a nucleobase sequence of a nucleotide represented by SEQ ID NO: 19 and an antisense strand including a nucleobase sequence of a nucleotide represented by SEQ ID NO: 20,
(g) a dsRNA formed from a sense strand including a nucleobase sequence of a nucleotide represented by SEQ ID NO: 25 and an antisense strand including a nucleobase sequence of a nucleotide represented by SEQ ID NO: 26,
(h) a dsRNA formed from a sense strand including a nucleobase sequence of a nucleotide represented by SEQ ID NO: 27 and an antisense strand including a nucleobase sequence of a nucleotide represented by SEQ ID NO: 28,
(i) a dsRNA formed from a sense strand including a nucleobase sequence of a nucleotide represented by SEQ ID NO: 29 and an antisense strand including a nucleobase sequence of a nucleotide represented by SEQ ID NO: 30,
(j) a dsRNA formed from a sense strand including a nucleobase sequence of a nucleotide represented by SEQ ID NO: 31 and an antisense strand including a nucleobase sequence of a nucleotide represented by SEQ ID NO: 32,
(k) a dsRNA formed from a sense strand including a nucleobase sequence of a nucleotide represented by SEQ ID NO: 33 and an antisense strand including a nucleobase sequence of a nucleotide represented by SEQ ID NO: 34,
(1) a dsRNA formed from a sense strand including a nucleobase sequence of a nucleotide represented by SEQ ID NO: 35 and an antisense strand including a nucleobase sequence of a nucleotide represented by SEQ ID NO: 36,
(m) a dsRNA formed from a sense strand including a nucleobase sequence of a nucleotide represented by SEQ ID NO: 37 and an antisense strand including a nucleobase sequence of a nucleotide represented by SEQ ID NO: 38,
(n) a dsRNA formed from a sense strand including a nucleobase sequence of a nucleotide represented by SEQ ID NO: 76 and an antisense strand including a nucleobase sequence of a nucleotide represented by SEQ ID NO: 77,
(o) a dsRNA formed from a sense strand including a nucleobase sequence of a nucleotide represented by SEQ ID NO: 80 and an antisense strand including a nucleobase sequence of a nucleotide represented by SEQ ID NO: 81,
(p) a dsRNA formed from a sense strand including a nucleobase sequence of a nucleotide represented by SEQ ID NO: 84 and an antisense strand including a nucleobase sequence of a nucleotide represented by SEQ ID NO: 85,
(q) a dsRNA formed from a sense strand including a nucleobase sequence of a nucleotide represented by SEQ ID NO: 86 and an antisense strand including a nucleobase sequence of a nucleotide represented by SEQ ID NO: 87,
(r) a dsRNA formed from a sense strand including a nucleobase sequence of a nucleotide represented by SEQ ID NO: 88 and an antisense strand including a nucleobase sequence of a nucleotide represented by SEQ ID NO: 89,
(s) a dsRNA formed from a sense strand including a nucleobase sequence of a nucleotide represented by SEQ ID NO: 92 and an antisense strand including a nucleobase sequence of a nucleotide represented by SEQ ID NO: 93,
(t) a dsRNA formed from a sense strand including a nucleobase sequence of a nucleotide represented by SEQ ID NO: 96 and an antisense strand including a nucleobase sequence of a nucleotide represented by SEQ ID NO: 97,
(u) a dsRNA formed from a sense strand including a nucleobase sequence of a nucleotide represented by SEQ ID NO: 102 and an antisense strand including a nucleobase sequence of a nucleotide represented by SEQ ID NO: 103,
(v) a dsRNA formed from a sense strand including a nucleobase sequence of a nucleotide represented by SEQ ID NO: 104 and an antisense strand including a nucleobase sequence of a nucleotide represented by SEQ ID NO: 105,
(w) a dsRNA formed from a sense strand including a nucleobase sequence of a nucleotide represented by SEQ ID NO: 110 and an antisense strand including a nucleobase sequence of a nucleotide represented by SEQ ID NO: 111,
(x) a dsRNA formed from a sense strand including a nucleobase sequence of a nucleotide represented by SEQ ID NO: 112 and an antisense strand including a nucleobase sequence of a nucleotide represented by SEQ ID NO: 113,
(y) a dsRNA formed from a sense strand including a nucleobase sequence of a nucleotide represented by SEQ ID NO: 114 and an antisense strand including a nucleobase sequence of a nucleotide represented by SEQ ID NO: 115,
(z) a dsRNA formed from a sense strand including a nucleobase sequence of a nucleotide represented by SEQ ID NO: 118 and an antisense strand including a nucleobase sequence of a nucleotide represented by SEQ ID NO: 119,
(aa) a dsRNA formed from a sense strand including a nucleobase sequence of a nucleotide represented by SEQ ID NO: 128 and an antisense strand including a nucleobase sequence of a nucleotide represented by SEQ ID NO: 129,
(ab) a dsRNA formed from a sense strand including a nucleobase sequence of a nucleotide represented by SEQ ID NO: 132 and an antisense strand including a nucleobase sequence of a nucleotide represented by SEQ ID NO: 133,
(ac) a dsRNA formed from a sense strand including a nucleobase sequence of a nucleotide represented by SEQ ID NO: 136 and an antisense strand including a nucleobase sequence of a nucleotide represented by SEQ ID NO: 137,
(ad) a dsRNA formed from a sense strand including a nucleobase sequence of a nucleotide represented by SEQ ID NO: 138 and an antisense strand including a nucleobase sequence of a nucleotide represented by SEQ ID NO: 139,
(ae) a dsRNA formed from a sense strand including a nucleobase sequence of a nucleotide represented by SEQ ID NO: 140 and an antisense strand including a nucleobase sequence of a nucleotide represented by SEQ ID NO: 141,
(af) a dsRNA formed from a sense strand including a nucleobase sequence of a nucleotide represented by SEQ ID NO: 142 and an antisense strand including a nucleobase sequence of a nucleotide represented by SEQ ID NO: 143,
(ag) a dsRNA formed from a sense strand including a nucleobase sequence of a nucleotide represented by SEQ ID NO: 144 and an antisense strand including a nucleobase sequence of a nucleotide represented by SEQ ID NO: 145,
(ah) a dsRNA formed from a sense strand including a nucleobase sequence of a nucleotide represented by SEQ ID NO: 150 and an antisense strand including a nucleobase sequence of a nucleotide represented by SEQ ID NO: 151,
(ai) a dsRNA formed from a sense strand including a nucleobase sequence of a nucleotide represented by SEQ ID NO: 152 and an antisense strand including a nucleobase sequence of a nucleotide represented by SEQ ID NO: 153,
(aj) a dsRNA formed from a sense strand including a nucleobase sequence of a nucleotide represented by SEQ ID NO: 158 and an antisense strand including a nucleobase sequence of a nucleotide represented by SEQ ID NO: 159,
(ak) a dsRNA formed from a sense strand including a nucleobase sequence of a nucleotide represented by SEQ ID NO: 160 and an antisense strand including a nucleobase sequence of a nucleotide represented by SEQ ID NO: 161,
(al) a dsRNA formed from a sense strand including a nucleobase sequence of a nucleotide represented by SEQ ID NO: 164 and an antisense strand including a nucleobase sequence of a nucleotide represented by SEQ ID NO: 165,
(am) a dsRNA formed from a sense strand including a nucleobase sequence of a nucleotide represented by SEQ ID NO: 168 and an antisense strand including a nucleobase sequence of a nucleotide represented by SEQ ID NO: 169,
(an) a dsRNA formed from a sense strand including a nucleobase sequence of a nucleotide represented by SEQ ID NO: 174 and an antisense strand including a nucleobase sequence of a nucleotide represented by SEQ ID NO: 175,
(ao) a dsRNA formed from a sense strand including a nucleobase sequence of a nucleotide represented by SEQ ID NO: 176 and an antisense strand including a nucleobase sequence of a nucleotide represented by SEQ ID NO: 177,
(ap) a dsRNA formed from a sense strand including a nucleobase sequence of a nucleotide represented by SEQ ID NO: 178 and an antisense strand including a nucleobase sequence of a nucleotide represented by SEQ ID NO: 179,
(aq) a dsRNA formed from a sense strand including a nucleobase sequence of a nucleotide represented by SEQ ID NO: 182 and an antisense strand including a nucleobase sequence of a nucleotide represented by SEQ ID NO: 183,
(ar) a dsRNA formed from a sense strand including a nucleobase sequence of a nucleotide represented by SEQ ID NO: 184 and an antisense strand including a nucleobase sequence of a nucleotide represented by SEQ ID NO: 185,
(as) a dsRNA formed from a sense strand including a nucleobase sequence of a nucleotide represented by SEQ ID NO: 186 and an antisense strand including a nucleobase sequence of a nucleotide represented by SEQ ID NO: 187,
(at) a dsRNA formed from a sense strand including a nucleobase sequence of a nucleotide represented by SEQ ID NO: 188 and an antisense strand including a nucleobase sequence of a nucleotide represented by SEQ ID NO: 189,
(au) a dsRNA formed from a sense strand including a nucleobase sequence of a nucleotide represented by SEQ ID NO: 194 and an antisense strand including a nucleobase sequence of a nucleotide represented by SEQ ID NO: 195,
(av) a dsRNA formed from a sense strand including a nucleobase sequence of a nucleotide represented by SEQ ID NO: 196 and an antisense strand including a nucleobase sequence of a nucleotide represented by SEQ ID NO: 197,
(aw) a dsRNA formed from a sense strand including a nucleobase sequence of a nucleotide represented by SEQ ID NO: 198 and an antisense strand including a nucleobase sequence of a nucleotide represented by SEQ ID NO: 199,
(ax) a dsRNA formed from a sense strand including a nucleobase sequence of a nucleotide represented by SEQ ID NO: 200 and an antisense strand including a nucleobase sequence of a nucleotide represented by SEQ ID NO: 201,
(ay) a dsRNA formed from a sense strand including a nucleobase sequence of a nucleotide represented by SEQ ID NO: 202 and an antisense strand including a nucleobase sequence of a nucleotide represented by SEQ ID NO: 203,
(az) a dsRNA formed from a sense strand including a nucleobase sequence of a nucleotide represented by SEQ ID NO: 204 and an antisense strand including a nucleobase sequence of a nucleotide represented by SEQ ID NO: 205,
(ba) a dsRNA formed from a sense strand including a nucleobase sequence of a nucleotide represented by SEQ ID NO: 206 and an antisense strand including a nucleobase sequence of a nucleotide represented by SEQ ID NO: 207,
(bb) a dsRNA formed from a sense strand including a nucleobase sequence of a nucleotide represented by SEQ ID NO: 210 and an antisense strand including a nucleobase sequence of a nucleotide represented by SEQ ID NO: 211,
(bc) a dsRNA formed from a sense strand including a nucleobase sequence of a nucleotide represented by SEQ ID NO: 212 and an antisense strand including a nucleobase sequence of a nucleotide represented by SEQ ID NO: 213,
(bd) a dsRNA formed from a sense strand including a nucleobase sequence of a nucleotide represented by SEQ ID NO: 214 and an antisense strand including a nucleobase sequence of a nucleotide represented by SEQ ID NO: 215,
(be) a dsRNA formed from a sense strand including a nucleobase sequence of a nucleotide represented by SEQ ID NO: 220 and an antisense strand including a nucleobase sequence of a nucleotide represented by SEQ ID NO: 221,
(bf) a dsRNA formed from a sense strand including a nucleobase sequence of a nucleotide represented by SEQ ID NO: 222 and an antisense strand including a nucleobase sequence of a nucleotide represented by SEQ ID NO: 223,
(bg) a dsRNA formed from a sense strand including a nucleobase sequence of a nucleotide represented by SEQ ID NO: 226 and an antisense strand including a nucleobase sequence of a nucleotide represented by SEQ ID NO: 227,
(bh) a dsRNA formed from a sense strand including a nucleobase sequence of a nucleotide represented by SEQ ID NO: 234 and an antisense strand including a nucleobase sequence of a nucleotide represented by SEQ ID NO: 235,
(bi) a dsRNA formed from a sense strand including a nucleobase sequence of a nucleotide represented by SEQ ID NO: 236 and an antisense strand including a nucleobase sequence of a nucleotide represented by SEQ ID NO: 237,
(bj) a dsRNA formed from a sense strand including a nucleobase sequence of a nucleotide represented by SEQ ID NO: 238 and an antisense strand including a nucleobase sequence of a nucleotide represented by SEQ ID NO: 239,
(bk) a dsRNA formed from a sense strand including a nucleobase sequence of a nucleotide represented by SEQ ID NO: 242 and an antisense strand including a nucleobase sequence of a nucleotide represented by SEQ ID NO: 243,
(bl) a dsRNA formed from a sense strand including a nucleobase sequence of a nucleotide represented by SEQ ID NO: 244 and an antisense strand including a nucleobase sequence of a nucleotide represented by SEQ ID NO: 245,
(bm) a dsRNA formed from a sense strand including a nucleobase sequence of a nucleotide represented by SEQ ID NO: 246 and an antisense strand including a nucleobase sequence of a nucleotide represented by SEQ ID NO: 247,
(bn) a dsRNA formed from a sense strand including a nucleobase sequence of a nucleotide represented by SEQ ID NO: 248 and an antisense strand including a nucleobase sequence of a nucleotide represented by SEQ ID NO: 249,
(bo) a dsRNA formed from a sense strand including a nucleobase sequence of a nucleotide represented by SEQ ID NO: 250 and an antisense strand including a nucleobase sequence of a nucleotide represented by SEQ ID NO: 251,
(bp) a dsRNA formed from a sense strand including a nucleobase sequence of a nucleotide represented by SEQ ID NO: 252 and an antisense strand including a nucleobase sequence of a nucleotide represented by SEQ ID NO: 253,
(bq) a dsRNA formed from a sense strand including a nucleobase sequence of a nucleotide represented by SEQ ID NO: 254 and an antisense strand including a nucleobase sequence of a nucleotide represented by SEQ ID NO: 255,
(br) a dsRNA formed from a sense strand including a nucleobase sequence of a nucleotide represented by SEQ ID NO: 256 and an antisense strand including a nucleobase sequence of a nucleotide represented by SEQ ID NO: 257,
(bs) a dsRNA formed from a sense strand including a nucleobase sequence of a nucleotide represented by SEQ ID NO: 258 and an antisense strand including a nucleobase sequence of a nucleotide represented by SEQ ID NO: 259,
(bt) a dsRNA formed from a sense strand including a nucleobase sequence of a nucleotide represented by SEQ ID NO: 260 and an antisense strand including a nucleobase sequence of a nucleotide represented by SEQ ID NO: 261,
(bu) a dsRNA formed from a sense strand including a nucleobase sequence of a nucleotide represented by SEQ ID NO: 262 and an antisense strand including a nucleobase sequence of a nucleotide represented by SEQ ID NO: 263,
(bv) a dsRNA formed from a sense strand including a nucleobase sequence of a nucleotide represented by SEQ ID NO: 264 and an antisense strand including a nucleobase sequence of a nucleotide represented by SEQ ID NO: 265,
(bw) a dsRNA formed from a sense strand including a nucleobase sequence of a nucleotide represented by SEQ ID NO: 266 and an antisense strand including a nucleobase sequence of a nucleotide represented by SEQ ID NO: 267,
(bx) a dsRNA formed from a sense strand including a nucleobase sequence of a nucleotide represented by SEQ ID NO: 268 and an antisense strand including a nucleobase sequence of a nucleotide represented by SEQ ID NO: 269,
(by) a dsRNA formed from a sense strand including a nucleobase sequence of a nucleotide represented by SEQ ID NO: 270 and an antisense strand including a nucleobase sequence of a nucleotide represented by SEQ ID NO: 271,
(bz) a dsRNA formed from a sense strand including a nucleobase sequence of a nucleotide represented by SEQ ID NO: 280 and an antisense strand including a nucleobase sequence of a nucleotide represented by SEQ ID NO: 281,
(ca) a dsRNA formed from a sense strand including a nucleobase sequence of a nucleotide represented by SEQ ID NO: 282 and an antisense strand including a nucleobase sequence of a nucleotide represented by SEQ ID NO: 283,
(cb) a dsRNA formed from a sense strand including a nucleobase sequence of a nucleotide represented by SEQ ID NO: 284 and an antisense strand including a nucleobase sequence of a nucleotide represented by SEQ ID NO: 285,
(cc) a dsRNA formed from a sense strand including a nucleobase sequence of a nucleotide represented by SEQ ID NO: 286 and an antisense strand including a nucleobase sequence of a nucleotide represented by SEQ ID NO: 287,
(cd) a dsRNA formed from a sense strand including a nucleobase sequence of a nucleotide represented by SEQ ID NO: 288 and an antisense strand including a nucleobase sequence of a nucleotide represented by SEQ ID NO: 289,
(ce) a dsRNA formed from a sense strand including a nucleobase sequence of a nucleotide represented by SEQ ID NO: 290 and an antisense strand including a nucleobase sequence of a nucleotide represented by SEQ ID NO: 291,
(cf) a dsRNA formed from a sense strand including a nucleobase sequence of a nucleotide represented by SEQ ID NO: 292 and an antisense strand including a nucleobase sequence of a nucleotide represented by SEQ ID NO: 293,
(cg) a dsRNA formed from a sense strand including a nucleobase sequence of a nucleotide represented by SEQ ID NO: 294 and an antisense strand including a nucleobase sequence of a nucleotide represented by SEQ ID NO: 295,
(ch) a dsRNA formed from a sense strand including a nucleobase sequence of a nucleotide represented by SEQ ID NO: 296 and an antisense strand including a nucleobase sequence of a nucleotide represented by SEQ ID NO: 297,
(ci) a dsRNA formed from a sense strand including a nucleobase sequence of a nucleotide represented by SEQ ID NO: 302 and an antisense strand including a nucleobase sequence of a nucleotide represented by SEQ ID NO: 303,
(cj) a dsRNA formed from a sense strand including a nucleobase sequence of a nucleotide represented by SEQ ID NO: 308 and an antisense strand including a nucleobase sequence of a nucleotide represented by SEQ ID NO: 309,
(ck) a dsRNA formed from a sense strand including a nucleobase sequence of a nucleotide represented by SEQ ID NO: 312 and an antisense strand including a nucleobase sequence of a nucleotide represented by SEQ ID NO: 313,
(cl) a dsRNA formed from a sense strand including a nucleobase sequence of a nucleotide represented by SEQ ID NO: 316 and an antisense strand including a nucleobase sequence of a nucleotide represented by SEQ ID NO: 317,
(cm) a dsRNA formed from a sense strand including a nucleobase sequence of a nucleotide represented by SEQ ID NO: 322 and an antisense strand including a nucleobase sequence of a nucleotide represented by SEQ ID NO: 323,
(cn) a dsRNA formed from a sense strand including a nucleobase sequence of a nucleotide represented by SEQ ID NO: 330 and an antisense strand including a nucleobase sequence of a nucleotide represented by SEQ ID NO: 331,
(co) a dsRNA formed from a sense strand including a nucleobase sequence of a nucleotide represented by SEQ ID NO: 332 and an antisense strand including a nucleobase sequence of a nucleotide represented by SEQ ID NO: 333,
(cp) a dsRNA formed from a sense strand including a nucleobase sequence of a nucleotide represented by SEQ ID NO: 336 and an antisense strand including a nucleobase sequence of a nucleotide represented by SEQ ID NO: 337,
(cq) a dsRNA formed from a sense strand including a nucleobase sequence of a nucleotide represented by SEQ ID NO: 344 and an antisense strand including a nucleobase sequence of a nucleotide represented by SEQ ID NO: 345,
(cr) a dsRNA formed from a sense strand including a nucleobase sequence of a nucleotide represented by SEQ ID NO: 346 and an antisense strand including a nucleobase sequence of a nucleotide represented by SEQ ID NO: 347,
(cs) a dsRNA formed from a sense strand including a nucleobase sequence of a nucleotide represented by SEQ ID NO: 348 and an antisense strand including a nucleobase sequence of a nucleotide represented by SEQ ID NO: 349,
(ct) a dsRNA formed from a sense strand including a nucleobase sequence of a nucleotide represented by SEQ ID NO: 350 and an antisense strand including a nucleobase sequence of a nucleotide represented by SEQ ID NO: 351,
(cu) a dsRNA formed from a sense strand including a nucleobase sequence of a nucleotide represented by SEQ ID NO: 352 and an antisense strand including a nucleobase sequence of a nucleotide represented by SEQ ID NO: 353,
(cv) a dsRNA formed from a sense strand including a nucleobase sequence of a nucleotide represented by SEQ ID NO: 356 and an antisense strand including a nucleobase sequence of a nucleotide represented by SEQ ID NO: 357,
(cw) a dsRNA formed from a sense strand including a nucleobase sequence of a nucleotide represented by SEQ ID NO: 358 and an antisense strand including a nucleobase sequence of a nucleotide represented by SEQ ID NO: 359,
(cx) a dsRNA formed from a sense strand including a nucleobase sequence of a nucleotide represented by SEQ ID NO: 360 and an antisense strand including a nucleobase sequence of a nucleotide represented by SEQ ID NO: 361,
(cy) a dsRNA formed from a sense strand including a nucleobase sequence of a nucleotide represented by SEQ ID NO: 364 and an antisense strand including a nucleobase sequence of a nucleotide represented by SEQ ID NO: 365,
(cz) a dsRNA formed from a sense strand including a nucleobase sequence of a nucleotide represented by SEQ ID NO: 368 and an antisense strand including a nucleobase sequence of a nucleotide represented by SEQ ID NO: 369,
(da) a dsRNA formed from a sense strand including a nucleobase sequence of a nucleotide represented by SEQ ID NO: 372 and an antisense strand including a nucleobase sequence of a nucleotide represented by SEQ ID NO: 373,
(db) a dsRNA formed from a sense strand including a nucleobase sequence of a nucleotide represented by SEQ ID NO: 378 and an antisense strand including a nucleobase sequence of a nucleotide represented by SEQ ID NO: 379,
(dc) a dsRNA formed from a sense strand including a nucleobase sequence of a nucleotide represented by SEQ ID NO: 380 and an antisense strand including a nucleobase sequence of a nucleotide represented by SEQ ID NO: 381,
(dd) a dsRNA formed from a sense strand including a nucleobase sequence of a nucleotide represented by SEQ ID NO: 386 and an antisense strand including a nucleobase sequence of a nucleotide represented by SEQ ID NO: 387,
(de) a dsRNA formed from a sense strand including a nucleobase sequence of a nucleotide represented by SEQ ID NO: 390 and an antisense strand including a nucleobase sequence of a nucleotide represented by SEQ ID NO: 391,
(df) a dsRNA formed from a sense strand including a nucleobase sequence of a nucleotide represented by SEQ ID NO: 392 and an antisense strand including a nucleobase sequence of a nucleotide represented by SEQ ID NO: 393,
(dg) a dsRNA formed from a sense strand including a nucleobase sequence of a nucleotide represented by SEQ ID NO: 394 and an antisense strand including a nucleobase sequence of a nucleotide represented by SEQ ID NO: 395,
(dh) a dsRNA formed from a sense strand including a nucleobase sequence of a nucleotide represented by SEQ ID NO: 396 and an antisense strand including a nucleobase sequence of a nucleotide represented by SEQ ID NO: 397,
(di) a dsRNA formed from a sense strand including a nucleobase sequence of a nucleotide represented by SEQ ID NO: 398 and an antisense strand including a nucleobase sequence of a nucleotide represented by SEQ ID NO: 399,
(dj) a dsRNA formed from a sense strand including a nucleobase sequence of a nucleotide represented by SEQ ID NO: 400 and an antisense strand including a nucleobase sequence of a nucleotide represented by SEQ ID NO: 401,
(dk) a dsRNA formed from a sense strand including a nucleobase sequence of a nucleotide represented by SEQ ID NO: 402 and an antisense strand including a nucleobase sequence of a nucleotide represented by SEQ ID NO: 403,
(dl) a dsRNA formed from a sense strand including a nucleobase sequence of a nucleotide represented by SEQ ID NO: 404 and an antisense strand including a nucleobase sequence of a nucleotide represented by SEQ ID NO: 405,
(dm) a dsRNA formed from a sense strand including a nucleobase sequence of a nucleotide represented by SEQ ID NO: 406 and an antisense strand including a nucleobase sequence of a nucleotide represented by SEQ ID NO: 407,
(dn) a dsRNA formed from a sense strand including a nucleobase sequence of a nucleotide represented by SEQ ID NO: 410 and an antisense strand including a nucleobase sequence of a nucleotide represented by SEQ ID NO: 411,
(do) a dsRNA formed from a sense strand including a nucleobase sequence of a nucleotide represented by SEQ ID NO: 412 and an antisense strand including a nucleobase sequence of a nucleotide represented by SEQ ID NO: 413,
(dp) a dsRNA formed from a sense strand including a nucleobase sequence of a nucleotide represented by SEQ ID NO: 414 and an antisense strand including a nucleobase sequence of a nucleotide represented by SEQ ID NO: 415,
(dq) a dsRNA formed from a sense strand including a nucleobase sequence of a nucleotide represented by SEQ ID NO: 416 and an antisense strand including a nucleobase sequence of a nucleotide represented by SEQ ID NO: 417,
(dr) a dsRNA formed from a sense strand including a nucleobase sequence of a nucleotide represented by SEQ ID NO: 418 and an antisense strand including a nucleobase sequence of a nucleotide represented by SEQ ID NO: 419,
(ds) a dsRNA formed from a sense strand including a nucleobase sequence of a nucleotide represented by SEQ ID NO: 422 and an antisense strand including a nucleobase sequence of a nucleotide represented by SEQ ID NO: 423,
(dt) a dsRNA formed from a sense strand including a nucleobase sequence of a nucleotide represented by SEQ ID NO: 424 and an antisense strand including a nucleobase sequence of a nucleotide represented by SEQ ID NO: 425,
(du) a dsRNA formed from a sense strand including a nucleobase sequence of a nucleotide represented by SEQ ID NO: 426 and an antisense strand including a nucleobase sequence of a nucleotide represented by SEQ ID NO: 427,
(dv) a dsRNA formed from a sense strand including a nucleobase sequence of a nucleotide represented by SEQ ID NO: 428 and an antisense strand including a nucleobase sequence of a nucleotide represented by SEQ ID NO: 429,
(dw) a dsRNA formed from a sense strand including a nucleobase sequence of a nucleotide represented by SEQ ID NO: 430 and an antisense strand including a nucleobase sequence of a nucleotide represented by SEQ ID NO: 431,
(dx) a dsRNA formed from a sense strand including a nucleobase sequence of a nucleotide represented by SEQ ID NO: 432 and an antisense strand including a nucleobase sequence of a nucleotide represented by SEQ ID NO: 433,
(dy) a dsRNA formed from a sense strand including a nucleobase sequence of a nucleotide represented by SEQ ID NO: 434 and an antisense strand including a nucleobase sequence of a nucleotide represented by SEQ ID NO: 435,
(dz) a dsRNA formed from a sense strand including a nucleobase sequence of a nucleotide represented by SEQ ID NO: 436 and an antisense strand including a nucleobase sequence of a nucleotide represented by SEQ ID NO: 437,
(ea) a dsRNA formed from a sense strand including a nucleobase sequence of a nucleotide represented by SEQ ID NO: 438 and an antisense strand including a nucleobase sequence of a nucleotide represented by SEQ ID NO: 439,
(eb) a dsRNA formed from a sense strand including a nucleobase sequence of a nucleotide represented by SEQ ID NO: 440 and an antisense strand including a nucleobase sequence of a nucleotide represented by SEQ ID NO: 441,
(ec) a dsRNA formed from a sense strand including a nucleobase sequence of a nucleotide represented by SEQ ID NO: 442 and an antisense strand including a nucleobase sequence of a nucleotide represented by SEQ ID NO: 443,
(ed) a dsRNA formed from a sense strand including a nucleobase sequence of a nucleotide represented by SEQ ID NO: 444 and an antisense strand including a nucleobase sequence of a nucleotide represented by SEQ ID NO: 445, and
(ee) a dsRNA formed from a sense strand including a nucleobase sequence of a nucleotide represented by SEQ ID NO: 446 and an antisense strand including a nucleobase sequence of a nucleotide represented by SEQ ID NO: 447.

4. The double-stranded RNA according to any one of claims 1 to 3, further comprising a modified nucleotide.

5. The double-stranded RNA according to any one of claims 1 to 4,
wherein the HBV protein is an HBs antigen (HBsAg) protein.

6. The double-stranded RNA according to any one of claims 1 to 5,
wherein the double-stranded RNA reduces an expression level of an HBV DNA.

7. The double-stranded RNA according to any one of claims 1 to 6,
wherein the double-stranded RNA reduces an expression level of a covalently closed circular DNA (cccDNA).

8. The double-stranded RNA according to any one of claims 1 to 7,
wherein the double-stranded RNA is an RNA interfering agent.

9. A pharmaceutical composition comprising the double-stranded RNA according to any one of claims 1 to 8.

10. The pharmaceutical composition according to claim 9,
wherein the double-stranded RNA is encoded in a recombinant expression vector.

11. The pharmaceutical composition according to claim 10,
wherein the recombinant expression vector is an adeno-associated virus vector, a retrovirus vector, an adenovirus vector, or an alphavirus vector.

12. The pharmaceutical composition according to claim 9, further comprising lipid nanoparticles (LNP) or N-acetylgalactosamine (GalNAc).

13. A vector comprising the double-stranded RNA according to any one of claims 1 to 8.

14. The vector according to claim 13,
wherein the vector is an expression vector, and
the expression vector is an adeno-associated virus vector, a retrovirus vector, an adenovirus vector, or an alphavirus vector.
